(19) Europäisches Patentamt
European Patent Office
Office européen des brevets

(11) **EP 2 667 891 B1**

(12) **EUROPEAN PATENT SPECIFICATION**

(45) Date of publication and mention
of the grant of the patent:
**06.10.2021 Bulletin 2021/40**

(21) Application number: **12739840.2**

(22) Date of filing: **27.01.2012**

(51) Int Cl.:
*A61K 39/145* (2006.01)     *A61K 39/125* (2006.01)
*A61K 39/36* (2006.01)       *A61K 39/085* (2006.01)
*A61K 39/215* (2006.01)      *A61P 11/00* (2006.01)
*A61K 39/09* (2006.01)       *A61K 39/235* (2006.01)
*A61P 31/16* (2006.01)       *A61K 39/12* (2006.01)
*A61K 39/00* (2006.01)       *C07K 16/10* (2006.01)

(86) International application number:
**PCT/AU2012/000069**

(87) International publication number:
**WO 2012/100302 (02.08.2012 Gazette 2012/31)**

(54) **COMBINATION VACCINES**

KOMBINATIONSIMPFSTOFFE

VACCINS ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.01.2011 AU 2011900262**

(43) Date of publication of application:
**04.12.2013 Bulletin 2013/49**

(73) Proprietor: **Gamma Vaccines Pty Limited
Yarralumla, ACT 2600 (AU)**

(72) Inventors:
• **MULLBACHER, Arno**
**Curtin, ACT 2601 (AU)**
• **ALSHARIFI, Mohammed**
**Gilles Plains, S.A. 5086 (AU)**
• **HIRST, Tim**
**Yarralumla, ACT 2600 (AU)**

(74) Representative: **Plasseraud IP
235 Cours Lafayette
69006 Lyon (FR)**

(56) References cited:
WO-A1-2010/012045     WO-A1-2010/012045
WO-A1-2011/133997     WO-A2-2006/120439
WO-A2-2007/092315

- **YUAN-ZHENG QIU ET AL: "Safety and immunogenicity of Sinovac's prototype pandemic influenza H5N1 vaccines: a review on clinical trials", INFLUENZA AND OTHER RESPIRATORY VIRUSES, vol. 2, no. 6, 1 November 2008 (2008-11-01), pages 237-242, XP055304371, UK ISSN: 1750-2640, DOI: 10.1111/j.1750-2659.2008.00067.x**
- **DIEBOLD S ET AL: "Innate antiviral responses by means of TLR7-mediated recognition of single-stranded RNA", SCIENCE, AMERICAN ASSOCIATION FOR THE ADVANCEMENT OF SCIENCE, US, vol. 303, 5 March 2004 (2004-03-05), pages 1529-1531, XP002358690, ISSN: 0036-8075, DOI: 10.1126/SCIENCE.1093616**
- **MÜLLBACHER A ET AL: "Gamma-irradiated influenza A virus can prime for a cross-reactive and cross-protective immune response against influenza A viruses", IMMUNOLOGY AND CELL BIOLOGY, CARLTON, AU, vol. 66, 1 January 1988 (1988-01-01), pages 153-157, XP008143623, ISSN: 0818-9641**
- **Mohammed Alsharifi ET AL: "Intranasal Flu Vaccine Protective against Seasonal and H5N1 Avian Influenza Infections", PLoS ONE, vol. 4, no. 4, 29 April 2009 (2009-04-29), page e5336, XP055743798, DOI: 10.1371/journal.pone.0005336**

EP 2 667 891 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Carl Woese: "THERMAL INACTIVATION OF ANIMAL VIRUSES", Annals of the New York Academy of Sciences, vol. 83, no. 4, 1 January 1960 (1960-01-01), pages 741-751, XP055745190, US ISSN: 0077-8923, DOI: 10.1111/j.1749-6632.1960.tb40943.x

**Description**

**Technical Field**

The present disclosure relates to the field of vaccines. More specifically the present disclosure relates to compositions and methods for enhancing immune responses induced by vaccines, including vaccines targeted at secondary infections and conditions associated with influenza infection.

**Background**

Influenza and its complications are a significant cause of morbidity and mortality worldwide. The World Health Organization estimates that influenza epidemics in the developed world cause at least 250,000-500,000 deaths and severe illness in 3-5 million people annually. The impact of influenza in the developing world is likely to be even higher.
Influenza infection is generally characterised by the onset of constitutional symptoms (e.g. fever, headache, myalgia, severe malaise, nausea) and respiratory symptoms (e.g. cough and sore throat). With the exception of particularly virulent strains (e.g. the 1918 pandemic flu strain, H5N1 "bird flu") capable of inducing viral pneumonia and pulmonary failure, influenza viruses are generally not sufficiently virulent to inflict serious illness or death on their own. However, secondary infections associated with influenza infection frequently cause pneumonia and other serious complications which may often prove fatal. For example, the high mortality rates experienced during the pandemics of 1918-1919 and 1957-1958 have been largely attributed to complications arising from secondary bacterial infections, and secondary bacterial pneumonia has been estimated to cause at least 20,000 deaths each year in the U.S. Secondary complications arising during influenza infection are also not restricted to those arising from pathogenic bacteria. Allergic responses can be enhanced during influenza infection, particularly those instigated by respiratory allergens. In addition, secondary viral infections (e.g. rhinovirus, coronavirus) may also arise during the course of flu infection.
Seasonal multivalent vaccines are the most common form of vaccination against influenza, however, the immunity induced by these vaccines is limited and generally restricted to the particular target strains included in the formulation. Although alternative influenza vaccines are available most suffer the disadvantage of providing little or no cross-protective immunity (i.e. immunity to multiple different strains) and consequently are not widely used. In addition, current flu vaccines provide inadequate protection against the development of secondary infections and conditions which are a leading cause of morbidity and mortality in both local influenza outbreaks and major influenza pandemics.
The patent application WO2010/012045A1 as well as Alsharifi et al, 2009, PloS ONE Volume 4, Issue 4, e5336, disclose methods for the treatment or prevention of an influenza virus infection in a subject. Diebold et al ((Diebold et al. Innate Antiviral Responses by Means of TLR7-Mediated Recognition of Single-Stranded RNA, Science, 2004; 303(5663): 1529-1531) teaches that <u>heat-inactivated</u> influenza virus ssRNA at certain temperatures can trigger IFN-$\alpha$ responses and could thus be exploited as an adjuvant for vaccination. The patent application WO2007/092315A2 relates to immunogenic compositions and more particularly to complexes formed between viruses and oligonucleotides with enhanced immunogenic properties.
Accordingly, a general need exists for agents capable of enhancing immune responses induced by vaccines. In addition, there is a need for more effective treatments capable of preventing or alleviating secondary infections and conditions associated with influenza infection.

**Summary**

The invention is defined in the claims.
In a first aspect, the disclosure provides a method for preventing or treating an infection or condition in a subject, the method comprising administering to the subject a combination of a gamma-irradiated influenza virus and an immunogen against an agent causative of the infection or condition.
In one embodiment of the first aspect, the infection or condition is a secondary infection or condition following influenza virus infection.
In a second aspect, the disclosure provides a method for preventing or treating a secondary infection or condition following influenza virus infection, the method comprising administering to a subject a combination of a gamma-irradiated influenza virus and an immunogen against an agent causative of the secondary infection or condition.
In one embodiment of the first or second aspect, the immunogen is a gamma-irradiated microorganism.
In one embodiment of the first or second aspect, the gamma-irradiated influenza virus and the immunogen are administered to the subject simultaneously.
In one embodiment of the first or second aspect, the gamma-irradiated influenza virus and the immunogen are administered to the subject separately.
In a third aspect, the disclosure provides a method for enhancing an immune response in a subject induced by a vaccine against an agent causative of an infection or condition, the method comprising administering the vaccine in combination with a gamma-irradiated influenza virus to the subject.

In one embodiment of the third aspect, the agent is:

(i) a bacterium selected from the group consisting of *Pseudomonas aeruginosa, Haemophilus influenzae, Streptococcus pneumoniae, Staphylococcus aureus, Moraxella catarrhalis,* and *Mycoplasma sp.;*
(ii) a virus selected from the group consisting of a rhinovirus, adenovirus, coxsackievirus, picornavirus, togavirus and coronavirus; or
(iii) a respiratory allergen selected from the group consisting of pollen, mould, house dust mite *(Dermatophagoides pteronyssinus),* dust, protein allergens from animals, and chemical allergens.

In one embodiment of the third aspect, the infection or condition is a secondary infection or condition following influenza virus infection.

In a fourth aspect, the disclosure provides a method for enhancing an immune response in a subject induced by a vaccine against a secondary infection or condition following influenza virus infection, the method comprising administering the vaccine in combination with a gamma-irradiated influenza virus to the subject.

In one embodiment of the third or fourth aspect, the vaccine comprises a gamma-irradiated microorganism causative of the secondary infection or condition.

In one embodiment of the third or fourth aspect, the vaccine comprises an immunogen.

In one embodiment of the third or fourth aspect, the gamma-irradiated influenza virus and the vaccine are administered to the subject simultaneously.

In one embodiment of the third or fourth aspect, the gamma-irradiated influenza virus and the vaccine are administered to the subject separately.

In one embodiment of the third and fourth aspect, the gamma-irradiated influenza virus and vaccine are administered to the subject using different modes of administration.

In one embodiment of the third and fourth aspect, the gamma-irradiated influenza virus and vaccine are administered to the subject at different administration sites.

In one embodiment of the third and fourth aspect, the gamma-irradiated influenza virus and vaccine are administered to the subject sequentially (e.g. within about 0.5, 1, 2, 3, 4, 5, 10, or 15 minutes).

In a fifth aspect, the disclosure provides a method for enhancing an immune response in a subject against a secondary infection or condition following influenza virus infection, the method comprising administering to the subject a gamma-irradiated influenza virus.

In one embodiment of the first, second, third, fourth or fifth aspect, the gamma-irradiated influenza virus is an influenza A H1N1 subtype virus.

In one embodiment of the first, second, third, fourth or fifth aspect, the influenza A H1N1 subtype virus is an APR/8/34 virus.

In one embodiment of the first, second, third, fourth or fifth aspect, the subject is administered multiple different strains of a gamma-irradiated influenza virus.

In one embodiment of the first, second, third, fourth or fifth aspect, the secondary infection is a bacterial infection, a viral infection, a fungal infection or a parasitic infection.

In one embodiment of the first, second, third, fourth or fifth aspect, the infection is a bacterial infection, a viral infection, a fungal infection or a parasitic infection.

In one embodiment of the first, second, third, fourth or fifth aspect, the secondary infection is a bacterial infection mediated by an organism selected from the group consisting of *Pseudomonas aeruginosa, Haemophilus influenzae, Streptococcus pneumoniae, Staphylococcus aureus, Moraxella catarrhalis,* and *Mycoplasma sp.*

In one embodiment of the first, second, third, fourth or fifth aspect, the secondary infection is selected from the group consisting of bacterial pneumonia, chronic obstructive pulmonary disease, bacterial sinusitis, and otitis media.

In one embodiment of the first, second, third, fourth or fifth aspect, the secondary infection is a viral infection mediated by an organism selected from the group consisting of rhinovirus, adenovirus, coxsackievirus, picornavirus, and coronavirus.

In one embodiment of the first, second, third, fourth or fifth aspect, the secondary infection is a common cold.

In one embodiment of the first, second, third, fourth or fifth aspect, the secondary condition is a respiratory allergy.

In one embodiment of the first, second, third, fourth or fifth aspect, the allergy arises from exposure of the subject to an allergen selected from the group consisting of pollen, mould, house dust mite *(Dermatophagoides pteronyssinus),* dust, protein allergens from animals, chemical allergens, and combinations thereof.

In one embodiment of the first, second, third, fourth or fifth aspect, the administering is intranasal.

In one embodiment of the first, second, third, or fourth aspect the immunogen is:

(i) a bacterium selected from the group consisting of *Pseudomonas aeruginosa, Haemophilus influenzae, Streptococcus pneumoniae, Staphylococcus aureus, Moraxella catarrhalis,* and *Mycoplasma sp.,* or a component thereof;
(ii) a virus selected from the group consisting of a rhinovirus, adenovirus, coxsackievirus, picornavirus, togavirus and coronavirus, or a component thereof; or
(iii) a respiratory allergen selected from the group consisting of pollen, mould, house dust mite *(Dermatophagoides pteronyssinus),* dust, protein allergens from animals, and chemical allergens.

In a sixth aspect, the disclosure provides use of a gamma-irradiated influenza virus for enhancing an immune response to an immunogen co-administered to a subject with said virus.

In one embodiment of the sixth aspect, the immunogen is not an influenza virus immunogen.

In one embodiment of the sixth aspect, the immune response induced by the immunogen is an antigen-specific immune response.

In one embodiment of the sixth aspect, the immunogen is a component of a vaccine.

In one embodiment of the sixth aspect, the use comprises administering the gamma-irradiated influenza virus and immunogen to a subject simultaneously.

In one embodiment of the sixth aspect, the use comprises administering the gamma-irradiated influenza virus and immunogen to a subject separately.

In one embodiment of the sixth aspect, the immunogen is an agent or is derived from an agent causative of a secondary infection or condition following influenza infection.

In one embodiment of the sixth aspect, the immunogen is, or is derived from, a bacteria, virus, fungus, or parasite, or is a respiratory allergen.

In one embodiment of the sixth aspect, the immunogen is:

(i) a bacterium selected from the group consisting of *Pseudomonas aeruginosa, Haemophilus influenzae, Streptococcus pneumoniae, Staphylococcus aureus, Moraxella catarrhalis,* and *Mycoplasma sp.,* or a component thereof;
(ii) a virus selected from the group consisting of a rhinovirus, adenovirus, coxsackievirus, picornavirus, togavirus and coronavirus, or a component thereof; or
(iii) a respiratory allergen selected from the group consisting of pollen, mould, house dust mite *(Dermatophagoides pteronyssinus),* dust, protein allergens from animals, and chemical allergens.

In one embodiment of the sixth aspect, the immunogen is a gamma-irradiated microorganism.

In one embodiment of the first, second, third, fourth, fifth or sixth aspect, the gamma-irradiated influenza virus is administered to the subject intranasally.

In one embodiment of the first, second or sixth aspect, the gamma-irradiated influenza virus and immunogen are administered to the subject using different modes of administration.

In one embodiment of the first, second or sixth aspect, the gamma-irradiated influenza virus and immunogen are administered to the subject at different administration sites.

In one embodiment of the first, second or sixth aspect, the gamma-irradiated influenza virus and immunogen are administered to the subject simultaneously or sequentially (e.g. within about 0.5, 1, 2, 3, 4, 5, 10, or 15 minutes).

In a seventh aspect, the disclosure provides a vaccine composition comprising a gamma-irradiated influenza virus and an additional immunogen.

In one embodiment of the seventh aspect, the gamma-irradiated influenza virus enhances an immune response induced by the additional immunogen upon administration to a subject.

In one embodiment of the seventh aspect, the immune response induced by the immunogen is an antigen-specific immune response.

In one embodiment of the seventh aspect, the additional immunogen is not an influenza virus immunogen.

In an eighth aspect, the disclosure provides a vaccine composition comprising a synergistic combination of a gamma-irradiated influenza virus and an additional immunogen that is not derived from an influenza virus.

In one embodiment of the eighth aspect, the immunogen induces an antigen-specific immune response when administered to the subject.

In one embodiment of the seventh or eighth aspect, the additional immunogen is a gamma-irradiated microorganism.

In one embodiment of the seventh or eighth aspect, the vaccine further comprises a pharmaceutically acceptable excipient, adjuvant or carrier.

In one embodiment of the sixth, seventh or eighth aspect, the gamma-irradiated influenza virus enhances interferon type I responses (e.g. IFNα responses) induced by the immunogen in a subject.

In one embodiment of the sixth, seventh or eighth aspect, the gamma-irradiated influenza virus enhances antigen-specific antibody responses (e.g. antigen specific IgG responses) induced by the immunogen upon administration of the gamma-irradiated influenza virus and immunogen to a subject.

In one embodiment of the sixth, seventh or eighth aspect, the gamma-irradiated influenza virus is an influenza A H1N1 subtype virus.

In one embodiment of the sixth, seventh or eighth aspect, the H1N1 subtype virus is APR/8/34.

In one embodiment of the sixth, seventh or eighth aspect, the vaccine comprises multiple different strains of a gamma-irradiated influenza virus.

In one embodiment of the seventh or eighth aspect, the additional immunogen is derived from an agent causative of a secondary infection or condition following or arising during influenza virus infection.

In one embodiment of the seventh or eighth aspect, the agent is a bacterium, virus, fungus, parasite or respiratory allergen.

In one embodiment of the seventh or eighth aspect, the agent is a bacterium selected from the group consisting of *Pseudomonas aeruginosa, Haemophilus influenzae, Streptococcus pneumoniae, Staphylococcus aureus, Moraxella catarrhalis,* and *Mycoplasma sp.*

In one embodiment of the seventh or eighth aspect, the agent is a virus selected from the group consisting of rhinovirus, adenovirus, coxsackievirus, picornavirus, and coronavirus.

In one embodiment of the seventh or eighth aspect, the respiratory allergen is selected from the group consisting of pollen, mould, house dust mite (*Dermatophagoides pteronyssinus*), dust, protein allergens from animals, chemical allergens, and combinations thereof.

In one embodiment of the seventh or eighth aspect, the vaccine is formulated for intranasal administration.

In one embodiment of the seventh or eighth aspect, the immunogen is:

(i) a bacterium selected from the group consisting of *Pseudomonas aeruginosa, Haemophilus influenzae, Streptococcus pneumoniae, Staphylococcus aureus, Moraxella catarrhalis,* and *Mycoplasma sp.,* or a component thereof;
(ii) a virus selected from the group consisting of a rhinovirus, adenovirus, coxsackievirus, picornavirus, togavirus and coronavirus, or a component thereof; or
(iii) a respiratory allergen selected from the group consisting of pollen, mould, house dust mite *(Dermatophagoides pteronyssinus),* dust, protein allergens from animals, and chemical allergens.

In a ninth aspect, the disclosure provides use of a gamma-irradiated influenza virus and an additional immunogen in the preparation of a medicament for preventing or treating an infection or condition in a subject, wherein the additional immunogen induces an immune response against an agent causative of the infection or condition.

In one embodiment of the ninth aspect, the infection or condition is a secondary infection or condition following influenza virus infection.

In a tenth aspect, the disclosure provides use of a gamma-irradiated influenza virus and an additional immunogen in the preparation of a medicament for preventing or treating a secondary infection or condition following influenza virus infection in a subject, wherein the additional immunogen induces an immune response against an agent causative of the secondary infection or condition.

In one embodiment of the ninth or tenth aspect, the medicament is formulated for separate administration of said gamma-irradiated influenza virus and said additional immunogen.

In one embodiment of the ninth or tenth aspect, the medicament is formulated for simultaneous administration of said gamma-irradiated influenza virus and said additional immunogen.

In an eleventh aspect, the disclosure provides a gamma-irradiated influenza virus and an additional immunogen for use in treating a secondary infection or condition following influenza virus infection in a subject, wherein the additional immunogen induces an immune response against an agent causative of the secondary infection or condition.

In a twelfth aspect, the disclosure provides a gamma-irradiated influenza virus and an additional immunogen for use in treating an infection or condition in a subject, wherein the additional immunogen induces an immune response against an agent causative of the infection or condition.

In a thirteenth aspect, the disclosure provides a gamma-irradiated influenza virus and an immunogen for use in modulating an immune response induced by the immunogen in a subject.

In one embodiment of the thirteenth aspect, the gamma-irradiated influenza virus and immunogen are co-administered to the subject.

In one embodiment of the thirteenth aspect, the immunogen induces an immune response against an agent causative of an infection or condition.

In a fourteenth aspect, the disclosure provides a gamma-irradiated influenza virus and a vaccine for use in modulating an immune response induced by the vaccine in a subj ect.

In one embodiment of the fourteenth aspect, the gamma-irradiated influenza virus and vaccine are co-administered to the subject.

In one embodiment of the fourteenth aspect, the immunogen induces an immune response against an agent causative of an infection or condition.

In one embodiment of the twelfth, thirteenth or fourteenth aspect, the infection or condition is a secondary infection or condition following influenza infection.

In one embodiment of the ninth, tenth, eleventh, twelfth, thirteenth, or fourteenth aspect, the secondary infection or condition is selected from the group consisting of bacterial pneumonia, chronic obstructive pulmonary disease, bacterial sinusitis, otitis media, common cold, and respiratory allergies.

In one embodiment of the ninth, tenth, eleventh, twelfth, thirteenth, or fourteenth aspect, the agent is a bacterium, virus, fungus, parasite or a respiratory allergen.

In one embodiment of the ninth, tenth, eleventh, twelfth, thirteenth, or fourteenth aspect, the agent is a bacterium selected from the group consisting of *Pseudomonas aeruginosa, Haemophilus influenzae, Streptococcus pneumoniae, Staphylococcus aureus, Moraxella catarrhalis,* and *Mycoplasma sp.*

In one embodiment of the ninth, tenth, eleventh, twelfth, thirteenth, or fourteenth aspect, the agent is a virus selected from the group consisting of a rhinovirus, adenovirus, coxsackievirus, picornavirus, togavirus, and coronavirus.

In one embodiment of the ninth, tenth, eleventh, twelfth, thirteenth, or fourteenth aspect, the respiratory allergen is selected from the group consisting of pollen, mould, house dust mite *(Dermatophagoides pteronyssinus),* dust, protein allergens from animals, chemical allergens, and combinations thereof.

In one embodiment of the ninth, tenth, eleventh, twelfth or thirteenth aspect, the immunogen is:

(i) a bacterium selected from the group consisting of *Pseudomonas aeruginosa, Haemophilus influenzae, Streptococcus pneumoniae, Staphylococcus aureus, Moraxella catarrhalis,* and *Mycoplasma sp.,* or a component thereof;
(ii) a virus selected from the group consisting of a rhinovirus, adenovirus, coxsackievirus, picornavirus, togavirus and coronavirus, or a component thereof; or
(iii) a respiratory allergen selected from the group consisting of pollen, mould, house dust mite *(Dermatophagoides pteronyssinus),* dust, protein allergens from animals, and chemical allergens.

In one embodiment of the above aspects, the secondary infection or condition occurs within 7, 10, 14, 17, 21, 24, 28, 31, 35, 38, or 42 days of influenza infection.

In one embodiment of the above aspects, the subject is a mammalian or avain subj ect.

In one embodiment of the above aspects, the subject is a human subject.

In one embodiment of the first, second, ninth, tenth, eleventh, twelfth, or thirteenth aspect, the gamma-irradiated influenza virus enhances interferon type I responses (e.g. IFN$\alpha$ responses) induced by the immunogen upon administration of the gamma-irradiated influenza virus and immunogen to the subject.

In one embodiment of the first, second, ninth, tenth, eleventh, twelfth, or thirteenth aspect, the gamma-irradiated influenza virus enhances antigen-specific antibody responses (e.g. antigen specific IgG responses) induced by the immunogen upon administration of the gamma-irradiated influenza virus and immunogen to the subject.

In one embodiment of the third, fourth or fourteenth aspect, the gamma-irradiated influenza virus enhances interferon type I responses (e.g. IFN$\alpha$ responses) induced by the vaccine upon administration of the vaccine and immunogen to the subject.

In one embodiment of the third, fourth or fourteenth aspect, the gamma-irradiated influenza virus enhances antigen-specific antibody responses (e.g. antigen specific IgG responses) induced by the vaccine upon administration of the vaccine and immunogen to the subject.

**Brief Description of the Figures**

Preferred embodiments of the present disclosure will now be described, by way of an example only, with reference to the accompanying figures wherein:

**Figure 1** is a bar graph showing weight loss of intravenously vaccinated animals following intranasal infection with A/JAP (50 HAU/mouse). Mice, from groups shown in Table 5, were weighed at day 6 post-infection.

**Figure 2** shows representative photomicrographs of immunohistochemically stained lung tissue derived from naive and $\gamma$-flu vaccinated mice. The effect of $\gamma$-flu (gamma-irradiated influenza virus) vaccination on lung inflammation in (A) naive mice, (B) naive mice at day 6 post A/WSN, (C) mice vaccinated with $\gamma$-flu and challenged with the homologous strain A/WSN and (D) mice vaccinated with $\gamma$-flu and challenged with the heterologous strain A/PC, is shown.

**Figure 3** is a bar graph illustrating the effect of $\gamma$-flu vaccination on CD8+ T cell infiltration. Mock or gamma-irradiated influenza virus (y-A/WSN, $\gamma$-A/JAP, $\gamma$-A/Pc) were used to vaccinate animals intravenously. Four weeks later, mice were challenged intranasally with A/WSN. Six days following A/WSN challenge, 3 mice from each group were sacrificed and percentages of CD8+ T cells within the total lung infiltrates were estimated by FACS.

**Figure 4** provides a series of bar graphs illustrating cross-reactive cytotoxic T lymphocyte (CTL) responses induced by γ-flu. BALB/c mice were either infected or vaccinated with A/WSN, γ-A/WSN, A/PC, or γ-A/Pc, and their splenocytes tested for killing activity against (A) mock, (B) A/WSN-infected, (C) A/PC-infected, and (D) NPP-labelled P815 targets.

**Figure 5** provides a series of bar graphs illustrating cross-reactive cytotoxic T cell responses induced by γ-flu. Splenocytes from BALB/c mice infected or vaccinated with A/PR8, γ-A/PR8, A/PC, or γ-A/PC were tested for their killing activity on mock (data not shown), (A) A/PC[H3N2]-infected, (B) A/PR8 [H1N1]-infected, (C) A/JAP [H2N2]-infected, and NPP-labelled P815 targets.

**Figure 6** shows a series of graphs illustrating mortality in mice after challenge with a lethal dose of A/PR8. Groups of mice: (A) naive (unvaccinated), (B) vaccinated intranasally with A/PR8 γ-flu (n=10), (C) vaccinated intranasally with A/PC γ-flu (n=10), and (D) vaccinated intranasally with formalin inactivated A/PC (n=8), were challenged intranasally with $3 \times 10^2$ HAU (2 x 10$^5$ pfu/mouse) of A/PR8. Weight loss and mortality (E) was monitored for 21 days post challenge. The end of an individual mouse's weight track indicates death of the animal.

**Figure 7** shows a series of graphs illustrating that intranasal vaccination with γ-flu provides superior protection to heterotypic virus challenge. Groups of 10 BALB/c mice were either mock treated (A) or vaccinated with γ-A/PC (3.2 x 10$^6$ PFU equivalent) intravenously (B) or intranasally (C). Mice were challenged intranasally after 3 weeks with a lethal dose (6 x 10$^2$ PFU) of A/PR8 and weight recorded daily for 21 days. Survival defined by 30% weight loss (D) of mice mock treated, or vaccinated i.n., i.v., i.p., or s.c. and challenged as for (A-C) and monitored for 21 days.

**Figure 8** shows a series of graphs illustrating weight loss following intranasal infection with H5N1 (A/Vietnam/1203/2004). Infected mice were monitored for weight loss and morbidity. The end of an individual mouse's weight track indicates sacrificing due to ~25% weight loss.

**Figure 9** shows two graphs illustrating body weight and mortality of BALB/c mice following challenge with H5N1. Groups of 10 mice were either (A) mock treated or (B) vaccinated with γ-A/PR8 [H1N1] intranasally. Weight was recorded daily for 21 days.

**Figure 10** provides a series of graphs showing that passive serum transfer fails to transfer heterosubtypic immunity induced by γ-irradiated A/PC to naive mice. (A, B & C) = weight loss; (D) = mortality; Endpoint: 25% weight loss; * P < 0.05 vs. control preimmune sera group; Fisher's exact test.

**Figure 11** provides a series of graphs showing an absence of heterosubtypic protection in B cell-deficient mice. (A) = weight loss in naive mice; (B) = weight loss in immunized mice; (C) = mortality naïve/immunized mice.

**Figure 12** provides a series of graphs showing an absence of heterosubtypic protection in MHC II deficient mice. (A) = weight loss in naive mice; (B) = weight loss in immunized mice; (C) = mortality naïve/immunized mice.

**Figure 13** provides a series of graphs showing a lack of heterosubtypic protection in β2M deficient mice. (A) = weight loss in naive mice; (B) = weight loss in immunized mice; (C) = mortality naïve/immunized mice.

**Figure 14** provides a series of graphs showing that adoptively transferred T cells, but not B cells, protect mice against heterosubtypic challenge. (A, B & C) = weight loss; (D) = mortality; * P < 0.05 vs. control nil group; Fisher's exact test.

**Figure 15** provides a series of graphs showing a lack of heterosubtypic protection in perforin deficient mice. (A & B) = weight loss; (C) = mortality.

**Figure 16** provides a series of graphs showing heterosubtypic protection in Type II IFN receptor knock-out mice. (A, B) = weight loss; (C) = mortality; * P < 0.05 vs. control nil group; Fisher's exact test.

**Figure 17** provides two graphs showing an absence of cross-neutralizing activity in serum of immunized mice. (A) = viral neutralizing activities against A/PC (H3N2); (B) = viral neutralizing activities against A/PR8 (H1N1).

**Figure 18** provides two graphs showing dose dependence of primary Tc cell responses induced by γ-irradiated A/PC. (A, B) = splenocytes harvested 6 days post-immunization. Error bar represents the mean percent ± S.D. Specific lysis values were interpolated from regression curve at effector:target ratio of 60:1.

**Figure 19** is a graph showing secondary *ex vivo* Tc cell responses. Specific lysis values were interpolated from a regression curve at effector:target ratio of 40:1.

**Figure 20** provides a series of graphs showing gamma-irradiated influenza virus A/PC protects mice against both homologous and heterosubtypic challenge. (A, F) = mock treated; (B, G) = intranasally immunized with formalin inactivated A/PC; (C, H) intranasally immunized with UV inactivated A/PC; (D, I) = intranasally immunized with γ-ray inactivated A/PC; (E, J) = survival after 20 days; * P < 0.05 vs. control naive group; Fisher's exact test.

**Figure 21** provides a series of graphs showing that multiple immunizations of formalin-inactivated influenza virus A/PC are required to induce homologous protection. (A, F) = mock treated; (B) = immunized once with formalin-inactivated A/PC; (C) = immunized twice with formalin-inactivated A/PC; (D, G) = immunized three times with formalin-inactivated A/PC; (E, H) = survival after 20 days; * P < 0.05 vs. control naive group; Fisher's exact test.

**Figure 22** provides a series of graphs showing that a trivalent influenza vaccine failed to provide protection against drifted strains. (A, D) = naive (B, E) = immunized; (C, F) = survival after 20 days.

**Figure 23** provides representative photomicrographs of immunohistochemically stained lung tissue following homologous challenge. (A) = naive lung; (B) = unvaccinated (infected); (C) = gamma-A/PC vaccinated (challenged); (D) = formalin-A/PC vaccinated (challenged); E = UV-A/PC vaccinated (challenged).

**Figure 24** provides representative photomicrographs of immunohistochemically stained lung tissue following heterosubtypic challenge. (A) = naive lung; (B) = unvaccinated (infected); (C) = gamma-A/PC vaccinated (challenged); (D) = formalin-A/PC vaccinated (challenged); E = UV-A/PC vaccinated (challenged).

**Figure 25** is a graph illustrating that various inactivated virus preparations do not prevent influenza infection but immunization with γ-ray inactivated A/PC leads to early viral clearance.

**Figure 26** is a graph showing a comparison of Tc cell responses induced by live and inactivated A/PC. Mean values ± SD of two mice per group are shown. Specific lysis values were interpolated from regression curves at effector:target ratio of 50:1. N.D.: not detected.

**Figure 27** provides a series of graphs illustrating that intranasal immunization with γ-irradiated A/PC provides protection against high-dose A/PR8 lethal challenge. (A, C) = mice challenged with LD50 A/PR8; (B, D) = mice challenged with 5 x LD50 A/PR8; (E) = mice challenged with 50 x LD50 A/PR. (F) = survival and weight loss after 20 days. * P < 0.05 vs. control naive group; Fisher's exact test.

**Figure 28** provides a series of graphs illustrating that heterosubtypic protective properties of γ-irradiated A/PC are maintained after a dry freezing process. (A) = mock treated; (B) = challenged with heterosubtypic strain A/PR8; (C) = survival and weight loss after 20 days. * P < 0.05 vs. control naive group; Fisher's exact test.

**Figure 29** provides a series of graphs illustrating that heterosubtypic protective properties of γ-irradiated A/PC are maintained after a dry freezing process. (A) = mock treated; (B) = challenged with freeze-dried γ-ray inactivated A/PR8; (C) = survival and weight loss after 20 days. * P < 0.05 vs. control naive group; Fisher's exact test.

**Figure 30** shows a series of flow cytometry histograms indicating that γ-FLU, but not γ-SFV, induces lymphocyte activation. Shaded histograms represent expression levels in naive mice (control); white open (unshaded) histograms represent the proportion of splenocytes positive for the relevant activation marker.

**Figure 31** provides column graphs showing that γ-FLU, but not γ-SFV, induces elevated serum levels of IFN-α. Serum levels of IFN-α are represented as (Units/ml). Results are presented as mean ± SEM (n=3). (*) denotes statistical significance * p < 0.05, **p<0.01.

**Figure 32** provides a column graph showing that prior vaccination of mice with γ-SFV prevents vireamia upon secondary challenge with live SFV. Naive mice infected with SFV served as the positive control (Naive--SFV). Results are presented as mean ± SEM (n=2).

**Figure 33** shows a column graph indicating that vaccination with γ-SFV induces a dose dependent anti-SFV antibody response. Results are presented as mean ± SEM (n=3). (*) denotes statistical significance *** p < 0.001.

**Figure 34** provides a timecourse graph showing that γ-FLU acts synergistically to enhance the level of anti-SFV specific antibodies, if co-administed with γ-SFV. Results are presented as mean ± SEM (n=3). (*) denotes statistical significance *** p < 0.001.

**Figure 35** provides a timecourse graph showing that co-administration of γ-FLU with γ-SFV significantly enhances the kinetics and overall magnitude of the anti-SFV specific antibody response. Results are presented as mean ± SEM (n=3).

**Figure 36** shows a column graph demonstrating increased SFV neutralization by sera from mice immunized with γ-SFV and γ-FLU compared to mice vaccinated with γ-SFV alone. Neutralisation of 100 PFU was measured as a (%). Results are presented as mean ± SEM (n=3). (*) denotes statistical significance * p < 0.05.

**Figure 37** provides a column graph showing that co-administration of γ-FLU and γ-SFV has no significant effect on the level of anti-FLU specific antibody responses. Results are presented as mean ± SEM (n=3).

**Figure 38** provides a column graph showing that co-administration of γ-FLU and γ-SFV does not impact FLU-specific IgG titres. Results are presented as mean ± SEM (n=3).

## Definitions

As used in this application, the singular form "a", "an" and "the" include plural references unless the context clearly dictates otherwise. For example, the term "an immunogen" also includes a plurality of immunogens.

As used herein, the term "comprising" means "including." Variations of the word "comprising", such as "comprise" and "comprises," have correspondingly varied meanings. Thus, for example, a vaccine "comprising" an immunogen may consist exclusively of that immunogen or may include one or more additional substances (including additional immunogens).

The term "therapeutically effective amount" as used herein, includes within its meaning a non-toxic but sufficient amount a compound or composition for use in the present disclosure to provide the desired therapeutic effect. The exact amount required will vary from subject to subject depending on factors such as the species being treated, the age and general condition of the subject, the severity of the condition being treated, the particular agent being administered and the mode of administration and so forth. Thus, it is not possible to specify an exact "effective amount". However, for any given case, an appropriate "effective amount" may be determined by one of ordinary skill in the art using only routine experimentation.

Reference herein to immunogen(s) or vaccine(s) "against", "targeted against', or "targeted at" a particular disease or condition will be understood to mean that the immunogen(s) or vaccine(s) are capable of inducing a specific immune response against a causative agent of the disease or condition, when administered to a subject.

Any description of prior art documents herein, or statements herein derived from or based on those documents, is not an admission that the documents or derived statements are part of the common general knowledge of the relevant art.

## Detailed Description

Secondary infections and conditions associated with influenza infection are a significant cause of morbidity and mortality. The prevalence of these secondary complications can be attributed at least in part due to the inability of current vaccines to induce cross-protective immunity against heterologous influenza strains. Recipients of current vaccines are generally still susceptible to infection by many existing flu strains and have little if any immunity against newly emerging strains, some of which may form the basis of future flu pandemics. The protection offered by current vaccines is thus limited and this in turn contributes significantly to the prevalence of secondary infections and conditions associated with influenza infection. Compositions and methods of the present disclosure induce cross-protective immunity (i.e. immunity against heterologous flu strains) and are therefore useful for preventing and/or treating secondary infections or conditions associated with flu infection. Accordingly, certain embodiments of the present disclosure relate to compositions and methods for enhancing the immune responses against secondary infections or conditions associated with influenza infection.

Vaccination of individuals against certain causative agents (e.g. microorganisms, allergens etc.) may assist in preventing secondary infections and conditions upon influenza virus infection. However, the potential benefits of preemptive vaccination against secondary agent(s) may be compromised due to a number of factors arising upon influenza infection. Without restriction to particular mechanisms or modes of action, it is firstly postulated that the burden of an influenza virus infection means that many individuals may not have a sufficient threshold level of immunity required to protect against secondary infection, regardless of prior vaccination against the relevant agent(s). Secondly, it is believed that synergistic interactions occurring between influenza virus and secondary pathogens (e.g. bacteria) may create a favourable environment enhancing persistence of secondary pathogens even in the face of prior vaccination. Thirdly, is it hypothesised that the pro-inflammatory immune environment induced by influenza virus infection may reduce thresholds of allergen responsiveness (i.e. reduce the dosage of allergen needed to invoke an allergic immune response).

The capacity of vaccination to prevent secondary infections and conditions upon influenza virus infection is therefore limited. Accordingly, certain embodiments of the present disclosure relate to compositions and methods for enhancing the immune response induced by vaccines against secondary infections or conditions associated with influenza infection.

Given the prevalence of secondary infections and conditions in flu-infected individuals, treatments capable of providing immunity against both the influenza virus and secondary infections/conditions are particularly desirable. Accordingly, certain embodiments of the present disclosure relate to a synergistic combination of components capable of inducing immunity against both infection by influenza virus and at least one other agent causative of an associated secondary infection or condition. Without restriction to a particular mechanism or mode of action, it is believed that the component of the combination providing immunity against influenza virus (gamma-irradiated flu virus) provides an adjuvant effect for the additional component(s) that induce immunity against secondary infections and conditions. Hence, the present disclosure provides effective treatments capable of preventing or alleviating both influenza infection and secondary infections/conditions associated with influenza infection.

In addition, the present inventors have surprisingly identified that gamma-irradiated influenza viruses enhance immune responses induced by co-administered vaccines targeted at diseases and conditions that occur independently of influenza infection.

## Compositions

The present disclosure provides gamma-irradiated influenza viruses and compositions comprising the same. Compositions of the present disclosure may comprise immunogen(s) capable of stimulating an immune response against agent(s) causative of secondary infection(s) or condition(s) associated with influenza infection. In certain embodiments, compositions of the disclosure are preventative and/or therapeutic vaccines.

*Gamma-irradiated influenza virus*

A gamma-irradiated influenza virus in accordance with the present disclosure may be derived from a subtype of the genus influenza virus A (type A), influenza virus B (type B), or influenza virus C (type C). Also contemplated are inter-subtype recombinants.

Suitable subtypes of influenza virus A include, but are not limited to, H1N1 (e.g. H1N1 09 Swine Flu/pandemic influenza A (H1N1)), H1N2, H1N7, H2N2, H3N1, H3N2, H3N8, H4N8, H5N1 (e.g. HPAI A(H5N1)), H5N2, H5N3, H5N8, H5N9, H6N5, H7N1, H7N2, H7N3, H7N4, H7N7, H8N4, H9N2, H10N7, H11N6, H12N5, H13N6, H14N5, and any other recombinant virus arising from re-assortment between influenza A subtypes.

In certain embodiments, the virus is an H1N1 subtype virus. The H1N1 virus may be strain APR/8/34.

Influenza viruses for use in accordance with the present disclosure can be generated using methods known in the art. For example, influenza viruses may be derived by serial passaging in embryonated eggs as described, for example, in Coico et al., (Eds) (2007), "Current Protocols in Microbiology", John Wiley and Sons, Inc. (see in particular Unit 15G.1 entitled *"Influenza: Propagation, Quantification, and Storage").* A brief description of this technique is provided below.

Embryonated eggs may be obtained 9-12 days after fertilization and candled to locate the air sac. The egg may then be pierced under aseptic conditions, and the seed-virus inoculated into the air-space with a syringe. The procedure may be carried out manually or automatically by machines. The inoculated egg may then be incubated for approximately two to three days in a humidified atmosphere. At the end of this period, the egg can be maintained at approximately 4°C if desired in order to terminate the embryo and aid clarification of the allantoic fluid. The top of the egg may then be removed, the membrane pierced, and the allantoic fluid collected. Again this can be achieved manually, or by automated machinery. The allantoic fluid may be clarified, for example, by centrifugation to remove cell debris and/or subjected to further purification prior to or following inactivation of the influenza virus by gamma-irradiation. Purification of allantoic fluid may be achieved for example, by temperature-dependent adsorption to chicken red blood cells (CRBC), sucrose gradient, or dialysis.

Modifications of the above-mentioned process also suitable for the production of influenza virus are described, for example, in United States Patent No. 7,270,990, PCT publication No. WO 2002/067983 and PCT publication No. WO 2005/113756.

Additionally or alternatively, influenza virus for use in use in accordance with the present disclosure may be generated in cell culture (see, for example, Furminger, (1998), "Vaccine production", in Nicholson et al. (Eds.), "Textbook of Influenza", Blackwell Science, Oxford, pp. 324-332; Merten et al., (1996), "Production of influenza virus in cell cultures for vaccine preparation", in Cohen & Shafferman (Eds.), "Novel Strategies in Design and Production of Vaccines", pp. 141-151; United States Patent No. 5,824,536; and United States Patent No. 6,344,354).

Non-limiting examples of suitable cell lines that may be used as substrates for the growth of influenza virus include vero cells, Madin Darby canine kidney (MDCK) cells, PERC6 cells (see, for example, United States Patent No. 7,192,759), chicken embryo cells (e.g. chicken embryo fibroblasts) and avian embryonic cell lines (see, for example, PCT publication No. WO 2006/108846). Variants of these cell lines may be used, including, but not limited to, those described in United States Patent No. 6,825,036, United States Patent No. 6,455,298 and PCT publication No. WO 2006/108846.

Propagation of influenza virus using cell lines will, in general, involve expanding the cells to the desired quantity in a chemically defined medium. Preferably, the medium is a serum free medium. Propagation of the virus can be assisted by the addition of proteases to the medium. Generally, the cells are infected with influenza virus and incubated for a period of time sufficient to generate the required numbers of virus (e.g. several days). Parameters such as multiplicity of infection, incubation time and temperature will generally need to be optimised for the specific cell line used and/or specific influenza strain/s being propagated. The optimisation of growth parameters including those referred to above can be readily determined by a person of ordinary skill in the field without undue experimentation. Following the incubation period, the virus may be harvested and purified if so desired.

Non-limiting examples of processes suitable for the production of influenza virus in cell culture include those described in United States Patent No. 5,698,433, United States Patent No. 5,753,489, United States Patent No. 6,146,873, United States Patent No. 6,455,298 and United States Patent No. 6,951,752.

The yield of influenza virus production in cell culture may be enhanced, for example, by modifying cellular genes encoding the protein kinase PKR or (2'-5') oligoadenylate (2-5A) synthetase genes (see, for example, United States Patent No. 6,673,591 and United States Patent No. 6,686,190), or modifying the viral backbone with an alternative nonstructural protein 1 (NS1) gene (see, for example, PCT publication No. WO 2005/024039). Additionally or alternatively, cell lines utilised for the propagation of influenza virus may over-express sialyltransferase (see, for example, United States Patent No. 7,132,271).

Influenza virus propagated using the methods above (or by any other means) may be purified and/or concentrated prior to gamma-irradiation. Any suitable method known in the art may be used for this purpose. For example, influenza virus may be purified by temperature-dependent adsorption to chicken red blood cells using the method described in Laver, (1969), "Purification of influenza virus", HKaS NP (Ed), New York and London: Academic Press, pp. 82-86. Additionally or alternatively, influenza virus may be purified by density gradient centrifugation (see, for example, Sokolov et al., (1971), "Purification and concentration of influenza virus", Archiv fiir die gesarate Virusforschung, 35, 356-363).

In certain embodiments, influenza virus is purified and/or concentrated prior to gamma-irradiation using tangential/cross-flow filtration. For example, virus-containing fluid may be applied to a filtering device such as a membrane having an appropriate pore size (e.g. less than about 80nm). The fluid is pumped tangentially along the surface of the membrane (i.e. across the surface) and pressure applied to force a portion of the fluid through the membrane to the filtrate side. The applied pressure will generally be of a degree that does not adversely affect virion structure and/or the integrity of viral antigens. Filtrate containing viral particles passes through the membrane, whereas particulates and macromolecules in the fluid that are too large to pass through the membrane pores are retained on the opposing side. In general, retentate (i.e. retained components) does not build up at the surface of the membrane and is instead swept along by the tangential flow. The retentate may be re-diluted with appropriate media (e.g. PBS containing dextran and/or sucrose) and the filtration process repeated if required.

The use of tangential/cross-flow filtration to purify influenza virus used for gamma-irradiation provides an advantage over purification techniques currently used for influenza vaccine preparation (e.g. ultracentrifugation) as the integrity of viral antigens is better preserved. This in turn enhances the immunogenicity of gamma-irradiated viral preparations, and in particular their ability to elicit cross-protective immunity against heterologous influenza subtypes and strains.

Influenza viruses for use in accordance with the present disclosure are gamma-irradiated. Any suitable source of gamma-radiation may be used. Suitable gamma emitters include, but are not limited to $Ba^{117}$, $Co^{60}$, $Cs^{137}$, $Ir^{192}$, $U^{235}$, $Se^{75}$ and $Yb^{169}$.

Gamma-irradiation of influenza virus may be performed using commercially available devices, for example, a Gammacell irradiator manufactured by Atomic Energy of Canada Ltd., Canada (e.g. Gammacell 40 Irradiator, Gammacell 220 Irradiator, Gammacell 1000 irradiator, Gammacell 3000 irradiator), a gamma-irradiator manufactured by J. L. Shepherd and Associates (San Fernando, California, USA), or a Nordion Gamma Cell-1000 irradiator manufactured by Nordion Inc. (Kanata, Ontario, Canada). Other suitable devices are described, for example, in United States Patent No. 3,557,370 and United States Patent No. 3,567,938.

In general, the influenza virus is exposed to a dose of gamma-irradiation sufficient to inactivate the virus. Preferably, the dose of gamma-irradiation is sufficient to inactivate the virus without substantially disrupting the structure of viral antigens, and in particular without substantially disrupting the structure of viral surface antigens. The immunogenicity of antigenic determinants may therefore be retained by the gamma-irradiated virus. Preferably, the dose of gamma-irradiation is administered to the virus over a period of time and at a level sufficient to ensure that all viruses under treatment are exposed without adversely affecting the structural integrity of viral antigenic determinants.

Influenza virus for use in accordance with the present disclosure may be exposed to a total dose of gamma-irradiation in the range of about $1 \times 10^3$ rad and about $2 \times 10^9$ rad (or about 10 Gy to about $2 \times 10^4$ kGy). In certain embodiments of the present disclosure, influenza virus is exposed to a total dose of gamma-irradiation of between about $1 \times 10^3$ rad and about $2 \times 10^9$ rad, between about $1 \times 10^3$ rad and about $1 \times 10^9$ rad, between about $1 \times 10^3$ rad and about $1 \times 10^8$ rad, between about $1 \times 10^3$ rad and about $1 \times 10^7$ rad, between about $1 \times 10^3$ rad and about $1 \times 10^6$ rad, between about $1 \times 10^3$ rad and about $1 \times 10^5$ rad, between about $1 \times 10^3$ rad and about $1 \times 10^4$ rad, between about $1 \times 10^3$ rad and about $2 \times 10^9$ rad, between about $1 \times 10^4$ rad and about $2 \times 10^9$ rad, between about $1 \times 10^5$ rad and about $2 \times 10^9$ rad, between about $1 \times 10^6$ rad and about $2 \times 10^9$ rad, between about $1 \times 10^7$ rad and about $2 \times 10^9$ rad, between about $1 \times 10^8$ rad and about $2 \times 10^9$ rad or between about $1 \times 10^9$ rad and about $2 \times 10^9$ rad.

In one embodiment of the present disclosure, the influenza virus is exposed to a total dose of gamma-rays of between about $6.5 \times 10^4$ rad and about $2 \times 10^7$ rad (about 0.65 KGy to about 200 kGy). In preferred embodiments of the present disclosure, the influenza virus is exposed to a total gamma-irradiation dose of about $1.26 \times 10^6$ rad (12.6 KGy), a total gamma-irradiation dose of about $1 \times 10^6$ rad (about 10 kGy) gamma-rays or a total gamma-irradiation dose of about $1 \times 10^5$ rad (1 KGy).

The optimal dose of gamma-irradiation may be influenced by factors such as the medium in which the virus is present, the amount of virus to be treated, the temperature of the virus present, and/or the subtype or strain of virus under treatment. Accordingly, the total dose of gamma-irradiation, the exposure time and/or the level of gamma-irradiation applied over the period of exposure may be optimised to enhance the effectiveness of the treatment.

The total dose of gamma-irradiation may be administered to the virus cumulatively over a period of time. For example, gamma-irradiation may be administered to the virus at a level lower than that of the total dose, over a time period sufficient to achieve the total dose of gamma-irradiation required.

In one embodiment, influenza virus preparations are maintained in a frozen state while being exposed to gamma-irradiation. This may facilitate the preservation of biological integrity and avoid unnecessary damage of viral antigens thereby enhancing the immunogenicity of gamma-irradiated viral preparations, and in particular, their ability to elicit cross-reactive/cross-protective immunity against multiple influenza types, subtypes and strains. In general, a gamma-irradiation dose of 10-20kGy may be effective for treating frozen viral preparations.

As mentioned above, it is preferable that treatment with gamma-irradiation is sufficient to inactivate the influenza virus without substantially disrupting the structure of viral antigens. Inactivation of the virus may be assessed using methods generally known in the art. For example, viral infectivity can be measured following gamma-irradiation by inoculating embryonic eggs and/or cell lines as described in the paragraphs above to determine whether the virus is capable of propagation.

The integrity of antigenic determinants can be assessed, for example, by assaying the virus for hemagglutinating activity following gamma-irradiation. Methods of performing hemagglutination assays are known in the art and are described, for example, in Coico et al. (Eds), (2007), "Current Protocols in Microbiology", John Wiley and Sons, Inc. (see in particular Unit 15G.1 entitled *"Influenza: Propagation, Quantification, and Storage"*); and Sato et al., (1983), "Separation and purification of the hemagglutinins from Bordetella pertussis", Infect. Immun., 41, 313-320.

Additionally or alternatively, a neuraminidase assay may be used to assess the integrity of viral antigenic determinants (see, for example, Khorlin et al., (1970), "Synthetic inhibitors of Vibrio cholerae neuraminidase and neuraminidases of some influenza virus strains", FEBS Lett., 8:17-19; and Van Deusen et al., (1983), "Micro neuraminidase-inhibition assay for classification of influenza A virus neuraminidases", Avian Dis., 27:745-50).

Additionally or alternatively, cytotoxic T cell responses against the internal proteins inducible by the gamma-irradiated preparations can be used to as indicator for protein integrity.

*Immunogens*

Compositions and vaccines of the present disclosure may comprise immunogens that stimulate the immune response against at least one agent causative of an infection or condition. The infection or condition may be a secondary infection or condition associated with influenza infection. Alternatively, the infection or condition may occur independently of influenza infection. The compositions and vaccines additionally comprise gamma-irradiated influenza viruses.

As contemplated herein, a secondary infection associated with influenza infection (also referred to hereinafter as "secondary infection(s)" and "secondary infection(s) following influenza virus infection") includes any infection that may occur concurrently with influenza virus infection and/or in the finite period following influenza virus clearance in which the immune system and innate clearance mechanisms of the respiratory tract have not fully recovered to optimal function, and in which the host remains more susceptible to secondary infections. For example, the secondary infection may occur within 7, 10, 14, 17, 21, 24, 28, 31, 35, 38, or 42 days of an influenza virus infection. Influenza infection is known to cause destruction of ciliated epithelial cells that can take, for example, up to six weeks to be replaced. Thus at any time during this period a subject may be more susceptible to secondary infections. Non-limiting examples of secondary infections include pneumonia (e.g. bacterial, viral, fungal pneumonia), chronic obstructive pulmonary disease, sinusitis, otitis media, bronchitis and the common cold.

In accordance with the present disclosure an infection (e.g. a secondary infection associated with influenza infection) may be caused by any microorganism. For example, the infection may arise from colonisation of the host by bacteria, fungi, viruses, and/or parasites (e.g. nematodes, protozoa and cestodes). Typically, microorganisms causative of a secondary infection are those which infect via mucosal surfaces although it will be understood that this is not a requirement. In certain embodiments the microorganisms are causative of a respiratory infection.

Non-limiting examples of bacteria that may be causative of an infection according to the present disclosure, which in some embodiments may be a secondary infectionassociated with influenza infection, include: *Acinetobacter* sp., *Actinomyces* sp., *Bacillus* sp. (e.g. *B. anthracis*), *Bacteroides* sp. (e.g. *Bacteroides melaninogenicus*), *Burkholderia* sp. (e.g. *B. pseudomallei, B. mallei*), *Bordetella* sp. (e.g. *B. pertussis),* *Branhamella* sp. *(e.g. B. catarrhalis*), *Chlamydia* sp. (e.g. *C. trachomatis, C. psittaci, C. pneumoniae),* *Corynebacterium* sp., (e.g. *C. diphtheriae), Coxiella* sp. (e.g. *C. burnetii, Enterobacteriaceae* (e.g. *Klebsiella pneumoniae),* *Francisella sp.* (e.g. *F. tularensis),* *Fusobacterium* (e.g. *Fusobacterium nucleatum),* *Haemophilus* sp. (e.g. *H. influenzae),* *Legionella* sp. (e.g. *Legionella pneumophila),* *Moraxella* sp. (e.g. *M. catarrhalis),* *Mycobacterium* sp. (e.g. *M. tuberculosis),* *Mycoplasma sp.* (e.g. *M. pneumoniae),* *Neisseria* sp. (e.g. *Neisseria meningitides) Nocardia* sp. (e.g. *N. asteroids),* *Peptostreptococcus* sp., *Peptococcus* sp., *Pseudomonas* sp. (e.g. *P. aeruginosa),* *Staphylococcus* sp. (e.g. *S. aureus),* *Streptococcus* sp. (e.g. *S. pneumoniae, S. pyogenes, S. agalactiae*), and *Yersinia* sp. (e.g. *Yersinia pestis*).

Non-limiting examples of viruses that may be causative of an infection according to the present disclosure, which in some embodiments may be a secondary infectionassociated with influenza infection, include: adenoviruses, coronaviruses, coxsackieviruses, cytomegaloviruses, echoviruses, Epstein-Barr viruses, herpes simplex viruses, influenza viruses (i.e. superinfection by additional different strains), measles viruses, myxoviruses, parainfluenza viruses, picornaviruses, respiratory syncytial viruses, rhinoviruses, togaviruses (e.g. semliki forest virus) and Varicella-Zoster viruses.

Non-limiting examples of fungi that may be causative of infection according to the present disclosure, which in some embodiments may be a secondary infection associated with influenza infection, include: *Aspergillus* sp., *Blastomyces* sp. (e.g. *B. dermatitidis*), *Candida* sp., *Cryptococcuss* sp. (e.g. *Cryptococcus neoformans*), *Histoplasma* sp. (e.g. *H. capsulatum),* *Coccidioides* sp. (e.g. *C. immitis, C. posadasii),* *Cryptococcus* sp. (e.g. *C. neoformans, C. diphtheriae*), *Mucorales* sp., *Paracoccidioides* sp. (e.g. *P. brasiliensis*) and *Pneumocystis* sp. (*P. carinii*).

Non-limiting examples of parasites that may be causative of infection according to the present disclosure, which in some embodiments may be a secondary infection associated with influenza infection, include: protozoa (e.g. *Plasmodium falciparum, Entamoeba histolytica, Toxoplasma gondii, Leishmania donovani),* nematodes (e.g. *Ascaris lumbricoides, Toxocara* sp., *Ancyclostoma duodenale*) and cestodes (e.g. *Echinococcus granulosus).*

A secondary condition "associated with influenza infection" (also referred to hereinafter as "secondary condition(s)") includes any condition arising from a non-infectious agent that may occur concurrently with influenza virus infection and/or in the finite period following influenza virus clearance in which the immune system and innate clearance mechanisms of the respiratory tract have not fully recovered to optimal function, and in which the host remains more susceptible to developing secondary conditions. For example, the secondary condition may arise within 7, 10, 14, 17, 21, 24, 28, 31, 35, 38, or 42 days after infection with the influenza virus. Non-limiting examples of secondary conditions include asthma, hay fever, allergic rhinitis, allergic sinusitis and the like.

A condition in accordance with the present disclosure, may be an allergic condition. In some embodiments, the allergic condition may be a secondary condition associated with influenza infection. Typically, the allergic condition is respiratory although it will be understood that non-respiratory allergic conditions are also contemplated.

Compositions and vaccines of the present disclosure may comprise immunogens against any agent. In some embodiments, the agent may be causative of a secondary infection or condition associated with influenza infection. An "immunogen" as contemplated herein encompasses any molecule capable of stimulating an immune response in a given host. Accordingly, an immunogen against an agent causative of a secondary infection or condition associated with influenza infection (i.e. also referred to hereinafter as an "immunogen against a secondary agent") encompasses any substance capable of inducing an immune response against that agent. An immune response stimulated by an immunogen of the present disclosure (e.g. and immunogen against a secondary agent) may induce one or more elements of innate immunity and/or one or more elements of adaptive immunity (e.g. humoral, cell-mediated immunity).

Non-limiting examples of suitable immunogens include whole microorganisms (e.g. live, attenuated or killed) or component(s) thereof, proteins (e.g. membrane proteins, coat proteins, cytoplasmic proteins), protein fragment(s), peptides, glycoproteins, glycolipids, polysaccharides (carbohydrates) or lipopolysaccharide polysaccharides, toxins and nucleic acids (e.g. DNA). In certain embodiments, the immunogen is a whole microorganism attenuated or killed by gamma-irradiation. Suitable methods for gamma-irradiating the microorganism are described in the subsection above entitled *"Gamma-irradiated influenza virus".*

The immunogen may induce an immune response against an agent causative of an allergic condition. Accordingly, in certain embodiments the immunogen is an allergen. Non-limiting examples of such allergens include pollen (e.g. from rye grass), mould, house dust mite *(Dermatophagoides pteronyssinus),* dust, protein allergens from animals such as dogs, mice, rabbits, horses and cockroaches (e.g. lipocalin), milk proteins, bee venom and chemical allergens.

In certain embodiments, the immunogen is a self-antigen (i.e. an antigen that although being a normal constituent of the host results in triggering of cell-mediated and/or humoral immune responses in the host).

An immunogen in accordance with the present disclosure may be obtained or produced using methods known to those of ordinary skill in the art.

For example, the immunogen may be a cultured microorganism (e.g. live, attenuated or killed) or a component of a cultured microorganism. Methods for the culture of microorganisms are known in the art and described, for example, in Coico et al. (Eds), (2000-2010), "Current Protocols in Microbiology", John Wiley & Sons, Inc.

Immunogenic components (e.g. proteins, nucleic acids etc.) may be isolated and purified from microorganisms (including naturally occurring or cultured microorganisms) using methods such as those described in Ausubel et al. (Eds), (2000-2010), "Current Protocols in Molecular Biology", John Wiley & Sons, Inc; and Coligan et al., (2000-2010), "Current Protocols in Protein Science", John Wiley and Sons.

In certain embodiments, allergens for use as immunogens in accordance with the present disclosure are obtained from natural source(s) and concentrated and/or purified for use.

In other embodiments, immunogens for use in accordance with the present disclosure are produced by recombinant methods.

Recombinant methods are known to those of ordinary skill in the art. For example, recombinant protein and polypeptide immunogens may be produced using techniques described in standard texts such as Sambrook et al., (1989), "Molecular Cloning: A Laboratory Manual', (2nd ed., Cold Spring Harbor Laboratory Press, Plainview, New York; Ausubel et al. (Eds), (2000-2010), "Current Protocols in Molecular Biology", John Wiley and Sons, Inc; Coligan et al. (Eds), (2000-2010), "Current Protocols in Protein Science", John Wiley and Sons, Inc; and Pharmacia Biotech., (1994), "The Recombinant Protein Handbook", Pharmacia Biotech.

Additionally or alternatively, immunogens for use in accordance with the present disclosure may be produced by chemical synthesis.

For example, protein and polypeptide immunogens of the present disclosure may be synthesised by solid phase chemistry techniques (see, for example, Steward et al., (1963), in "Solid Phase Peptide Synthesis", H. Freeman Co.,

San Francisco; Meienhofer, (1973), in "Hormonal Proteins and Peptides", volume 2, 46) or by classical solution synthesis (see, for example, Schroder et al. (1965), in "The Peptides", volume 1, 72-75, Academic Press (New York). In general, such methods comprise the addition of one or more amino acids or suitably protected amino acids to a growing sequential polypeptide chain on a polymer. Typically, either the amino or carboxyl group of the first amino acid is protected by a suitable protecting group. The protected and/or derivatised amino acid is then either attached to an inert solid support or utilized in solution by adding the next amino acid in the sequence having the complimentary (amino or carboxyl) group suitably protected and under conditions suitable for forming the amide linkage. The protecting group may then be removed from the newly added amino acid residue and the next amino acid (suitably protected) is then added to form a growing polypeptide chain.

Nucleic acid immunogens of the present disclosure may be manufactured by chemical synthesis techniques known in the art including, but not limited to, the phosphodiester and phosphotriester methods (see, for example, Narang et al., (1979), "Improved phosphotriester method for the synthesis of gene fragments", Meth. Enzymol. 68:90; Brown et al. (1979), "Chemical Synthesis and Cloning of a Tyrosine tRNA Gene", Meth. Enzymol. 68:109-151; and United States Patent No. 4,356,270) and the diethylphosphoramidite method (see Beaucage and Caruthers, (1981), "Deoxynucleotide phosphoramidite", Tetrahedron Letters, 22:1859-1862). A method for synthesising oligonucleotides on a modified solid support is described in United States Patent No. 4,458,066.

In certain embodiments, immunogen(s) (including immunogen(s) against secondary agent(s)) are administered as vaccines either alone or in combination with gamma-irradiated flu viruses. Methods for the preparation of vaccines are well known in the art and no limitation exists regarding the particular type of vaccine or its method of administration. Non-limiting examples of vaccines in accordance with the present disclosure and methods for their preparation are described below in the subsection entitled *"Vaccines"*. For example, a vaccine of the present disclosure may be a live, attenuated or killed whole organism vaccine, a subunit vaccine, a split vaccine, a conjugate vaccine, a toxoid vaccine, a DNA vaccine, or a recombinant vector vaccine. Preferably, the vaccine is formulated for intranasal administration.

The level of immunogenicity induced by a composition or vaccine of the present disclosure may be determined by measuring an immune response of a subject to which it has been administered. The immune response to a composition of the present disclosure may be measured, for example, by analysis of antibody production, cellular, proliferative and/or cytotoxic responses, and/or cytokines secretion. Non-limiting examples of specific assays for the measurement of immune responses include solid-phase heterogeneous assays (e.g. enzyme-linked immunosorbent assay), solution phase assays (e.g. electrochemiluminescence assay) and amplified luminescent proximity homogeneous assays. Other non-limiting examples include flow cytometry, intracellular cytokine staining, functional T-cell assays functional B-cell assays, functional monocyte-macrophage assays, dendritic and reticular endothelial cell assays, NK cell response, oxidative burst assays, and phagocytosis and apoptosis evaluation.

Compositions and vaccines of the present disclosure may be multivalent or monovalent. Accordingly, in certain embodiments compositions and vaccines of the present disclosure comprise single or multiple strains of gamma-irradiated influenza virus.

In other embodiments, compositions and vaccines of the present disclosure comprise a single gamma-irradiated strain of influenza virus in combination with a single type of immunogen against a secondary agent.

In additional embodiments, compositions and vaccines of the present disclosure comprise a multiple strains of gamma-irradiated virus in combination with a multiple different immunogens (e.g. multiple immunogens against secondary agent(s)). The immunogens may be against single or multiple agents causative of infection, including secondary infection(s) or condition(s) associated with influenza infection.

*Synergistic combinations*

The present disclosure provides a synergistic combination comprising at least one gamma-irradiated influenza virus strain and at least one additional immunogen.

The additional immunogen is an agent capable of inducing an immune response against a particular disease or condition. The gamma-irradiated influenza virus may be capable of enhancing the immunogenicity of the additional immunogen when the two are co-administered. A subject co-administered the gamma-irradiated influenza virus and additional immunogen may benefit from an enhanced immune response against the disease or condition targeted by the immunogen regardless of whether or not the disease or condition is associated with influenza infection.

Accordingly, is some embodiments gamma-irradiated influenza viruses of the present disclosure may enhance the immunogenicity of a vaccine or immunogen against a disease or condition that arises independently of influenza infection.

In other embodiments, the gamma-irradiated influenza viruses may enhance the immunogenicity of a vaccine or immunogen targeting a secondary infection or condition associated with influenza infection. In such cases, the synergistic combination may be capable of inducing immunity against the influenza virus and enhancing the immune response against the secondary infection or condition induced by the vaccine or immunogen targeting it. The secondary infection or condition may arise within 7, 10, 14, 17, 21, 24, 28, 31, 35, 38, or 42 days from an influenza infection.

Certain embodiments of the present disclosure relate to compositions and vaccines comprising the synergistic combination. Other embodiments of the present disclosure relate to methods for the production of the compositions and vaccines. Additional embodiments of the present disclosure relate to methods of prophylactic and/or therapeutic treatment using the compositions and vaccines.

Without restriction to a particular mechanism or mode of action, it is believed that the component in the combination providing immunity against influenza virus (i.e. gamma-irradiated influenza virus) provides an adjuvanting effect on the second component of the combination (i.e. the immunogen). This adjuvanting effect is postulated to arise from component(s) present in the gamma-irradiated virus of the combination vaccine. Accordingly, the immunogenicity induced by the immunogen against a target disease or condition (e.g. a secondary infection or condition associated with influenza infection) in synergistic combinations of the present disclosure is greater than that gained from the additive effect of each component used independently of the other.

It will be understood that a synergistic combination of the present disclosure may comprise any gamma-irradiated influenza type/subtype/strain or any number of different gamma-irradiated influenza types/subtypes/strains. Non-limiting examples of suitable influenza types, subtypes and strains along with methods for their preparation are provided in the preceding subsection entitled *"Gamma-irradiated influenza viruses"*. In certain embodiments, the gamma-irradiated viruses of the synergistic combination are H1N1 subtype viruses. The H1N1 viruses may be strain APR/8/34.

A synergistic combination of the present disclosure may comprise any immunogen or any number of different immunogens against a target disease or condition. In certain embodiments, the immunogen may be agent(s) causative of secondary infection(s) or condition(s) associated with influenza infection (i.e. immunogens against secondary agent(s)). Non-limiting examples of suitable immunogens are provided in the preceding subsection entitled *"Immunogens"*.

In certain embodiments, the immunogen(s) induce immunity in a recipient against microorganisms (e.g. bacteria, viruses, fungi, or parasites).

In other embodiments, the immunogen(s) induce immunity in a recipient against allergens.

Compositions and vaccines of the present disclosure comprising synergistic combinations may be used prophylactically (i.e. preventative) or therapeutically (i.e. post-infection) against infection(s) or condition(s) including, but not limited to, secondary infection(s) or condition(s) associated with influenza infection.

Non-limiting examples of secondary infections associated with influenza infection include pneumonia (e.g. bacterial, viral, fungal), chronic obstructive pulmonary disease, sinusitis, otitis media, bronchitis and the common cold.

Non-limiting examples of secondary conditions associated with influenza infection include asthma, hay fever, allergic rhinitis, allergic sinusitis and the like.

It will be understood that synergistic combinations of the present disclosure can be used to treat any one or more infections or conditions provided that a suitable immunogen targeted against the infection or condition is included in the composition.

Further, it will be recognised that individual components of the synergistic combinations described herein may be administered to a subject simultaneously or sequentially.

*Enhancement of immune responses to co-administered immunogens*

Gamma-irradiated influenza viruses of the present disclosure may be co-administered with other vaccines or immunogens to enhance the immunogenicity of those other vaccines or immunogens.

The co-administered vaccines and immunogens may be targeted against any disease or condition. The disease or condition may arise independently of influenza infection. Additionally or alternatively, the disease or condition may be a secondary infection or condition associated with influenza infection. The secondary infection or condition may arise within 7, 10, 14, 17, 21, 24, 28, 31, 35, 38, or 42 days of an influenza infection.

Gamma-irradiated influenza viruses of the present disclosure may be used to enhance the immunogenicity induced by any immunogen, suitable examples of which are set out above in the subsection entitled *"Immunogens"*. For example, the immunogen may induce immunity in a recipient against microorganisms (e.g. bacteria, viruses, fungi, or parasites). Additionally or alternatively, the immunogen may induce immunity in a recipient against an allergen.

In some embodiments, the co-administered vaccine or immunogen may be a viral vaccine or immunogen. Non-limiting examples of suitable viruses that the co-administered vaccine may target include adenoviruses, coronaviruses, coxsackieviruses, cytomegaloviruses, echoviruses, Epstein-Barr viruses, herpes simplex viruses, influenza viruses, measles viruses, myxoviruses, parainfluenza viruses, picornaviruses, respiratory syncytial viruses, rhinoviruses, togaviruses (e.g. semliki forest virus) and Varicella-Zoster viruses.

In some embodiments, gamma-irradiated influenza viruses of the present disclosure may be used to provide an adjuvant effect for a co-administered vaccine or immunogen by inducing a range of immunomodulatory effects including, but not limited to, the induction of an interferon (IFN) type I response (e.g. IFN-$\alpha$).

Additionally or alternatively, the gamma-irradiated influenza viruses may be used to provide an adjuvant effect for a co-administered vaccine or immunogen that induces antigen-specific antibody responses (e.g. antigen specific IgG responses).

It will be understood that reference to co-administration of gamma-irradiated influenza viruses with other vaccines and immunogens encompasses both simultaneous and sequential administration.

*Pharmaceutical compositions*

The present disclosure provides compositions comprising gamma-irradiated influenza viruses and/or additional immunogen(s). The additional immunogen(s) are capable of stimulating the immune response against agent(s) causative of a target disease or condition (e.g. a secondary infection or condition associated with influenza infection). Non-limiting examples of suitable gamma-irradiated influenza viruses, immunogens against secondary agents, and methods for their preparation are described above in the subsections entitled *"Gamma-irradiated influenza viruses"* and *"Immunogens"*.

In certain embodiments, compositions of the present disclosure are pharmaceutical compositions, non-limiting examples of which include preventative and/or therapeutic vaccines. Pharmaceutical compositions of the present disclosure may be prepared using methods known to those of ordinary skill in the art. Non-limiting examples of suitable methods are described in Gennaro et al. (Eds), (1990), "Remington's Pharmaceutical Sciences", Mack Publishing Co., Easton, Pennsylvania, USA.

A pharmaceutical composition of the present disclosure may be administered to a recipient in isolation or in combination with other additional therapeutic agent(s). In embodiments where a pharmaceutical composition is administered with therapeutic agent(s), the administration may be simultaneous or sequential (i.e. pharmaceutical composition administration followed by administration of the agent(s) or *vice versa*).

Pharmaceutical compositions of the present disclosure may comprise a pharmaceutically acceptable carrier, excipient, diluent and/or adjuvant. "Pharmaceutically acceptable" carriers, excipients, diluents and/or adjuvants as contemplated herein are substances which do not produce adverse reaction(s) when administered to a particular recipient such as a human or non-human animal. Pharmaceutically acceptable carriers, excipients, diluents and adjuvants are generally also compatible with other ingredients of the composition. Non-limiting examples of suitable excipients, diluents, and carriers can be found in the *"Handbook of Pharmaceutical Excipients"* 4th Edition, (2003) Rowe et al. (Eds), The Pharmaceutical Press, London, American Pharmaceutical Association, Washington.

Non-limiting examples of pharmaceutically acceptable carriers, excipients or diluents include demineralised or distilled water; saline solution; vegetable based oils such as peanut oil, safflower oil, olive oil, cottonseed oil, maize oil, sesame oils, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethylcellulose; lower alkanols, for example ethanol or isopropanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrridone; agar; carrageenan; gum tragacanth or gum acacia, and petroleum jelly. Typically, the carrier or carriers will form from 10% to 99.9% by weight of the compositions.

Compositions of the present disclosure can be administered to a recipient by standard routes, including, but not limited to, parenteral (e.g., intravenous, intraspinal, subcutaneous or intramuscular), oral, mucosal (e.g. intranasal) or topical routes.

Accordingly, compositions of the present disclosure may be in a form suitable for administration by injection, in the form of a formulation suitable for oral ingestion (such as capsules, tablets, caplets, elixirs, for example), in the form of an ointment, cream or lotion suitable for topical administration, in a form suitable for delivery as an eye drop, in an aerosol form suitable for administration by inhalation, such as by intranasal inhalation or oral inhalation, or in a form suitable for parenteral administration, that is, subcutaneous, intramuscular or intravenous injection.

Solid forms of compositions of the present disclosure for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms of compositions of the present disclosure for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions comprising compositions of the disclosure for oral administration may further comprise dispersing agents and/or suspending agents. Suitable suspending agents include sodium carboxymethylcellulose, methylcellulose, hydroxypropylmethyl-cellulose, poly-vinyl-pyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or -laurate, polyoxyethylene sorbitan mono- or di-oleate, -stearate or -laurate and the like.

For preparation of compositions as injectable solutions or suspensions, non-toxic parenterally acceptable diluents or carriers may be used such as Ringer's solution, isotonic saline, phosphate buffered saline, ethanol and 1,2 propylene glycol.

Emulsions for oral administration may further comprise one or more emulsifying agents. Suitable emulsifying agents include dispersing agents as exemplified above or natural gums such as guar gum, gum acacia or gum tragacanth.

Topical formulations of the present disclosure comprise an active ingredient(s) (e.g. gamma-irradiated influenza viruses and/or immunogen(s)) together with one or more acceptable carriers, and optionally any other therapeutic ingredients. Formulations suitable for topical administration include liquid or semi-liquid preparations suitable for penetration through the skin to the site of where treatment is required, such as liniments, lotions, creams, ointments or pastes, and drops suitable for administration to the eye, ear or nose.

Drops according to the present disclosure may comprise sterile aqueous or oily solutions or suspensions. These may be prepared by dissolving the active ingredient in an aqueous solution of a bactericidal and/or fungicidal agent and/or any other suitable preservative, and optionally including a surface active agent. The resulting solution may then be clarified by filtration, transferred to a suitable container and sterilised. For example, sterilisation may be achieved by filtration followed by transfer to a container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present disclosure include those suitable for application to the skin or eye. An eye lotion may comprise a sterile aqueous solution optionally containing a bactericide and may be prepared by methods similar to those described above in relation to the preparation of drops. Lotions or liniments for application to the skin may also include an agent to hasten drying and to cool the skin, such as an alcohol or acetone, and/or a moisturiser such as glycerol, or oil such as castor oil or arachis oil.

Creams, ointments or pastes according to the present disclosure are semi-solid formulations of the active ingredient for external application. They may be made by mixing the active ingredient in finely-divided or powdered form, alone or in solution or suspension in an aqueous or non-aqueous fluid, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogols.

Compositions of the present disclosure may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silicaceous silicas, and other ingredients such as lanolin, may also be included.

Compositions of the present disclosure may be administered in the form of liposomes. Liposomes are generally derived from phospholipids or other lipid substances, and are formed by mono- or multi-lamellar hydrated liquid crystals that are dispersed in an aqueous medium. Any non-toxic, physiologically acceptable and metabolisable lipid capable of forming liposomes can be used. The compositions in liposome form may contain stabilisers, preservatives, excipients and the like. The preferred lipids are the phospholipids and the phosphatidyl cholines (lecithins), both natural and synthetic. Methods to form liposomes are known in the art, and in relation to this specific reference is made to: Prescott, Ed., *Methods in Cell Biology*, Volume XIV, Academic Press, New York, N.Y. (1976), p. 33 *et seq.*

Supplementary active ingredients, such as adjuvants or biological response modifiers, can also be incorporated into compositions of the disclosure.

Preferably, an adjuvant will enhance the immune response induced and/or enhanced by component(s) of a given composition thereby improving protective efficacy. Preferably, the adjuvant will enable the induction of protective immunity utilising a lower dose of other active component(s) (e.g. gamma-irradiated influenza viruses and/or immunogens against agent(s) causative of an infection or condition).

Any suitable adjuvant may be included in a composition of the present disclosure. For example, an aluminium-based adjuvant may be utilised. Suitable aluminium-based adjuvants include, but are not limited to, aluminium hydroxide, aluminium phosphate and combinations thereof. Other specific examples of aluminium-based adjuvants that may be utilised are described in European Patent No. 1216053 and United States Patent No. 6,372,223.

Oil in water emulsions may be utilised as adjuvants in compositions of the present disclosure. Oil in water emulsions are well known in the art. In general, the oil in water emulsion will comprise a metabolisable oil, for example, a fish oil, a vegetable oil, or a synthetic oil. Examples of suitable oil in water emulsions include those described in European Patent No. 0399843, United States Patent No. 7,029,678 and PCT Publication No. WO 2007/006939. The oil in water emulsion may be utilised in combination with other adjuvants and/or immunostimulants.

Non-limiting examples of other suitable adjuvants include immunostimulants such as granulocyte-macrophage colony-stimulating factor (GM-CSF), monophosphoryl lipid A (MPL), cholera toxin (CT) or its constituent subunit, heat labile enterotoxin (LT) or its constituent subunit, toll-like receptor ligand adjuvants such as lipopolysaccharide (LPS) and derivatives thereof (e.g. monophosphoryl lipid A and 3-Deacylated monophosphoryl lipid A), muramyl dipeptide (MDP) and F protein of Respiratory Syncytial Virus (RSV).

Adjuvants in compositions of the present disclosure typically include emollients, emulsifiers, thickening agents, preservatives, bactericides and buffering agents. Another type of "self adjuvant" is provided by the conjugation of immunogenic peptides to lipids such as the water soluble lipopeptides Pam3Cys or its dipalmitoyl derivative Pam2Cys. Such adjuvants have the advantage of accompanying and immunogenic component into the antigen presenting cell (such as dendritic cells) and thus producing enhanced antigen presentation and activation of the cell at the same time. These agents act at least partly through toll-like receptor 2 (see, for example, Brown and Jackson, (2005), "Lipid based self adjuvanting vaccines", Current Drug Delivery, 23:83).

Suitable adjuvants are commercially available such as, for example, Freund's Incomplete Adjuvant and Complete Adjuvant (Difco Laboratories, Detroit, Mich.); Merck Adjuvant 65 (Merck and Company, Inc., Rahway, N.J.); AS-2 (SmithKline Beecham, Philadelphia, Pa.); aluminium salts such as aluminium hydroxide gel (alum) or aluminium phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

In certain embodiments, an adjuvant included in a composition of the present disclosure may induce an immune response predominantly of the TH1 type. Suitable adjuvants for use in eliciting a predominantly TH1-type response include, for example, a combination of monophosphoryl lipid A, preferably 3-de-O-acylated monophosphoryl lipid A (3D-MPL) together with an aluminium salt. For example, the composition or vaccine may be formulated with adjuvant AS04 containing aluminium hydroxide (alum) and 3-O-deacylated monophosphorylated lipid A (MPL) such as described in Thoelen et al. (2001), "A prophylactic hepatitis B vaccine with a novel adjuvant system", Vaccine, 19:2400-2403. Other known adjuvants which preferentially induce a TH1 type immune response include CpG containing oligonucleotides. The oligonucleotides are characterised in that the CpG dinucleotide is unmethylated. Such oligonucleotides are known to those of ordinary skill in the field and are described, for example, in PCT Publication No. WO 1996/02555. Immunostimulatory DNA sequences are also described, for example, in Sato et al., (1996), "Immunostimulatory DNA sequences necessary for effective intradermal gene immunization", Science, 273:352-354.

Another example of an adjuvant is a saponin, preferably QS21 (Aquila Biopharmaceuticals Inc., Framingham, Mass.), which may be used alone or in combination with other adjuvants. For example, an enhanced adjuvant system may be utilised involving the combination of a monophosphoryl lipid A and saponin derivative, such as the combination of QS21 and 3D-MPL as described in PCT Publication No. WO 1994/00153, or a less reactogenic composition where the QS21 is quenched with cholesterol, as described in PCT publication No. WO 1996/33739. Other alternative formulations comprise an oil-in-water emulsion and tocopherol. An adjuvant formulation involving QS21, 3D-MPL and tocopherol in an oil-in-water emulsion is described in PCT Publication No. WO 1995/17210. An adjuvant included in a composition of the disclosure may include a formulation involving QS21, 3D-MPL and tocopherol in an oil in water emulsion such as described in PCT publication No. WO 1995/17210. In one embodiment a composition of the disclosure comprises the adjuvant Montanide ISA720 (M-ISA-720; Seppic, Fairfield, N.J.), an adjuvant based on a natural metabolisable oil.

*Vaccines*

The present disclosure provides vaccine compositions comprising gamma-irradiated influenza viruses and/or additional immunogen(s). The additional immunogen(s) are capable of stimulating the immune response against agent(s) causative of a target disease or condition (e.g. a secondary infection or condition associated with influenza infection).

Vaccine compositions of the present disclosure may be administered to naive recipients, being individuals seronegative for particular target strain(s) of influenza and/or seronegative for a particular agent causative of a different disease or condition targeted by the vaccine composition (e.g. secondary agent(s) associated with influenza infection). Alternatively, the vaccine compositions may be administered to primed recipients, being individuals seropositive for particular target strain(s) of influenza and/or seropositive for a particular agent causative of a different disease or condition targeted by the vaccine composition (e.g. secondary agent(s) associated with influenza infection).

Vaccine compositions of the present disclosure include both preventative vaccines (i.e. vaccines administered for the purpose of preventing infections and/or conditions) and therapeutic vaccines (i.e. vaccines administered for the purpose of treating infections and/or conditions). A vaccine of the present disclosure may therefore be administered to a recipient for prophylactic, ameliorative, palliative, or therapeutic purposes.

Without placing any limitation regarding the particular form, a vaccine composition of the present disclosure may be a live, attenuated or killed whole organism vaccine, a subunit vaccine, a split vaccine, a conjugate vaccine, a toxoid vaccine, a DNA vaccine, a recombinant vector vaccine, or a combination of any two or more of the aforementioned.

In certain embodiments, vaccine compositions of the present disclosure comprise gamma-irradiated influenza viruses. In other embodiments, vaccine compositions of the present disclosure comprise immunogens against at least one agent causative of an infection or condition (e.g. a secondary infection or condition associated with influenza infection). In additional embodiments, vaccine compositions of the present disclosure comprise a synergistic combination of gamma-irradiated influenza viruses and immunogens against at least one agent causative of an infection or condition (e.g. a secondary infection or condition associated with influenza infection). Non-limiting examples of suitable gamma-irradiated influenza viruses, immunogens against secondary agents, and methods for their preparation are described above in the subsections entitled *"Gamma-irradiated influenza viruses"* and *"Immunogens"*.

Vaccine compositions of the present disclosure may be prepared according to standard methods known to those of ordinary skill in the art. Methods for vaccine preparation are generally described in Voller et al., (1978), "New Trends and Developments in Vaccines", University Park Press, Baltimore, Maryland, USA.

Non-limiting examples of suitable pharmaceutically acceptable excipients, diluents, carriers and adjuvants that may be included in vaccine compositions are provided in the subsection above entitled *"Pharmaceutical compositions"*.

Although adjuvant(s) may be included in vaccine compositions of the present disclosure, experimental data provided herein demonstrates that gamma-irradiated influenza viruses can enhance the immunogenicity of co-administered vaccines targeting other diseases and conditions, regardless of whether those other diseases and conditions arise as a consequence of influenza infection. Vaccine compositions of the present disclosure comprising gamma-irradiated influenza viruses need not necessarily comprise or be administered with other dedicated adjuvant component(s), which may allow reactogenicity problems that can arise from using dedicated adjuvant component(s) to be avoided.

In general, adjuvant activity in the context of a vaccine composition includes, but is not limited to, the ability to enhance the immune response (quantitatively or qualitatively) induced by immunogenic components in the vaccine (e.g. gamma-irradiated influenza virus and/or immunogens against secondary agents). This may reduce the dose or level of the immunogenic components required to produce an immune response and/or reduce the number or the frequency of immunisations required to produce the desired immune response.

*Routes of administration*

Compositions of the present disclosure (including vaccines) can be administered to a recipient by standard routes, including, but not limited to, parenteral (e.g. intravenous, intraspinal, subcutaneous or intramuscular), oral, topical, or mucosal routes (e.g. intranasal).

Preferably, vaccine compositions of the present disclosure are administered by the mucosal route. Non-limiting examples of acceptable routes of mucosal vaccine administration including intranasal, occular, buccal, genital tract (vaginal), rectal, intratracheal, skin, and the gastrointestinal tract.

Preferably, vaccine compositions of the disclosure are administered by the intranasal route. Without limitation to theory or particular mode(s) of action, intranasal administration of vaccine compositions of the present disclosure is believed to be advantageous for enhancing immunity against agents causative of secondary infections and conditions associated with influenza infection as both the influenza virus and many of the secondary agents enter the host through mucosal surfaces of the upper and/or lower respiratory tracts. In addition, mucosal vaccination (e.g. intranasal vaccination) may induce mucosal immunity not only in the respiratory tracts but also in distant mucosal sites including the genital mucosa.

Intranasal vaccine compositions of the present disclosure can be formulated, for example, in liquid form as nose drops, spray, or suitable for inhalation, as powder, as cream, or as emulsion. Nebulised or aerosolised intranasal vaccine compositions may also be utilised. Administration of vaccine compositions of the present disclosure to mucosa of the upper and/or lower respiratory tract via inhalation of mists, powders, or sprays, or by intranasal administration of nose drops, swabs, powders, sprays, mists, aerosols, and the like is preferred.

Vaccine compositions of the present disclosure may comprise an adjuvant such as, for example, those described in the subsection above entitled *"Pharmaceutical compositions"*. Any suitable adjuvant may be included in a vaccine composition of the present disclosure and the adjuvant may be included in any suitable form (e.g. a powder, a solution, a non-vesicular solution, or a suspension).

Non-limiting examples of adjuvants suitable for inclusion in vaccine compositions of the present disclosure and methods for their preparation are also described in "Vaccine Adjuvants: Preparation Methods and Research Protocols (Methods in Molecular Medicine)", (2000), Ohagan (Ed), Humana Press Inc. Specific examples of such adjuvants include, but are not limited to, aluminum hydroxide; polypeptide adjuvants including interferons, interleukins, and other cytokines; AMPHIGEN, oil-in-water and water-in-oil emulsions; and saponins such as QuilA.

Preferably, the adjuvant is a mucosal adjuvant effective in enhancing mucosal immunity and/or systemic immunity to immunogenic components administered via the mucosal route. Mucosal adjuvants may be broadly classified as those that facilitate vaccine delivery (e.g. liposomes, cochleates, live-attenuated vectors, poly D,L-lactide-co-glycolide or PLGA, chitans, DNA vaccines, mucoadhesives) to enhance the induction of protective immunity induced by other immunogenic components of the vaccine, and those having an immunostimulatory role (e.g. innate immunity associated toxin-based, cytokine-based etc.). Without limitation to a particular mechanism, it is postulated that the advantageous effects of mucosal adjuvants partially derive from an ability to assist the passage of immunogenic components in the vaccine across the mucosal barrier. Upon traversing the mucosal barrier, the mucosal adjuvant may enhance immunity, for example, by complement activation, the induction of cytokines, stimulation of antibody production or antibody type switching, stimulating antigen presenting cells, and/or influencing MHC class I and/or class II expression.

In one embodiment, vaccine compositions of the present disclosure for intranasal administration are provided in a freeze-dried powder form capable of re-constitution immediately prior to use. Powder vaccine formulations of vaccines and compositions of the present disclosure provide a means of overcoming refrigerated storage and distribution requirements associated with liquid-based vaccine stability and delivery. Dry powder formulations offer the advantage of being more stable and also do not support microbial growth.

As demonstrated herein, freeze-dried formulations comprising gamma-inactivated influenza virus induce levels of heterosubtypic immunity similar to that of non freeze-dried formulations. Vaccine compositions of the present disclosure may be freeze-dried using any suitable technique known in the art. For example, liquid preparations of gamma-irradiated influenza virus and/or immunogens against secondary infections or conditions associated with influenza infection may be frozen in a dry ice - isopropanol slurry and lyophilized in a freeze Dryer (e.g. Virtis Model 10-324 Bench, Gardiner, NY) for a suitable time period (e.g. 24 hours).

In one embodiment, a dry powder nasal vaccine formulation of a vaccine composition of the present disclosure is produced by generating spray-freeze-drying (SFD) particles (see, for example, Costantino et al., (2002), "Protein spray freeze drying. 2. Effect of formulation variables on particle size and stability", J Pharm Sci., 91:388-395; Costantino, et al., (2000), "Protein spray-freeze drying. Effect of atomization conditions on particle size and stability", Pharm Res.,17:1374-1383; Maa et al., (1999), "Protein inhalation powders: spray drying vs spray freeze drying", Pharm Res, 16:249-254; Carrasquillo et al., (2001); "Non-aqueous encapsulation of excipient-stabilized spray-freeze dried BSA into poly(lactide-co-glycolide) microspheres results in release of native protein", J Control Release,76:199-208; Carrasquillo et al., (2001), "Reduction of structural perturbations in bovine serum albumin by non-aqueous microencapsulation", J Pharm Pharmacol., 53:115-120; and United States Patent No. 6,569,458). For example, aqueous solutions containing gamma-irradiated influenza virus and/or immunogens against secondary agent(s) and 10% solids (e.g. trehalose) may be passed through a sprayer with atomizing nitrogen gas and droplets collected in trays containing liquid nitrogen then lyophilized in a Manifold Freeze-Dryer. The freeze-dried formulation may be re-constituted immediately prior to use.

Preferred devices for intranasal administration of vaccine compositions of the disclosure are nasal spray devices (e.g. devices available commercially from Pfeiffer GmBH, Valois and Becton Dickinson). Non-limiting examples of suitable devices are described, for example, in Bommer, (1999), "Advances in Nasal drug delivery Technology", Pharmaceutical Technology Europe, p26-33. Intranasal devices may produce droplets in the range 1 to 500 $\mu$m. Preferably, only a small percentage of droplets (e.g. <5%) are below 10 $\mu$m to minimise the chance of inhalation. Intranasal devices may be capable of bi-dose delivery, that is, the delivery of two subdoses of a single vaccine dose, one sub-dose to each nostril.

A vaccine composition of the present disclosure may be administered to a recipient in isolation or in combination with other additional therapeutic agent(s). In embodiments where a vaccine composition is administered with therapeutic agent(s), the administration may be simultaneous or sequential (i.e. vaccine administration followed by administration of the agent(s) or *vice versa*).

*Dosages*

In general, a composition of the present disclosure is administered in a manner compatible with the route of administration and physical characteristics of the recipient (including health status) and in such a way that it is elicits the desired effect(s) (i.e. therapeutically effective, immunogenic and/or protective).

For example, the appropriate dosage of a composition of the disclosure may depend on a variety of factors including, but not limited to, a subject's physical characteristics (e.g. age, weight, sex), whether the compound is being used as single agent or adjuvant therapy, the type of MHC restriction of the patient, the progression (i.e. pathological state) of the influenza infection and/or secondary infection(s), and other factors that may be recognized by one skilled in the art. Various general considerations that may be considered when determining an appropriate dosage of a composition of the disclosure are are described, for example, in Gennaro et al. (Eds), (1990), "Remington's Pharmaceutical Sciences", Mack Publishing Co., Easton, Pennsylvania, USA; and Gilman et al., (Eds), (1990), "Goodman And Gilman's: The Pharmacological Bases of Therapeutics", Pergamon Press.

In general, compositions of the disclosure may be administered to a patient in an amount of from about 50 micrograms to about 5 mg of active component(s) (i.e. gamma-irradiated viruses and/or immunogens against secondary agents). Dosage in an amount of from about 50 micrograms to about 500 micrograms is especially preferred.

One skilled in the art would be able, by routine experimentation, to determine an effective, non-toxic amount of gamma-irradiated influenza virus and/or immunogen against a secondary agent to include in a composition of the disclosure for the desired therapeutic outcome.

Generally, an effective dosage is expected to be in the range of about 0.0001mg to about 1000mg of active component(s) (i.e. gamma-irradiated viruses and/or immunogens against secondary agents) per kg body weight per 24 hours; typically, about 0.001mg to about 750mg per kg body weight per 24 hours; about 0.01mg to about 500mg per kg body weight per 24 hours; about 0.1mg to about 500mg per kg body weight per 24 hours; about 0.1mg to about 250mg per kg body weight per 24 hours; about 1.0mg to about 250mg per kg body weight per 24 hours. More typically, an effective dose range is expected to be in the range about 1.0mg to about 200mg per kg body weight per 24 hours; about 1.0mg to about 100mg per kg body weight per 24 hours; about 1.0mg to about 50mg per kg body weight per 24 hours; about 1.0mg to about 25mg per kg body weight per 24 hours; about 5.0mg to about 50mg per kg body weight per 24 hours; about 5.0mg to about 20mg per kg body weight per 24 hours; about 5.0mg to about 15mg per kg body weight per 24 hours.

Alternatively, an effective dosage may be up to about 500mg/m$^2$ of active component(s) (i.e. gamma-irradiated viruses and/or immunogens against secondary agents). Generally, an effective dosage is expected to be in the range of about 25 to about 500mg/m$^2$, preferably about 25 to about 350mg/m$^2$, more preferably about 25 to about 300mg/m$^2$, still more preferably about 25 to about 250mg/m$^2$, even more preferably about 50 to about 250mg/m$^2$, and still even more preferably about 75 to about 150mg/m$^2$.

Typically, in therapeutic applications, the treatment would be for the duration of the disease state or condition. Further, it will be apparent to one of ordinary skill in the art that the optimal quantity and spacing of individual dosages will be determined by the nature and extent of the disease state or condition being treated, the form, route and site of administration, and the nature of the particular individual being treated. Also, such optimum conditions can be determined by conventional techniques.

In many instances, it will be desirable to have several or multiple administrations of a composition of the present disclosure. For example, compositions of the present disclosure may be administered 1, 2, 3, 4, 5, 6, 7, 8, 9, 10, or more times. The administrations may be from about one to about twelve week intervals, and in certain embodiments from about one to about four week intervals. Periodic re-administration may be desirable in the case of recurrent exposure to a particular pathogen or allergen targeted by a composition of the present disclosure.

It will also be apparent to one of ordinary skill in the art that the optimal course of treatment can be ascertained using conventional course of treatment determination tests.

Where two or more therapeutic entities are administered to a subject "in conjunction", they may be administered in a single composition at the same time, or in separate compositions at the same time or in separate compositions separated in time.

In certain embodiments, the methods of the disclosure involve the administration of gamma-irradiated influenza virus (or compositions/vaccines comprising gamma-irradiated influenza virus) in multiple separate doses. Accordingly, the methods for the prevention (*i.e.* vaccination) and treatment of influenza virus infection described herein encompass the administration of multiple separated doses to a subject, for example, over a defined period of time. Accordingly, the methods for the prevention (*i.e.* vaccination) and treatment of influenza virus infection disclosed herein include administering a priming dose of gamma-irradiated influenza virus (or composition/vaccine comprising gamma-irradiated influenza virus) of the present disclosure. The priming dose may be followed by a booster dose. The booster may be for the purpose of revaccination. In various embodiments, the composition or vaccine is administered at least once, twice, three times or more.

Furthermore, it will be understood that compositions, vaccines and medicaments of the present disclosure that comprise gamma-irradiated influenza viruses in combination with one or more additional immunogenic components (e.g. a second immunogen or vaccine) may be administered to a subject in various different ways. For example, the gamma-irradiated influenza virus and additional immunogenic component(s) may be administered simultaneously or separately. Hence, the composition, vaccine and medicament need not necessarily be provided in a single dosage form comprising both the gamma-irradiated influenza virus and additional immunogenic component(s) which, in certain embodiments, may instead be administered as separate components of the same composition, vaccine, or medicament.

**Preventative and therapeutic methods**

The present disclosure provides methods for enhancing immune responses against an infection or condition in a subject (e.g. secondary infections and conditions associated with influenza virus infection).

"Enhancing" an immune response as contemplated herein refers to augmenting the immune response, for example, innate immunity and/or adaptive immunity (e.g. humoral and/or cell mediated immune responses) of a subject against one or more target secondary infections or conditions associated with influenza virus infection.

Methods for measuring the immune response are known to persons of ordinary skill in the art. For example, a biological sample from a subject treated by the methods of the present disclosure may be compared to a sample from the same subject taken prior to treatment. The immune response, for example, to an agent causative of a secondary infection or condition associated with influenza infection may be measured by way of standard assays known in the art including, but not limited to, solid-phase heterogeneous assays (e.g. enzyme-linked immunosorbent assay), solution phase assays (e.g. electrochemiluminescence assay), amplified luminescent proximity homogeneous assays, flow cytometry, intracellular cytokine staining, functional T-cell assays, functional B-cell assays, functional monocyte-macrophage assays, dendritic and reticular endothelial cell assays, measurement of NK cell responses, oxidative burst assays, cytotoxic specific cell lysis assays, pentamer binding assays, and phagocytosis and apoptosis evaluation.

Additionally or alternatively, the enhancement of immune responses may be determined by assessing resistance to secondary infections or conditions induced by compositions and vaccines of the present disclosure in subject(s) infected by influenza virus. Influenza-infected subjects may be administered a composition of the disclosure targeted at a particular secondary infection or condition. Treated subjects may then be exposed to a corresponding agent causative of that secondary infection or condition, and assessed for the presence or absence of infection and/or the presence or absence of symptoms associated with the particular secondary infection or condition over a suitable time period. Comparison of symptoms with suitable control subject(s) allows determination of whether immunity to the secondary infection or condition in treated subject(s) is enhanced by the administered composition.

As demonstrated in the experimental data provided herein (see "Examples" section below), gamma-irradiated influenza viruses induce cross-protective immunity against flu infection (i.e. immunity against heterologous flu strains). Moreover, gamma-irradiated influenza viruses of the present disclosure can enhance immune responses (e.g. adaptive immune responses) induced by co-administered immunogens and vaccines. Accordingly, certain embodiments of the disclosure relate to the use of gamma-irradiated influenza viruses as agents for preventing and/or treating secondary infections and conditions associated with influenza infection. Without limitation to particular mechanism(s) of action, it is thought that changes imposed on the immune status of the subject, in combination with cross-reactive immunity against influenza viruses invoked by administration of gamma-irradiated influenza, provide a means of protecting a recipient against the secondary infections and conditions. Furthermore, the changes imposed on the immune status of the influenza-infected subject are believed to reduce the propensity to develop strong TH2 responses upon subsequent exposure to allergens (e.g. respiratory allergens) thereby protecting against the development secondary allergic conditions. It has also been identified that gamma-irradiated influenza viruses enhance immune responses induced by co-administered immunogens/vaccines targeted against diseases and conditions that occur independently of influenza infection.

Accordingly, certain embodiments of the present disclosure provide methods for enhancing the immune response against secondary infections or conditions associated with influenza infection by administering a therapeutically effective amount of gamma-irradiated influenza viruses to a subject. The secondary infection or condition referred to in the embodiments above may arise within 7, 10, 14, 17, 21, 24, 28, 31, 35, 38, or 42 days of an influenza infection. The gamma-irradiated influenza viruses may be administered to the subject prior to developing the secondary infection or condition (i.e. as a prophylactic treatment). In such a case the vaccine may be administered before and/or after influenza infection occurs. Additionally or alternatively, the gamma-irradiated influenza viruses may be administered after the secondary infection or condition arises (i.e. as a therapeutic treatment). It will also be understood that the methods may comprise administering a mixture of different gamma-irradiated influenza types, subtypes and/or strains to the subject. The gamma-irradiated influenza viruses may be administered to a subject in the form of a composition, medicament or vaccine of the present disclosure.

Certain embodiments of the present disclosure provide methods for enhancing the immune response against an agent causative of a disease or condition. The methods of the present disclosure may enhance the immune response to any particular immunogen. The immunogen may induce an immune response against a microorganism (e.g. bacteria, viruses, fungi, or parasites) and/or allergen. Non-limiting examples of applicable microorganisms are set out above in the subsection entitled *"Immunogens"*.

In some embodiments, the immune response may be enhanced by inducing stronger interferon (IFN) type I responses (e.g. IFN-$\alpha$) against the agent causative of a disease or condition.

Additionally or alternatively, the immune response may be enhanced by inducing stronger antigen-specific antibody responses (e.g. antigen specific IgG responses) against the agent causative of a disease or condition.

In certain embodiments, the immune response may be enhanced against a viral infection. Non-limiting examples of suitable viruses that the co-administered vaccine may target include adenoviruses, coronaviruses, coxsackieviruses, cytomegaloviruses, echoviruses, Epstein-Barr viruses, herpes simplex viruses, influenza viruses, measles viruses, myxoviruses, parainfluenza viruses, picornaviruses, respiratory syncytial viruses, rhinoviruses, togaviruses (e.g. semliki forest virus) and Varicella-Zoster viruses.

The benefits of vaccination against agents causative of secondary infection(s) and condition(s) associated with influenza infection may be compromised upon influenza infection.

Accordingly, certain embodiments of the present disclosure relate to methods for enhancing immune responses induced by vaccines against agent(s) causative of secondary infection(s) and condition(s) associated with influenza infection. The methods comprise administering gamma-irradiated influenza viruses and at least one type of immunogen against an agent causative of the secondary infection or condition. The secondary infection or condition referred to in the embodiments above may arise within 7, 10, 14, 17, 21, 24, 28, 31, 35, 38, or 42 days of an influenza infection. The gamma-irradiated influenza viruses may be administered to a subject simultaneously with the immunogen, prior to administering the immunogen, or after administering the immunogen. The gamma-irradiated influenza viruses and/or immunogen(s) may be administered to a subject in the form of a composition or vaccine of the present disclosure. It will also be understood that the method may comprise administering a mixture of different gamma-irradiated influenza types, subtypes and/or strains, and/or a mixture of immunogens against different agents to the subject.

Further embodiments of the present disclosure relate to methods for preventing and/or treating secondary infections or conditions arising after influenza virus infection, the methods comprising administering to a subject a synergistic combination of a gamma-irradiated influenza virus and an immunogen from an agent causative of the secondary infection or condition. The secondary infection or condition referred to in the embodiments above may arise within 7, 10, 14, 17, 21, 24, 28, 31, 35, 38, or 42 days of an influenza infection. The gamma-irradiated influenza viruses and immunogens may be administered to a subject in the form of a composition or vaccine of the present disclosure. It will be understood that the method may comprise administering a mixture of different gamma-irradiated influenza types, subtypes and/or strains, and/or a mixture of immunogens against different agents to the subject.

As discussed above (see subsection entitled *"Synergistic combinations"*) the present disclosure provides a synergistic combination comprising at least one gamma-irradiated influenza virus strain and at least one additional immunogen against a secondary infection or condition associated with influenza infection (i.e. at least one "immunogen against a secondary agent"). In accordance with the methods of the present disclosure, a synergistic combination of the present disclosure may be administered to a subject to induce immunity against both the influenza virus and at least one other agent causative of an associated secondary infection or condition. In general, the immunogenicity induced against a secondary infection or condition by the synergistic combination is greater than could be gained from the additive effect of each component when used independently of the other.

The methods of the present disclosure may be used to enhance the immune response of a subject against secondary infections and conditions associated with influenza infection. Accordingly, the methods of the present disclosure may be used to prevent and/or treat secondary infections and conditions associated with influenza infection. Non-limiting examples of secondary infections and conditions associated with influenza infection and causative agents are provided in the subsection above entitled *"Immunogens"*.

In certain embodiments, methods of the present disclosure are used to enhance and/or modulate the immune response of a subject against a secondary condition associated with influenza infection (e.g. an allergy). For example, the methods of the disclosure may be used to modulate the immune status of a subject infected by influenza virus such that hypersensitivity (e.g. type I hypersensitivity) to allergens is reduced (e.g. respiratory allergens such as, for example, pollen, mould, house dust mite *(Dermatophagoides pteronyssinus),* dust, protein allergens from animals, chemical allergens, and combinations thereof).

Gamma-irradiated influenza viruses administered in accordance with the methods of the present disclosure may be of any type, subtype or strain, or a mixture of any number of different types, subtypes and/or strains. Non-limiting examples of suitable influenza types, subtypes and strains along with methods for gamma-irradiation of the viruses are provided in the preceding subsection entitled *"Gamma-irradiated influenza viruses"*.

In certain embodiments, gamma-irradiated viruses administered in accordance with the methods of the present disclosure are H1N1 subtype viruses. The H1N1 viruses may be strain APR/8/34.

Immunogens against agent(s) causative of secondary infection(s) or condition(s) associated with influenza infection to be administered in accordance with the methods of the present disclosure include, but are not limited to, any one or more of those provided in the preceding subsection entitled *"Immunogens"*. Non-limiting examples of agents which the immunogens induce immunity against are also provided in the preceding subsection entitled *"Immunogens"*.

Non-limiting examples of secondary infections and conditions associated with influenza infection that may be treated and/or prevented using the methods of the present disclosure are also provided in the subsection above entitled *"Immunogens"*. Accordingly, non-limiting examples of secondary infections and conditions that may be prevented or treated include pneumonia (e.g. bacterial, viral, fungal pneumonia), chronic obstructive pulmonary disease, sinusitis, otitis media, bronchitis and the common cold.

"Subjects" and "recipients" as contemplated herein include mammals (e.g. humans) and individuals of any species of social, economic or research importance including, but not limited to, ovine, bovine, equine, porcine, feline, canine, avian, primate, and rodent species. The subject or receipient may be mammalian. The subject or receipient may be human. In certain embodiments, the subject may be avian (e.g. chicken/*Gallus gallus domesticus*).

Gamma-irradiated influenza virus, immunogens against secondary agents casusative of infections or conditions associated with influenza infection, and compositions and vaccines of the present disclosure may be administered to a subject by the mucosal route (e.g. the intranasal route). Mucosal and intranasal compositions and methods for administering the same are described in the subsections above entitled *"Pharmaceutical compositions"* and *Vaccines".*

Administration via the intranasal route, and in particular gamma-irradiated microorganisms by the intranasal route may provide advantages over other routes of administration. For example, administration by the intranasal route is thought to induce secretory IgA production at mucosal epithelium eliciting cross protection more effectively than serum IgG. Without being bound to a particular mechanism, inactivation of influenza virus and/or other secondary infectious agents by gamma-irradiation is believed to cause inactivation without significantly affecting the antigenic structure of the microorganism. Thus, the combination of gamma-irradiation of microorganisms followed by intranasal administration is thought to offers several advantages, including, but not limited to, 1) facilitating the binding of inactivated microorganism to tissue specific receptors, 2) allowing the induction of tissue specific immune responses, 3) reducing the systemic exposure to whole mircroorganism antigen, and 4) limiting the side effects associated with whole mircroorganism vaccines.

In addition, a number of secondary infections and conditions associated with influenza infection affect the upper and/or lower respiratory tracts. For example, many causative agents induce pneumonia (e.g. bacterial, viral, fungal pneumonia), chronic obstructive pulmonary disease, sinusitis, otitis media, bronchitis or the common cold. Accordingly, administration of compositions of the disclosure via mucosal routes and in particular via the intranasal route provides localised stimulation of the immune response in tissues affected by relevant pathogens and allergens.

Various embodiments of the present disclosure relate to co-administering gamma-irradiated influenza viruses with an additional immunogen (or vaccine comprising an additional immunogen) for the purpose of enhancing immune responses induced by the additional immunogen/vaccine. This provides a means of preventing and/or treating various diseases and conditions, including secondary infections associated with influenza infection.

It will be understood that "co-administering" in this context encompasses both simultaneous and sequential administration of the gamma-irradiated influenza viruses and additional immunogen/vaccine at the same site or at a different site. Accordingly, the gamma-irradiated influenza viruses and additional immunogen/vaccine may be administered together as a single formulation, or, be administered as separate components.

The gamma-irradiated influenza viruses and additional immunogen/vaccine may be administered by the same or different modes of administration. By way of non-limiting example only, the gamma-irradiated influenza viruses may be administered intranasally while the additional immunogen/vaccine may be administered intravenously, intraperitoneally or subcutaneously. Alternatively, the gamma-irradiated influenza viruses and additional immunogen/vaccine may both be administered intranasally.

In embodiments where administration is sequential, the gamma-irradiated influenza viruses may be administered to the subject first and the immunogen/vaccine second, or *vice versa.* The order of administration of the additional immunogen/vaccine and the gamma-irradiated influenza viruses may be contemporaneous (i.e. sufficiently close in time so that the gamma-irradiated influenza viruses result in an enhancement of an immune response induced by the additional immunogen/vaccine). Contemporaneous administration encompasses administration of the gamma-irradiated influenza viruses up to about one week before or one week after administration of the immunogen/vaccine. For example, the immunogen/vaccine and gamma-irradiated influenza viruses may be administered to the subject on the same day or within several hours of each other.

**Medicaments and kits**

Certain embodiments of the present disclosure relate to medicaments and kits for enhancing an immune response in a subject against an infection or condition (e.g. a secondary infection or condition associated with influenza virus infection). The medicaments and kits may be prepared by incorporating one or more different types, subtypes and/or strains of gamma-irradiated influenza viruses.

Other embodiments of the present disclosure relate to medicaments and kits for enhancing an immune response in a subject induced by a vaccine or immunogen against an agent causative of an infection or condition (e.g. a secondary infection or condition following influenza virus infection). The medicaments and kits may be prepared by incorporating one or more different types, subtypes and/or strains of gamma-irradiated influenza viruses with a vaccine or immunogen against an agent causative of a target disease or infection (e.g. a secondary infection or condition following influenza virus infection). Typically, the vaccine comprises one or more different immunogens against an agent causative of the infection or condition.

Additional embodiments of the present disclosure relate to medicaments and kits for preventing or treating an infection or condition (e.g. a secondary infection or condition associated with influenza virus infection). The medicaments and kits may be prepared by incorporating one or more different types, subtypes and/or strains of gamma-irradiated influenza viruses and one or more different immunogens against the agent causative of the infection or condition.

Also provided by the present disclosure is gamma-irradiated influenza virus in combination with an immunogen against an agent causative of a secondary infection or condition associated with influenza infection, for use in the treatment or prevention of the secondary infection or condition.

Kits of the present disclosure may further comprise an intranasal administration device and/or other components required to conduct the methods of the present disclosure, such as buffers and/or diluents. The kits typically include containers for housing the various components and instructions for using the kit components in the methods of the disclosure.

Medicaments and kits of the disclosure may be used for the prevention and/or treatment of one or more infections or conditions (e.g. secondary infections or conditions associated with influenza virus infection). Non-limiting examples of infections and conditions associated with influenza infection and causative agents are provided in the subsection above entitled *"Immunogens"*. Accordingly, non-limiting examples of infections (e.g. secondary infections) include pneumonia (e.g. bacterial, viral, fungal pneumonia), chronic obstructive pulmonary disease, sinusitis, otitis media, bronchitis and the common cold. Non-limiting examples of conditions (e.g. secondary conditions) include allergic responses to respiratory allergic responses (e.g. asthma, hay fever, allergic rhinitis, allergic sinusitis and the like).

Gamma-irradiated influenza viruses incorporated in medicaments and kits of the present disclosure may be any type, subtype or strain, or a mixture of any number of different types, subtypes and/or strains. Non-limiting examples of suitable influenza types, subtypes and strains along with methods for gamma-irradiation of the viruses are provided in the preceding subsection entitled *"Gamma-irradiated influenza viruses"*.

In certain embodiments, gamma-irradiated viruses incorporated in medicaments and kits of the present disclosure are H1N1 subtype viruses. The H1N1 viruses may be strain APR/8/34.

Immunogens against agent(s) causative of an infection or condition (e.g. secondary infection(s) or condition(s) associated with influenza infection) incorporated in medicaments and kits of the present disclosure include, but are not limited to, any one or more of those provided in the preceding subsection entitled *"Immunogens"*. Non-limiting examples of agents to which the immunogens induce immunity against are also provided in the preceding subsection entitled *"Immunogens"*.

It will be appreciated by persons skilled in the art that numerous variations and/or modifications may be made to the present disclosure as shown in the specific embodiments without departing from the spirit or scope of the present disclosure as broadly described. The present embodiments are, therefore, to be considered in all respects as illustrative and not restrictive.

Various embodiments of the present disclosure relate to medicaments and kits comprising gamma-irradiated influenza viruses and an additional immunogen (or vaccine comprising an additional immunogen) which may be used for enhancing immune responses induced by the additional immunogen/vaccine. The medicament and kits may thus be used for preventing and/or treating various diseases and conditions, including secondary infections associated with influenza infection.

The gamma-irradiated influenza viruses and additional immunogen/vaccine may be provided together as a single formulation (i.e. in a single medicament, or a single composition in a kit), or, as separate components. Thus, "a medicament" as contemplated herein may comprise two or more components capable of separate administration. Similarly, a kit may comprise gamma-irradiated influenza viruses and an additional immunogen/vaccine as separate components.

In embodiments where medicaments and kits of the present disclosure comprise gamma-irradiated influenza viruses and the additional immunogen/vaccine as separate components of a medicament or kit, the components will generally be for (i.e. "formulated for") co-administration to a subject. It will be understood that "co-administration" in this context encompasses both simultaneous and sequential administration of the gamma-irradiated influenza virus and additional immunogen/vaccine components at the same site or at a different site.

Gamma-irradiated influenza virus and additional immunogen/vaccine components of the medicament or kit may be for administration by same or different modes of administration. By way of non-limiting example only, the gamma-irradiated influenza virus component may be for intranasal administration while the additional immunogen/vaccine component may be for intravenous, intraperitoneal or subcutaneous administration. Alternatively, the gamma-irradiated influenza virus component and additional immunogen/vaccine component may both be for intranasal administration.

In embodiments where the components are for sequential administration, the gamma-irradiated influenza viruses may be for administration to the subject first and the immunogen/vaccine second, or *vice versa*. The additional immunogen/vaccine and gamma-irradiated influenza viruses may be for contemporaneous administration (i.e. sufficiently close in time so that the gamma-irradiated influenza viruses result in an enhancement of an immune response induced by the additional immunogen/vaccine). Contemporaneous administration encompasses administration of the gamma-irradiated influenza viruses up to about one week before or one week after administration of the immunogen/vaccine. For example, the immunogen/vaccine and gamma-irradiated influenza viruses may be for administration to the subject on the same day and or within several hours of each other.

**Examples**

**Example 1: Intravenous vaccination of BALB/c mice with gamma-irradiated influenza A virus**

(i) Materials and Methods

*Animals*

BALB/c mice of the same sex and within a similar age group (8-12 weeks old) were used in each experiment.

*Viruses and immunization*

Influenza virus strains A/WSN (H1N1), A/JAP (H2N2) and A/PC (H3N2) were grown and titrated as described by Yap et al., (1977), "Cytotoxic T cells specific for influenza virus-infected target cells", Immunology, 32: 151. Virus titres are expressed as haemagglutinating units (HAU). A/JAP influenza virus was inactivated either by exposure of crude allantoic fluid to $1.26 \times 10^6$ rad (12.6 KGy) from a $Co^{60}$ source (60 hours at 350 rad/min) or by exposure as dialysed, infectious allantoic fluid to UV radiation (320 $\mu$W/cm$^2$) for 10 min. Exposure to y or UV radiation for these periods destroyed infectivity completely as tested in embryonated eggs. Animals were immunized by a single injection of $10^3$ HAU intravenously.

*Target cells*

P815 thioglycollate-induced peritoneal macrophages (TGM), concanavalin-A (con-A) and lipopolysacharide (LPS) induced lymphoblasts were obtained, prepared and infected as described in Yap et al., 1977, "Cytotoxic T cells specific for influenza virus-infected target cells", Immunology, 32: 151, and Parish and Müllbacher, 1983, "Automated colorimetric assay for T cell cytotoxicity", J. Immunol Meth., 58: 225-237.

*Generation of effector cells*

Memory cultures for the generation of secondary *in vitro* influenza-immune Tc cells were generated using methods described in Müllbacher, 1984, "Hyperthermia and the generation and activity of murine influenza-immune cytotoxic T cells in vitro", J. Virol., 52, 928-931. Briefly, $8 \times 10^7$ spleen cells from mice immunized with influenza virus 3 months previously were co-cultured with $1 \times 10^7$ virus-infected stimulator cells for 5 days *in vitro*. The stimulator cells were infected with infectious or inactivated virus at a multiplicity of infection of approximately $10^3$ HAU per $10^6$ cells.

*Cytotoxicity assay*

The methods used for tumour cells and macrophage targets are described in detail in Yap et al., 1977, "Cytotoxic T cells specific for influenza virus-infected target cells", Immunology, 32: 151, Parish and Müllbacher, 1983, "Automated colorimetric assay for T cell cytotoxicity", J. Immunol Meth., 58: 225-237, and Müllbacher, 1984, "Hyperthermia and the generation and activity of murine influenza-immune cytotoxic T cells in vitro ", J. Virol., 52, 928-931. The duration of the assays was 6 hours. The per cent specified lysis was calculated using the formula:

$$\text{Specific lysis (\%)} \quad = \quad \frac{\text{experimental release - medium release}}{\text{maximum release - medium release}} \quad X \quad 100$$

(ii) Results

*Priming of BALB/c mice with infectious or inactivated influenza virus for memory Tc cells*

BALB/c mice were injected with $10^3$ HAU of either infectious, $\gamma$-irradiated or UV inactivated A/JAP virus. Three months later, spleens were removed and the cells boosted *in vitro* with infectious A/JAP-infected stimulator spleen cells and the Tc cell response measured 5 days later at three effector:target cell ratios. **Table 1** shows representative data of percent specific lysis of infected P815 target cells. Clearly, infectious virus primed for a Tc cell response more effectively than $\gamma$-irradiated or UV-irradiated virus, but the $\gamma$-irradiated virus gave substantial lysis using all three infected target cells (A/WSN, A/JAP and A/PC) compared with a much weaker response by UV-irradiated virus. From precursor frequency analysis under limiting dilution conditions, animals primed with infectious virus gave approximately three-fold higher estimates of Tc cell precursor frequency in spleens than animals primed with $\gamma$-irradiated virus. This is in agreement with the values of lysis obtained in bulk cultures (**Table 1**).

**Table 1:** *Secondary in vitro stimulation with infectious A/JAP virus of spleen cells from mice previously immunized with infectious, $\gamma$-irradiated or UV irradiated A/JAP virus.*

| Primary immunization | Effector cell: target cell ratio | %Specific lysis of P815 cells* infected with: | | |
|---|---|---|---|---|
| | | A/WSN(H1N1) | A/JAP(H2N2) | A/PC(H3N2) |
| Infectious | 10 | 78 | 70 | 66 |
| | 3 | 86 | 67 | 76 |
| | 1 | 43 | 52 | 44 |
| $\gamma$-irradiated | 10 | 36 | 25 | 22 |
| | 3 | 35 | 27 | 20 |
| | 1 | 11 | 8 | 6 |
| UV-irradiated | 10 | 14 | 28 | 0 |
| | 3 | 0 | 13 | 0 |
| | 1 | 2 | 7 | 0 |
| *Values for percent specific lysis given were obtained by subtracting the values of lysis by effector cells on uninfected P815 cells. The standard errors of the means of triplicate samples were always less than 8% and usually less than 4%. Spontaneous $^{51}$Cr release ranged from 16 to 19%. | | | | |

*Ability of inactivated virus to boost memory influenza-immune Tc cells*

Spleen cells of mice primed with infectious A/JAP virus ($10^3$ HAU) 3 months previously were boosted *in vitro* with A/JAP treated stimulator cells, using either infectious virus, $\gamma$-irradiated or UV-inactivated virus and Tc cell activity assays were carried out. The results shown in **Table 2** demonstrate that all three viruses were able to restimulate cross-reactive Tc memory cells, but that $\gamma$-irradiated virus was superior to UV-irradiated virus. Cells boosted with UV-inactivated virus gave significant lysis only on target cells infected with the homologous virus.

**Table 2**: *Secondary in vitro stimulation with infectious or inactivated A/JAP virus of spleen cells from mice previously immunized with infectious A/JAP virus.*

| Primary immunization | Effector cell: target cell ratio | %Specific lysis of P815 cells* infected with: | | |
|---|---|---|---|---|
| | | A/WSN(H1N1) | A/JAP(H2N2) | A/PC(H3N2) |
| Infectious | 30 | 71 | 62 | 65 |
| | 10 | 80 | 82 | 84 |
| | 3 | 82 | 87 | 82 |
| | 1 | 38 | 59 | 31 |
| $\gamma$-irradiated | 30 | 47 | 62 | 41 |
| | 10 | 30 | 66 | 31 |
| | 3 | 16 | 33 | 24 |
| | 1 | 4 | 12 | 4 |
| UV-irradiated | 30 | 18 | 51 | 18 |
| | 10 | 15 | 28 | 19 |
| | 3 | 6 | 12 | 15 |

(continued)

| Primary immunization | Effector cell: target cell ratio | %Specific lysis of P815 cells* infected with: | | |
|---|---|---|---|---|
| | | A/WSN(H1N1) | A/JAP(H2N2) | A/PC(H3N2) |
| | 1 | 2 | 7 | 0 |
| *Values for percent specific lysis given were obtained by subtracting the values of lysis by effector cells on uninfected P815 cells. The standard errors of the means of triplicate samples were always less than 8% and usually less than 4%. Spontaneous $^{51}$Cr release ranged from 8 to 10%. | | | | |

*Sensitization of target cells with infectious and inactivated A/JAP virus*

To determine if inactivated influenza virus was able to sensitize target cells, P815 tumour cells, TGM, and LPS and con-A lymphoblasts were treated with either infectious, $\gamma$-irradiated or UV-inactivated virus at $10^3$ HAU per $10^6$ cells ($2 \times 10^6$ cells/ml) for 2 hours. These targets were then tested for specific lysis by secondary *in vitro* influenza immune Tc cells (**Table 3**). Only infectious virus was able to sensitize targets (especially P815 and TGM) to give significant lysis above uninfected control targets.

**Table 3**: *Sensitization of target cells by infectious and inactivated A/JAP virus as tested by secondary influenza-immune Tc cells.*

| Target | K:T | Specific $^{51}$Cr release (%)* | | |
|---|---|---|---|---|
| | | Infectious | $\gamma$ | UV |
| P815 | 30 | 73 | 4 | 1 |
| | 10 | 72 | 4 | 2 |
| | 3 | 53 | 2 | 1 |
| TGM | 30 | 22 | 1 | 0 |
| | 10 | 16 | 1 | 0 |
| | 3 | 11 | 3 | 2 |
| LPS-blasts | 30 | 10 | 6 | 7 |
| | 10 | 11 | 9 | 0 |
| | 3 | 3 | 9 | 3 |
| Con-A blasts | 30 | 10 | 0 | 0 |
| | 10 | 10 | 0 | 0 |
| | 3 | 6 | 0 | 0 |
| *Values for percent specific lysis given were obtained by subtracting the values of lysis by effector cells on uninfected P815 cells. The standard errors of the means of triplicate samples were always less than 8% and usually less than 4%. Spontaneous $^{51}$Cr release range: 10-12% for P815, 13-17% for TGM, 37-40% for LPS and 22-25% for con-A blasts. | | | | |

*Protection from lethal influenza virus infection with inactivated virus*

To investigate whether mice immunized with such inactivated virus preparations would be protected against lethal influenza virus infections, mice were primed with infectious, $\gamma$-irradiated or UV-irradiated A/JAP virus 4-5 weeks prior to a challenge with a lethal dose of live influenza virus of the same or different subtypes. The results from one of two experiments are shown in **Table 4.** Mice primed with any of the three viruses survived a challenge by the homologous A/JAP and heterologous A/WSN virus, though mice primed with UV-irradiated virus and challenged with A/WSN sickened and one died. The major difference observed was with A/PC virus. Mice primed with UV-irradiated virus were as susceptible as unprimed animals, whereas mice primed with $\gamma$-irradiated virus survived the challenge at least as well as mice primed with infectious virus. It is unlikely that antibody was responsible for the observed cross-protection, as transfer of immune serum from animals primed with either infectious or $\gamma$-inactivated virus significantly reduced virus titres in the lungs of animals infected with the homologous A/JAP virus but not in A/WSN-infected animals.

**Table 4:** *The effect of pre-immunization of mice with infectious, g-irradiated or UV-irradiated A/JAP on survival following a subsequent challenge with a lethal dose of infectious A/WSN (H1N1), A/JAP (H2N2) or A/PC (H3N2).*

| Pre-immunization (A/JAP) | Number of mice surviving challenge with: | | |
|---|---|---|---|
| | A/WSN | A/JAP | A/PC |
| Nil | 1 | 0 | 1 |
| Infectious | 8 | ND* | 4 |
| $\gamma$-irradiated | 8 | 8 | 6 |
| UV-irradiated | 7 | ND* | 0 |
| *Previous experiments had shown that primed mice would always withstand a challenge by the homologous virus. BALB/c mice were injected intravenously (i.v.) with $10^3$ HAU infectious, UV-irradiated or g-irradiated A/JAP virus or with nothing. Eight weeks later, groups of mice (eight mice per group) were inoculated intranasally with a lethal dose of A/WSN ($10^4$ EID$_{50}$), A/JAP ($3 \times 10^5$ EID$_{50}$) or A/PC ($1.5 \times 10^8$ EID$_{50}$). Death of mice was recorded for 20 days with the results given above. | | | |

These results demonstrate that $\gamma$-inactivated virus protects animals against lethal infection by heterologous A strain viruses at least as well as infectious virus. UV-inactivated virus on the other hand did not confer this protection to the more distantly related virus A/PC (H3N2). The finding that $\gamma$-irradiation is more effective than UV-irradiation in retaining the ability to prime for cross-reactive Tc cells upon destroying viral infectivity suggests that $\gamma$-irradiation is less damaging to the antigenic structure of at least some internal proteins than is UV irradiation. Surprisingly, there is little previously published work on the affect of $\gamma$-irradiation on viruses or proteins. In contrast to infectious virus, neither the $\gamma$-irradiated or UV-irradiated virus was able to sensitize target cells (activated macrophages, lymphoblasts or P815 cells) facilitating lysis by virus-specific Tc cells. However, it is possible that the cells which are mainly involved in antigen presentation of irradiated virus after intravenous injection may have characteristics different from those of activated macrophages or lymphocytes.

**Example 2: Intravenous vaccination with the $\gamma$-irradiated influenza A strains A/WSN [H1N1], A/PR8 [H1N1], A/JAP [H2N2] and A/PC [H3/N2]**

(i) Materials and Methods

*Animals and Viruses*

Stocks of influenza A virus (strains A/WSN, A/Pr8 [H1N1]; A/JAP [H2N2]; A/PC [H3N2]) were prepared in 10-day-embryonated eggs. Virus stocks were prepared from allantoic fluid and stored in aliquots at -70°C. Initially, BALB/c and C57B1/6 mice were infected intranasally, and severity of flu infection was evaluated in terms of mortality, weight loss, lung histology and lung infiltration.

*$\gamma$-irradiation of influenza strains*

$\gamma$-ray dose response studies of frozen and room temperature-kept viral stocks were undertaken at ANSTO/Lucas Heights/NSW to define the conditions that give sterile virus preparations with optimal immunogenicity. A radiation dose of $5 \times 10^5$ rad (5 KGy) was sufficient to induce sterility determined by hemagglutination (HA) assay following amplification of residual infectious virus in embryonated eggs. To safeguard for absolute virion inactivation, a dose of $1 \times 10^6$ rad (10 KGy) of $\gamma$-ray was chosen for the $\gamma$-flu (y-irradiated influenza virus) preparations, which were used to vaccinate mice prior to their challenge with infectious influenza. Virus stocks were kept on dry ice through out the process of irradiation.

*Intravenous vaccination and intranasal challenge*

Groups of 8 BALB/c mice were intravenously vaccinated twice, 4 weeks apart, with $\gamma$-flu prior to lethal intranasal challenge with A/JAP. $\gamma$-A/WSN ($6 \times 10^3$ haemagglutinating units (HAU)/mouse), $\gamma$-A/PC ($6 \times 10^3$ HAU/mouse), and $\gamma$-A/JAP ($3 \times 10^3$ HAU/mouse) were injected intravenously. Four weeks following the 2nd dose of $\gamma$-flu, mice were challenged with A/JAP (50 HAU/mouse) and then monitored for 20 days.

*Immunohistochemistry*

Lungs were fixed in 10% neutral buffered formalin for one week and embedded in paraffin. For examination of tissue morphology, 4 micron sections were stained with hematoxylin and eosin (H&E).

*Lymphocyte isolation from the lung and FACS analysis*

To evaluate the effect of vaccination with $\gamma$-flu on tissue infiltration of CD8+T cells, lungs from mock and vaccinated mice were harvested into ice-cold MEM containing 5% FCS on days 6 post challenge with influenza virus. The samples were digested with 2 mg/mL collagenase type 1 (Gibco-Life Technologies) in MEM/5% FCS for 30 min at 37°C with shaking and homogenised by gently pressing through a 100 $\mu$m mesh tissue sieve. Homogenates were then centrifuged at 400 x g for 10 min, and the pellets were resuspended in 2 mL 90% Percoll (Sigma-Aldrich) in MEM/5% FCS. The suspension was transferred to a 15 mL tube and overlayed gently with 60, 40, and 10% Percoll in MEM. The gradients were centrifuged at 800 x g for 45 min. The lymphocytes were collected from the 40-60% interface and washed twice with MEM/5% FCS. Expression of cell surface markers on freshly isolated lymphocytes from lungs of A/WSN-infected (both mock and vaccinated) mice was determined by staining with Ab specific for CD8 (PharMingen). Cells from a single mouse were suspended in 100 $\mu$L ice-cold MEM/5% FCS and incubated with Fc Block (PharMingen) for 15 min at 4°C. Cells were washed and incubated with the relevant Ab at 4°C for 30 min in the dark and then washed twice, fixed with 2% w/v paraformaldehyde, and stored in the dark at 4°C until analysis using a FACScan (Becton Dickinson).

*Cross-reactive CTL responses induced by $\gamma$-flu*

To test whether $\gamma$-flu induces cross-reactive Tc cell responses, A/WSN and A/PC and their corresponding $\gamma$-flu preparations were used to intravenously infect or vaccinate mice. 10-week-old BALB/c mice were either infected or vaccinated with A/WSN, $\gamma$-A/WSN, A/PC, and $\gamma$-A/Pc. Five-days later, splenocytes from infected, vaccinated, and mock-immunized animals were tested for their killing activity on mock, A/WSN-infected, A/PC-infected, and target cells modified with the appropriate $K^d$ restricted nucleoprotein derived peptide (NPP-labelled P815 targets). CTL response were measured using a $Cr^{51}$ release assay as described in Müllbacher et al., 1993, "Spontaneous mutation at position 114 in H-2Kd affects cytotoxic T cell responses to influenza virus infection" Eur. J. Immunol. 29, 1228-1234.

*Statistics*

All statistical analyses were conducted using GraphPad InStat software. One-Way-ANOVA test was used to compare the MEAN, and SEM for significant differences among vaccinated and un-vaccinated groups in terms of weight loss.

(ii) Results

*Intravenous $\gamma$-flu vaccination protects against lethal challenge with influenza A/JAP*

The capacity of $\gamma$-flu preparations to induce protective immunity was tested by challenging $\gamma$-flu primed mice with homologous and heterologous influenza viruses. Respiratory infection with influenza A/JAP (H2N2) in mice is associated with lethality, and $LD_{50}$ is 50 HAU/mouse intranasally (i.n.) in 10-week-old females BALB/c mice **(Table 5).**

**Table 5:** *The protective effect of $\gamma$-flu preparations against homologous or heterologous influenza A infection.*

| Group* | 1st $\gamma$-flu (i.v.) | 2nd $\gamma$-flu (i.v.) | Challenge (i.n.) | Survival |
|---|---|---|---|---|
| Un-vaccinated | - | - | A/JAP | 4/8 |
| A | $\gamma$-A/JAP | $\gamma$-A/JAP | A/JAP | 8/8 |
| B | $\gamma$-A/WSN | $\gamma$-A/PC | A/JAP | 6/8 |
| C | $\gamma$-A/PC | $\gamma$-A/WSN | A/JAP | 8/8 |
| *Groups of 8 BALB/c mice were vaccinated twice, 4 weeks apart, with □$\gamma$-flu prior to lethal challenge with A/JAP. $\gamma$-A/WSN ($6\times10^3$ HAU/mouse), $\gamma$-A/PC($6\times10^3$ HAU/mouse), and $\gamma$-A/JAP($3\times10^3$ HAU/mouse) were injected i.v. 4 weeks following the 2nd dose of $\gamma$-flu, mice were challenge i.n with A/JAP (50 HAU/mouse) and mice were monitored for 20 days. | | | | |

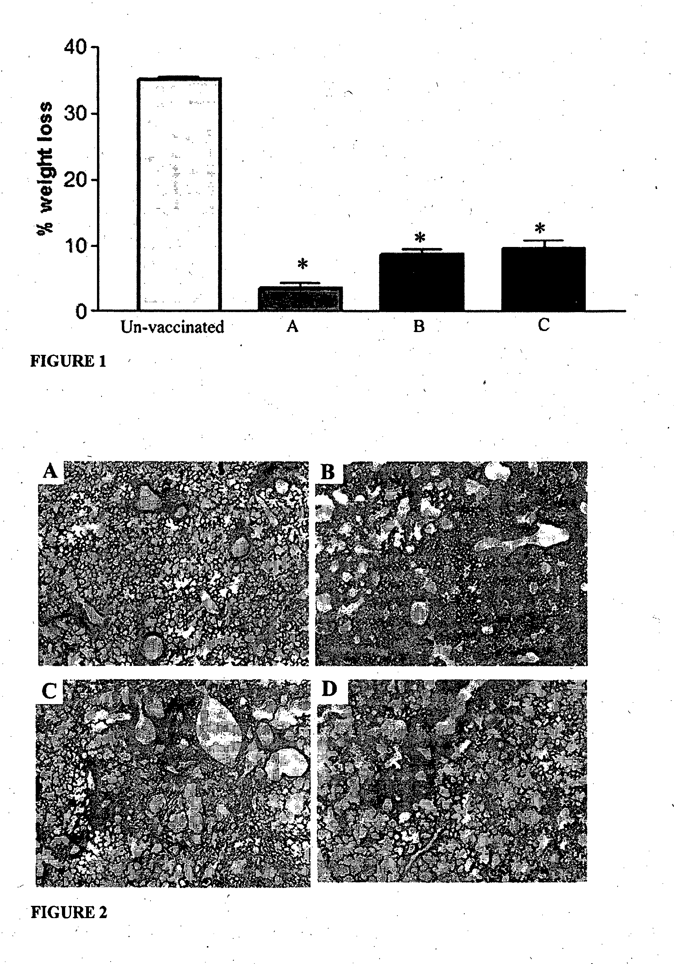

Prior intravenous (i.v.) injections of γ-ray-inactivated homologous or heterologous influenza A strains enhanced the survival rate of LD$_{50}$ A/JAP challenged mice (Table 5). When compared to unvaccinated mice, all vaccinated groups (A, B, and C) showed significant reductions in weight loss following intravenous infection with A/JAP (P value of <0.01 using ANOVA test) (**Figure 1**). Naive unvaccinated animals presented a weight loss of >30% at 6 days post A/JAP challenge. In comparison, all vaccinated animals only showed mild weight loss of <10% (**Figure 1**).

*γ-flu vaccination reduces lung inflammation following challenge with influenza A/WSN*

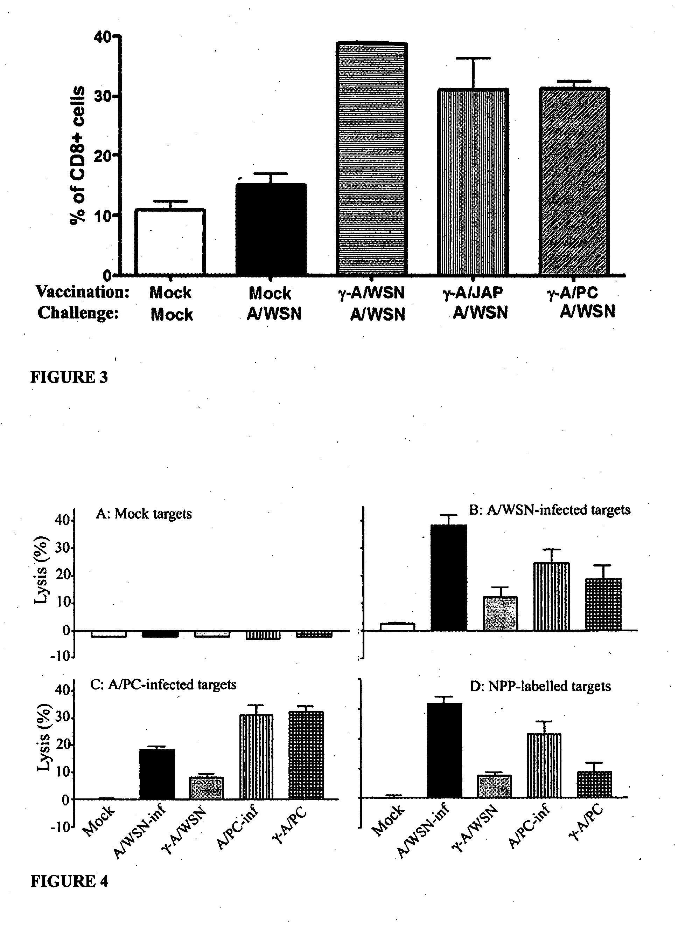

In addition to mortality and weight loss following A/JAP infection, lung inflammation and infiltration following A/WSN infection was assessed. Intranasal infection with A/WSN is characterized by a severe inflammatory response, evident in comparative histology of naive (**Figure 2A**) versus infected (**Figure 2B**) lungs. Inflammation is substantially reduced in γ-flu vaccinated animals challenged with homologous (**Figure 2C**) or heterologous (**Figure 2D**) influenza virus. Despite lower total inflammation, CD8+T cells preferentially infiltrated lungs of γ-flu vaccinated animals (**Figure 3**).

*γ-flu induces cross-reactive cytotoxic (Tc) cell responses*

The protective effect of γ-flu against infection with homologous virus is expected to involve both humoral and cellular immunity. The mechanism responsible for the observed cross-protective immunity (protection from heterologous infection) may be at least in part cytotoxic T (Tc) cell-mediated. To test whether γ-flu induces cross-reactive Tc cell responses, A/WSN and A/PC and their corresponding γ-flu preparations were used to infect or vaccinate mice, and splenic effectors were then tested for their killing activity on mock, A/WSN-infected, A/JAP-infected, and target cells modified with the appropriate K$^d$ restricted nucleoprotein derived peptide (NPP). As shown in **Figure 4,** effector splenocytes from infected and vaccinated animals lysed homologous and heterologous virus-infected P815 targets. In addition, all splenocytes, except those from mock-infected mice, showed killing activity on nuclear protein peptide labelled targets.

**Example 3: Intranasal vaccination with gamma-irradiated influenza A virus protects against H5N1 (bird flu)**

(i) Materials and Methods

*Animals*

10 week old BALB/c mice were utilised in this experiment.

*Viruses*

Virus stocks of two A strains of influenza viruses (A/PR8 [H1N1] and A/PC [H3N2]), were grown in embryonated hen eggs and purified by temperature-dependent adsorption to chicken red blood cells, and virus titres estimated by standard plaque assays on Madin-Darby canine kidney (MDCK) cells and titres expressed as pfu/mls.

*γ-irradiation of influenza strains*

The purified stocks were exposed to $1 \times 10^6$ rad (10 kGy) of γ-rays (ANSTO, Lucas Height, Australia) as described in Example 2 above. The residual viral infectivity in irradiated stocks was tested by using embryonated hen eggs. Virus stocks were sterile but retained full haemagglutinating activity after irradiation.

*Statistics*

All statistical analyses were conducted using GraphPad InStat software. Fisher's exact and Chi Square tests were used to compare survival rates for significant differences.

*Cross-reactive cytotoxic T cell responses in BALB/c mice*

Ten week old BALB/c mice were either infected or vaccinated with A/PR8, γ-A/PR8, A/PC, or γ-A/PC. Six-days later, splenocytes from these mice were tested for their killing activity on mock, A/PC-, A/PR8-, A/JAP-infected, and NPP-labelled P815 targets using Cr$^{51}$ release assay as described above in Examples 1 and 2 above.

*Intranasal vaccination with γ-flu for homologous and heterologous protection*

Groups of mice were vaccinated with either $3.2 \times 10^6$ pfu/mouse of γ-A/PR8 (n=10), γ-A/PC (n=10), formalin inactivated A/PC (n=8), or mock treated (n=10). Four weeks post vaccination mice were challenged intranasally with $2 \times 10^5$ pfu/mouse of A/PR8 and monitored for weight loss and mortality for 21 days.

*Intranasal γ-flu vaccination versus other routes of administration*

Different routes (intranasal (i.n.), intravenous (i.v.), intraperitoneal (i.p.) and subcutaneous (S.c.) of inoculation were used to vaccinate BALB/c mice (10 mice/group) with $3.2 \times 10^6$ pfu equivalent of γ-A/PC. Three weeks post vaccination mice were challenged i.n. with a lethal dose of live A/PR8 ($6 \times 10^2$ pfu) and monitored for mortality and clinical symptoms using a 30% body weight loss as the end point.

*Base parameters of lethal H5N1 infections in BALB/c mice*

Groups of 10-week-old BALB/c mice were infected intranasally with 10-fold serial dilutions of H5N1 virus stock. Mice were monitored for weight loss and morbidity. The end of an individual mouse's weight track indicates sacrificing due to ~25% weight loss.

*Protection against H5N1 using intranasal vaccination with γ-FLU (γ-A/PR8 [H1N1])*

The protective effect of γ-flu against a lethal challenge of H5N1 was tested. BALB/c mice (10 mice/group) were either mock treated or vaccinated intranasally with a single dose of γ-A/PR8 [H1N1] ($3.2 \times 10^6$ pfu equivalent/mouse). Four weeks post vaccination mice were challenged intranasally with 3x mouse infectious dose 50 (3xMID50) of A/Vietnam/1203/2004 [H5N1] and monitored daily for mortality and clinical symptoms for 21 days using a 20% body weight loss as the end point.

*Real-time PCR (RT- PCR) to estimate the effect of γ-flu vaccine on H5N1 genetic load*

Following the addition of 100 μl of lung or brain homogenate into 600 μl of RLT buffer, RNA was extracted using an RNeasy Mini Kit (Qiagen) according to the manufacturer's instructions. The total RNA concentration of each sample was determined by spectrophotometry and adjusted to 40 ng $\mu l^{-1}$ with nuclease free water. Standardised amounts (200 ng) of template were subsequently reverse transcribed using the TaqMan Reverse Transcription Reagents kit (Applied Biosystems) in 20 μl reactions following the recommendations of the manufacturer. For quantitation of viral cDNA, universal influenza virus type A-specific primers and TaqMan probe, which amplified and detected a product from within the viral matrix gene, were use as described previously (see Heine, H.G., et al, 2007 "Rapid detection of highly pathogenic avian influenza H5N1 virus by TaqMan reverse transcriptase-polymerase chain reaction" Avian Dis. 51: 370-372).

Reactions were performed in triplicate and contained 12.5 μl of TaqMan 2X Universal PCR Master Mix, 900 nM of each primer, 250 nM of probe, 2 μl of cDNA template and 6.8 μl of water. Separate triplicate reactions to quantify 18S rRNA (TaqMan Ribosomal Control Reagents, Applied Biosystems) were also performed to exclude the presence of PCR inhibitors in all samples tested. Reactions were performed in 96-well plates using the 7500 Fast Real-Time PCR System (Applied Biosystems) and the following cycling parameters: 50 °C for 2 min; 95 °C for 10 min; 45 cycles of 95 °C for 15 sec and 60 °C for 1 min. For relative quantitation of viral genetic loads, a standard curve was generated using, as template, 10-fold serial dilutions of extracted stock virus RNA in 40 ng $\mu l^{-1}$ of RNA prepared from uninfected mouse lung. To facilitate interpretation of data, 1 unit (1 U) of viral RNA was arbitrarily defined as the number of RNA molecules which, when reverse transcribed and subjected to real-time PCR, produced a $C_T$ value of 38.

(ii) Results

*A/PR8 γ-flu and A/PC γ-flu preparations induce cross-reactive cytotoxic T cell responses in BALB/c mice*

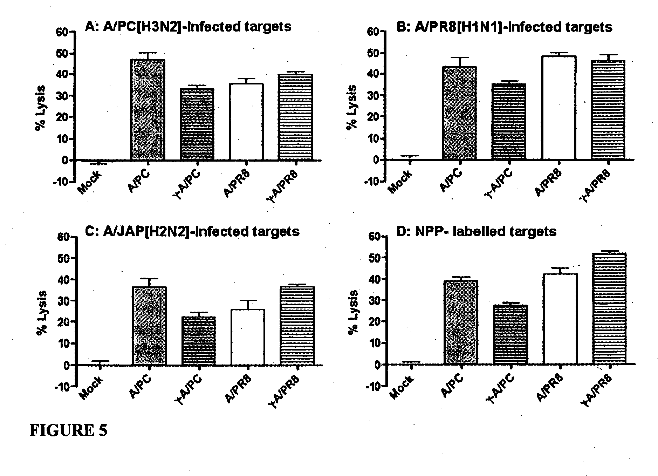

The induction of cross-reactive cytotoxic T (Tc) cell responses was tested in γ-flu vaccinated mice. A/PR8 and A/PC and their corresponding γ-flu preparations were used to infect or vaccinate mice. Six days later, splenocytes from infected, vaccinated, and mock-immunized animals were tested for their killing activity on mock, A/PC-, A/PR8-or A/JAP-infected and target cells modified with the appropriate $K^d$ restricted nucleoprotein derived peptide (NPP) using $Cr^{51}$ release assay (as described in Examples 2 and 3 above). As shown in **Figure 5,** effector splenocytes from influenza-infected and γ-flu vaccinated animals induced killing activity against all influenza infected P815 targets regardless of the virus strains used. In addition, all splenocyte populations, except those from mock-infected mice, showed killing activity on influenza virus nuclear protein peptide labelled targets. These data illustrate the ability of $\gamma$-flu preparations to induce cross-reactive Tc cell responses in mice.

*Intranasal $\gamma$-flu vaccination protects against lethal dose of influenza A/PR8*

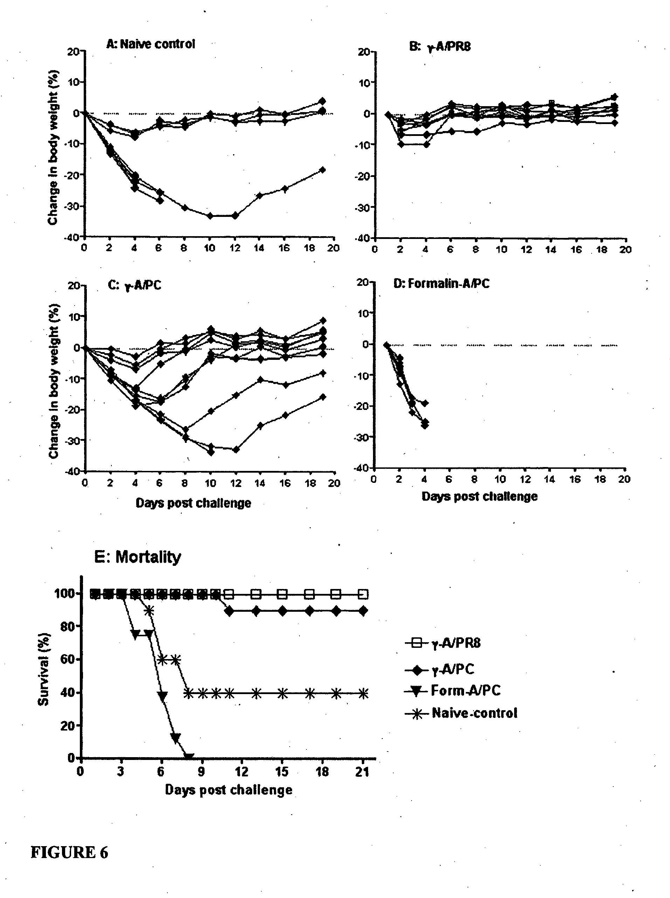

Considering that whole virus vaccine elicits complications due to a high reactogenicity and that $\gamma$-irradiation has little impact on viral immunogenecity (as shown above), the intranasal administration of $\gamma$-flu was tested. The efficacy of the intranasal $\gamma$-flu vaccine formulation was compared to that of chemically inactivated vaccine preparations used in current human influenza virus preparations. Mice were vaccinated intranasally with equal doses of one of three inactivated virus preparations (formalin A/PC, $\gamma$-A/PC [H3N2] or $\gamma$-A/PR8 [H1N1]). In these experiments mice were vaccinated with a single dose only and challenged 4 weeks later with a lethal dose (>300x the lethal dose 50) of A/PR8. Mice were weighed and observed for mortality for 3 weeks post challenge **(Figure 6)**. $\gamma$-A/PR8 vaccinated animals fully recovered with little weight loss after challenge with homologous virus **(Figure 6B)**. Challenge of $\gamma$-A/PC vaccinated animals with A/PR8 (*i.e.* with heterologous virus) caused in a proportion of animals weight loss with most starting recovery by day 4 **(Figure 6C)** coinciding with the peak of a memory Tc cell response (Müllbacher and Tha Hla, 1993, "In vivo administration of major histocompatibility complex class I-specific peptides from influenza virus induces specific cytotoxic T cell hyporesponsiveness", Eur. J. Immunol., 23, 2526-2531). The survival rate of $\gamma$-A/PC vaccinated animals is significant compare to unvaccinated group (p<0.05 using Chi-square test). All mice vaccinated with formalin inactived vaccine preparations rapidly lost weight **(Figure 6D)**. The mortality data **(Figure 6E)** confirm the morbidity data and show that intranasal vaccination with $\gamma$-flu protects from homologous and heterologous influenza A challenges.

*Intranasal $\gamma$-flu vaccination versus other routes of administration*

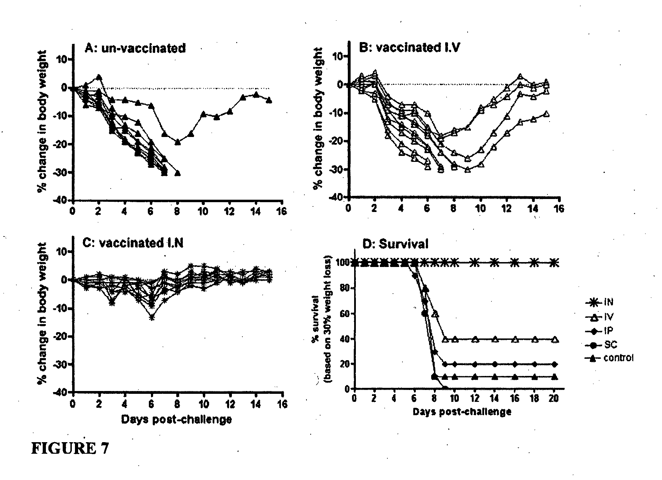

Different routes (intranasal (i.n.), intravenous (i.v.), intraperitoneal (i.p.) and subcutaneous (S.c.) of inoculation were used to vaccinate BALB/c mice (10 mice/group) with $3.2 \times 10^6$ plaque forming units (PFU) equivalent of $\gamma$-A/PC. Three weeks post vaccination mice were challenged i.n. with a lethal dose of live A/PR8 ($6 \times 10^2$ PFU) and monitored for mortality and clinical symptoms using a 30% body weight loss as the end point (**Figure 7**). All i.n. vaccinated animals fully recovered with little if any weight loss after challenge with heterotypic virus (**Figure 7C**). In contrast, the majority of unvaccinated (**Figure 7A**), i.v. vaccinated (**Figure 7B**), and i.p. and S.c. vaccinated (data not shown) mice lost weight progressively to reach 30% body weight loss at days 7 and 8 post infection. The survival data (**Figure 7D**) show that despite the use of unnaturally high challenge doses of A/PR8, i.n. vaccinated mice survived at significant levels the heterotypic challenge (P<0.05 using Fisher's Exact test).

*Base parameters of lethal H5N1 infections in BALB/c mice*

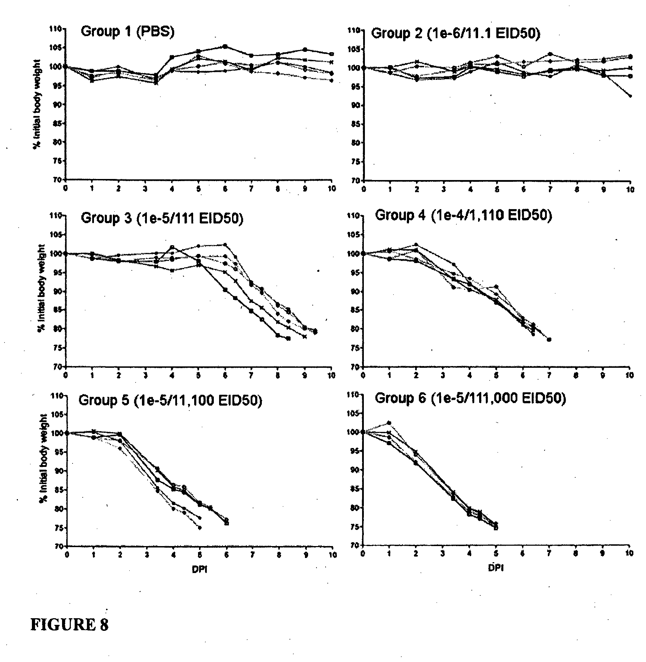

Weight loss in BALB/c mice was assessed following intranasal infection with H5N1 (A/Vietnam/1203/2004). As shown in **Figure 8,** groups of 5 mice were challenged with either diluent alone (Group 1) or 10-fold serial dilutions of stock virus (Groups 2-6, in order of increasing concentration of inoculum). Mice were weighed and observed for morbidity and sacrificed before reaching a body weight loss of >25%. Mice infected with 111 EID50 (group 3) and those infected with 11100 EID50 (group 5) started to show weight loss by day 6 and 2 post-infection, respectively.

*Protection against H5N1 avian influenza virus*

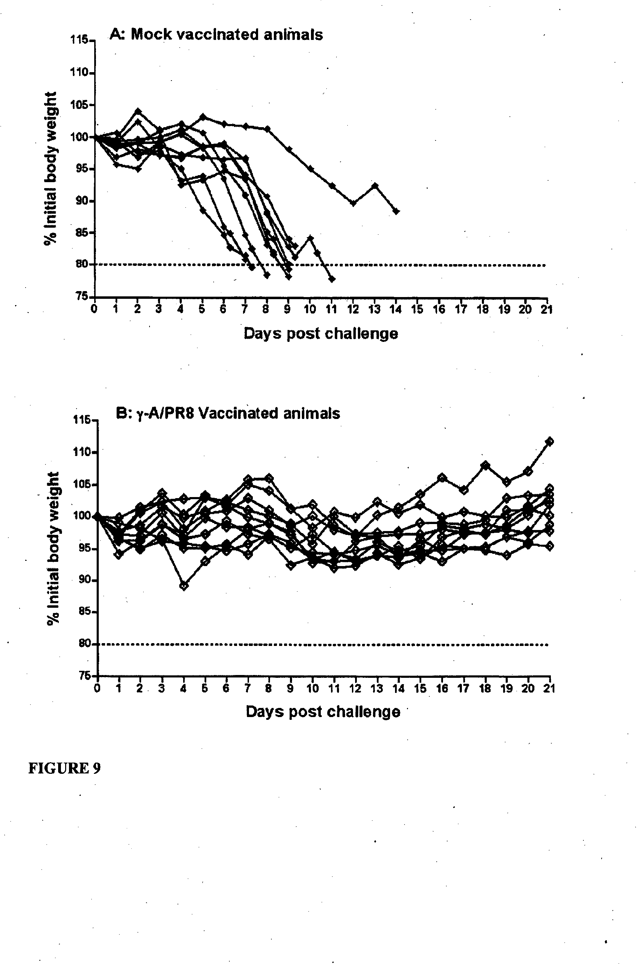

The protective effect of $\gamma$-flu against a lethal challenge of H5N1 as tested. BALB/c mice (10 mice/group) were vaccinated intranasally with a single dose of $\gamma$-A/PR8 [H1N1] ($3.2 \times 10^6$ PFU equivalent/mouse). Four weeks post vaccination mice were challenged intranasally with 3x mouse infectious dose 50 (3xMID50) of A/Vietnam/1203/2004 [H5N1] and monitored for mortality and clinical symptoms using a 20% body weight loss as the end point (**Figure 9**). All ten mice in the control group (mock vaccinated) developed clinical signs consistent with H5N1 infection and were euthanized between DPI 7 and 14 days post infection according to the experimental end-points that were approved by the AEC (where weight loss of 20% or more was observed, where any neurological sign was detected or where the infection had led to an inability to eat/drink (*e.g.* severe hunching, severe dehydration, inactivity)) (**Figure 9A**). In general, unvaccinated mice developed greasy/ruffled fur from days 4 post-infection, two mice developed neurological signs categorised by an abnormal hindlimb gait and hindlimb weakness at which time they were euthanized, and all other mice were euthanized at ~20% body weight loss (with varying degrees of depression, inactivity and dehydration).

In contrast, all vaccinated mice (gamma-A/PR8 [H1N1]) remained bright and active throughout the study and were euthanized at the conclusion of the trial on day 21 post-infection (**Figure 9B**). A single animal lost 11% body weight by day 4 post-infection but was bright and active and regained the pre-challenge weight by the end of the trial. In general, all mice survive the lethal challenge with H5N1 and some animals gained more body weight to exceed their pre-challenge weight.

*Intranasal γ-flu vaccination results in early clearance of H5N1 from the lung*

As shown above, all vaccinated mice survived the lethal challenge with H5N1. Therefore, the data demonstrates that a single dose of γ-flu preparation administered intranasally induces cross-protective immunity in mice against a lethal challenge with H5N1 virus. Quantitation of viral infectivity and viral genetic loads confirmed the protective effect of γ-A/PR8[H1N1] against avian influenza and show clearance of H5N1 virus from lung tissues by day 6 post-challenge (**Table 6**). The detection of viral infectivity and viral RNA in the lung of one vaccinated animal on day 3 post-challenge but none at day 6 indicates that the observed cross-protective immunity is most likely mediated by memory Tc cells, which express accelerated activation kinetics over that of naive Tc cells, as described previously (Bennink et al, 1978, "Influenzal pneumonia: early appearance of cross-reactive T cells in lungs of mice primed with heterologous type A viruses", Immunology 35: 503-509).

**Table 6:** *H5N1 infectivity and viral genetic loads in lung and brain*

| DPI* | Treatment | Mouse | Infectivity | | Genetic load | |
|---|---|---|---|---|---|---|
| | | | Lung | Brain | Lung | Brain |
| 3 | Mock | 1 | 6.5 | ≤ 3.2 | 3.4 ± 0.03 | -† |
| | | 2 | 5.8 | | 2.0 ± 0.02 | |
| | Vaccinated | 1 | | | | |
| | | 2 | 7.4 | - | 3.6 ± 0.02 | - |
| 6 | Mock | 1 | 6.4 | - | 4.2 ± 0.003 | - |
| | | 2 | 6.4 | - | 4.2 ± 0.01 | - |
| | | 3 | 7.4 | 5.0 | 4.4 ± 0.03 | 3.0 ± 0.03 |
| | Vaccinated | 1 | - | - | - | - |
| | | 2 | - | - | - | - |
| | | 3 | - | - | - | - |

Viral infectivity and relative viral genetic loads are expressed as $\log_{10}$ TCID$_{50}$ g$^{-1}$ and $\log_{10}$ U per 20 ng of extracted RNA (geometric mean ± s.d. of triplicate reactions), respectively, where 1 unit (1 U) of viral RNA is arbitrarily defined as the number of RNA molecules which, when reverse transcribed and subjected to real-time PCR, produced a $C_T$ value of 38.
* Days post infection.
† Undetectable (< $10^{3.2}$ TCID$_{50}$ g$^{-1}$ (infectivity) or < 1 U per 20 ng of extracted RNA (genetic load)).

**Example 4: Gamma-ray inactivated influenza A viruses induce cross-protective immunity in mice that is primarily mediated by cytotoxic T cells**

(i) Materials and Methods

*Cells and viruses*

P815 mastocytoma and Madin-Darby canine kidney (MDCK) cells were maintained in EMEM plus 5% FCS at 37°C in a humidified atmosphere with 5% $CO^2$. The influenza type A viruses, A/PR/8 [A/Puerto Rico/8/34 (H1N1)] and A/PC [A/Port Chalmers/1/73 (H3N2)] were grown in 10-day-old embryonated chicken eggs. Each egg was injected with 0.1 ml normal saline containing 1 hemagglutinin unit (HAU) of virus, incubated for 48 hours at 37°C, then held at 4°C for overnight. The amniotic/allantoic fluids were harvested, pooled and stored at -80°C. Titres were $10^7$ PFU/ml (A/PC) and 2 x $10^8$ PFU/ml (A/PR8) using plaque assays on MDCK cells. Viruses were purified using chicken red blood cells for vaccine preparation as described in (Sheffield, et al. (1954), "Purification of influenza virus by red-cell adsorption and elution", British journal of experimental pathology, 35:214-222). Briefly, infectious allantoic fluid was incubated with red blood cells for 45 minutes at 4°C allowing the viral hemagglutinin to bind red blood cells, and then centrifuged to remove the allantoic fluid supernatant. The pellets were resuspended in normal saline, incubated for 1 hour at 37°C to release the red blood cells from the virus and then centrifuged to remove the red blood cells and collect the virus in the supernatant.

Purified A/PC stock titre was 5 x 10$^8$ PFU/ml.

*Virus inactivation*

For formalin inactivation, the viruses were incubated with 0.2 % formalin at 4°C for a week. Formalin was then removed by pressure dialysis using normal saline for 24 hours at 4°C. The dialysis method was adapted from Current Protocols in Immunology (see Andrew et al., (2001), "Dialysis and concentration of protein solutions", in Current protocols in immunology, Coligan et al., (eds), Appendix 3: Appendix 3H). For UV inactivation, the viruses were placed in 60-mm petri dishes with a fluid depth of 10 mm. The virus was exposed to 4000 ergs per cm$^2$ for 45 minutes at 4°C. For gamma ray inactivation, influenza viruses received a dose of 10 kGy from a $^{60}$Co source (Australian Nuclear Science and Technology Organization - ANSTO). The virus stocks were kept frozen on dry ice during gamma irradiation. Loss of viral infectivity was confirmed by titration of inactivated virus preparations in eggs. The HAU titres of inactivated virus stock were determined to be 7.3 x 10$^4$ HAU/ml for gamma-inactivated A/PC, 2.4 x 10$^4$ HAU/ml for formalin- and UV-inactivated A/PC.

*Protection experiments*

BALB/c, C57BL/6, 129Sv/Ev, β2-microglobulin (β2m$^{-/-}$), Ig μ-chain (μMT$^{-/-}$), perforin (Prf$^{-/-}$), IFN-γ receptor (IFN-IIR$^{-/-}$) and MHC-II$^{-/-}$ mice were bred under specific pathogen-free conditions. 10~14-week-old females were used. Mice were immunized intranasally with inactivated virus preparations (3.2 x10$^6$ PFU equivalent). For lethal challenge, at 3 weeks post-immunization, mice were infected intranasally with A/PR8 (7 x 10$^2$ PFU). Mice were weighed daily and monitored for mortality until day 20 post-challenge.

*Adoptive immune lymphocyte transfer experiment*

10-week-old donor BALB/c mice were immunized intravenously with γ-irradiated A/PC (1 x 10$^8$ PFU equivalent). Splenocytes were collected at week 3 post immunization. Single-cell suspensions were prepared and red blood cells were lysed. The splenic lymphocytes were separated into B and T cell populations by passing the cells through nylon wool columns. 2ml of 5 x 10$^7$ cells/ml were loaded onto columns and incubated for 2 hours at 37°C. The columns were washed with warm (37°C) Hanks balanced salt solution + 5% FCS and non-adherent T cells in the first effluent were collected. Nylon wool-bound B cells were then collected by washing the columns with cold (4°C) Hanks-balanced salt solution. Purity of T (82.8%, + 7.94% B cell) and B (84.2%, + 8.3% T cell) cell populations was confirmed by flow cytometric analysis. Small samples of purified splenocytes were washed in PBS with 2% FCS. Fc receptors were blocked by incubation with mouse CD16/CD32 (Fcγ III/II receptor) Ab (BD Pharmingen) for 20 min at 4°C. Cells were washed and further incubated with a mixture of fluorescent-conjugated anti-CD3, anti-CD8, anti-CD19 (BD Pharmingen) Abs. Dead cells were labelled with 7-aminoactinomycin D (Sigma-Aldrich). Stained cells were quantified using a FACS Calibur (Becton Dickinson). Purified T or B cells (1.1 x 10$^7$ cells in a volume of 0.2 ml) were intravenously injected into recipient mice, which were then challenged with A/PR8 (7 x 10$^1$ PFU) intranasally at 3 hours after the adoptive cell transfer. Mice were monitored for bodyweight loss and mortality until day 20 post-challenge.

*Passive serum transfer experiment*

Sera from intranasally immunized mice with γ-irradiated A/PC were collected at 3 weeks post-immunization. The pooled immune sera were heated for 30 minutes at 56°C to inactivate complement. Recipient mice received 200μL of immune sera intravenously. After two hours, the recipient mice were challenged with A/PR8 (7 x10$^2$ PFU). Mice were monitored for body weight and mortality until day 20 post challenge.

*Plaque reduction assay*

Immune sera were collected 3 weeks post-immunization from mice vaccinated with live, γ-irradiated, formalin or UV-inactivated A/PC. After heat inactivation of serum samples at 56°C for 30 minutes, 190 μL of serially diluted (x10, x30, x90, x270) serum was mixed with 10μL virus (A/PC or A/PR8 strain) suspension containing roughly 100 PFU. After 60 minute incubation at 37°C the residual virus infectivity was measured by plaque assay on MDCK cells.

*Cytotoxic T Lymphocyte (Tc cell) Assay*

Influenza-specific Tc cells were generated by intravenously injecting BALB/c mice with either live A/PC (~2 x 10$^6$ PFU) or inactivated A/PC (gamma-, formalin-, or UV-inactivated, ~1 x 10$^8$ PFU equivalent). Spleens were harvested at 7 days post immunization and red blood cell-depleted cell suspensions were prepared for use as effector cells. Target cells were prepared by infecting P815 cells with live A/PC at a multiplicity of infection (m.o.i) of 1, followed by 1 hour incubation in medium containing 100~200 $\mu$Ci of $^{51}$Cr. After washing, target cells were mixed with effector cells at different ratios in an 8 hour chromium release assay. The level of radioactivity in the supernatant was measured in a gamma counter. Specific lysis is given as mean percent lysis of triplicate wells and values were calculated using the formula: (experimental cpm - spontaneous cpm)/(maximal release cpm - spontaneous cpm) x100. For secondary *ex vivo* Tc cell responses, the primed mice received an intravenous secondary immunization at 3 months post primary immunization and splenocytes were harvested at 7 days post-immunization for chromium release assay.

(ii) Results

*Role of immune sera and B and T lymphocytes in heterosubtypic immunity induced by $\gamma$-irradiated influenza* virus

To determine the role of antibodies in cross-protective immunity, mice were intranasally immunized with either live A/PR8 (7 x 10$^1$ PFU) or $\gamma$-irradiated A/PC (3.2 x 10$^6$ PFU equivalent), and 3 weeks later blood was collected. Groups of naive mice injected intravenously with 200$\mu$l of either $\gamma$-irradiated A/PC immune serum, hyper-immune serum (from mice that received two doses of live A/PR8 at three week intervals) or pre-immune serum and challenged with a lethal dose of A/PR8 virus (7 x 10$^2$ PFU) 2 hour post serum transfer.

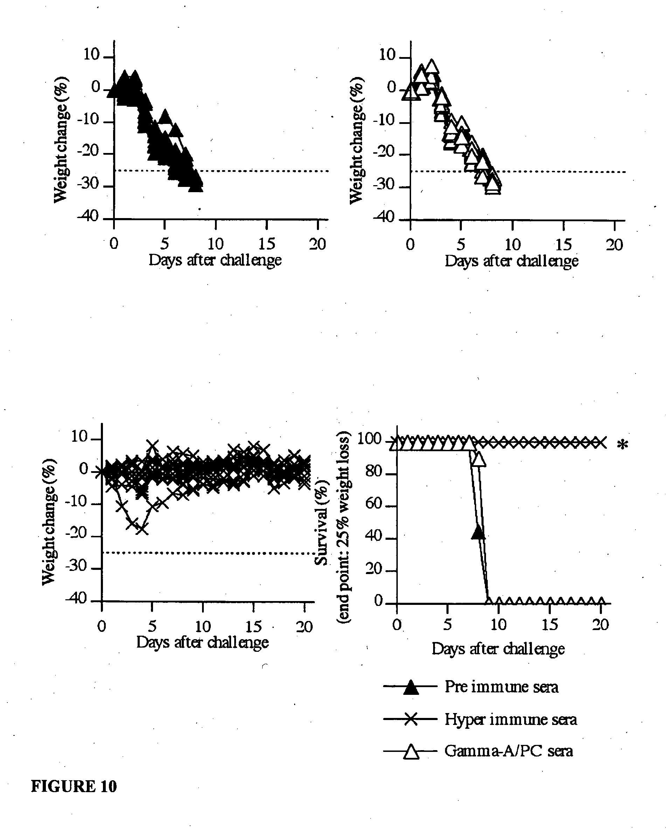

**Figure 10** illustrates that passive serum transfer fails to transfer heterosubtypic immunity induced by $\gamma$-irradiated A/PC, to naive mice. Serum samples were pooled from donor mice immunized with either a single dose of $\gamma$-irradiated A/PC (3.2 x 10$^6$ PFU equivalent) or two doses of live A/PR8 (7 x 10$^2$ PFU) (hyper immune). Recipient mice (9~10 mice per group) were given intravenously 0.2 ml of immune sera or preimmune sera as a control. At two hours post serum transfer, mice were challenged intranasally with A/PR8 (7 x 10$^2$ PFU). Mice were monitored daily for weight loss (**Figure 10 A, B & C**) and mortality (**Figure 10D**). Naive mice that received $\gamma$-irradiated A/PC immune serum developed clinical signs and weight loss similarly to those that received pre-immune serum (Figure 10A, C & D). These mice rapidly lost weight to reach the endpoint of 25% weight loss and accordingly were not protected from heterosubtypic challenge. In contrast, mice that received the hyper-immune serum were fully protected with virtually no weight loss when challenged with homologous A/PR8 (7 x 10$^1$ PFU) (Figure 10B & D). These data indicate that $\gamma$-irradiated A/PC induced antibodies are not cross-protective.

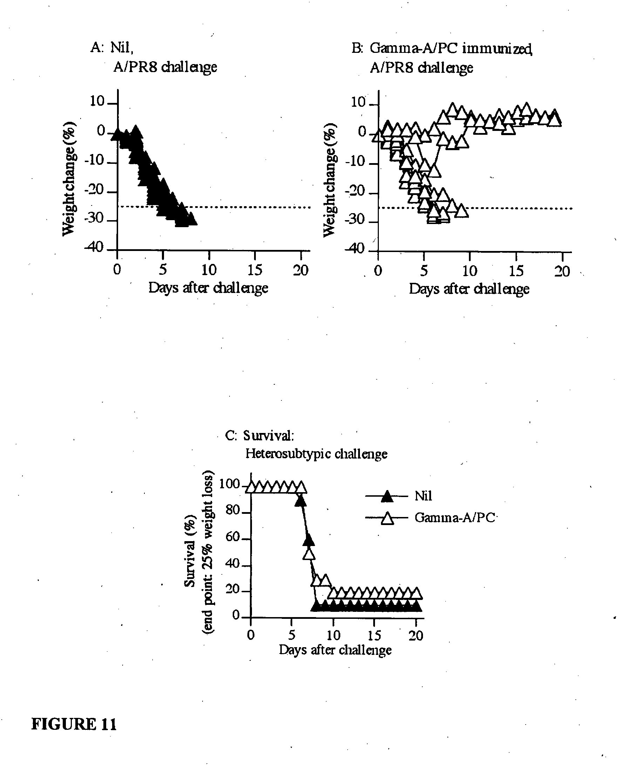

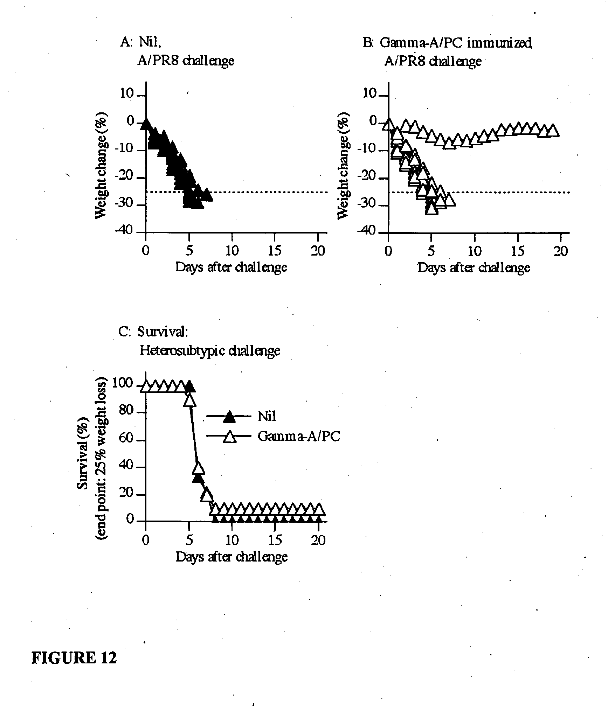

Secondly, B cell-deficient $\mu$MT$^{-/-}$ mice were used to assess the role of B cells in cross-protective immunity. 10-week-old $\mu$MT$^{-/-}$ mice were immunized intranasally with $\gamma$-irradiated A/PC (3.2 x 10$^6$ PFU equivalent) and challenged with the heterosubtypic strain A/PR8 (7 x 10$^2$ PFU) three weeks post-immunization. Mice were monitored daily for weight loss and mortality for 20 days. The vaccinated $\mu$MT$^{-/-}$ mice displayed a survival rate similar to that of naive mice (**Figure 11A, B & C**), implying that an absence of B cells does impair the development of cross-protective immunity. Furthermore, intranasal vaccination with $\gamma$-irradiated A/PC (3.2 x 10$^6$ PFU equivalent) failed to protect MHC-II$^{-/-}$ mice against heterosubtypic challenge with A/PR8 (**Figure 12A, B & C**). MHCII$^{-/-}$ mice were immunized intranasally with $\gamma$-irradiated A/PC (3.2 x 10$^6$ PFU equivalent). 3 weeks post- immunization, naive and immunized mice (9~10 mice per group) were challenged with heterosubtypic strain A/PR8 (7 x 10$^2$ PFU). Mice were monitored daily for weight loss and mortality for 20 days. Vaccination with $\gamma$-irradiated A/PC failed to protect NMC-II$^{-/-}$ mice against heterosubtypic challenge with A/PR8. This provides evidence that B and CD4+ T cells participate in the induction of cross-protective immunity by $\gamma$-irradiated influenza virus.

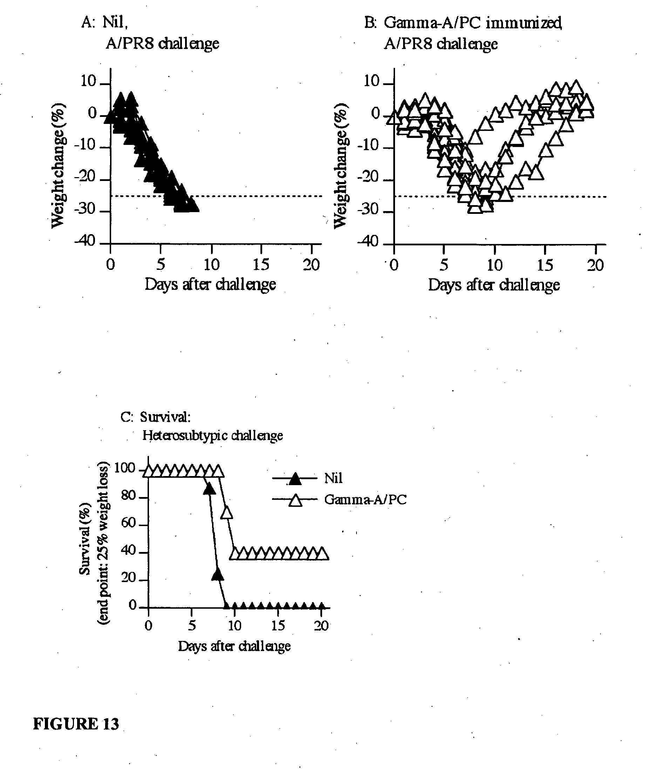

$\beta$2M$^{-/-}$ mice which are deficient in CD8+ Tc cell responses were used to evaluate the contribution of CD8+ T (Tc) cells in the cross-protective immunity induced by intranasal immunisation with $\gamma$-irradiated A/PC (3.2 x 10$^6$ PFU equivalent). A heterosubtypic challenge with A/PR8 (7 x 10$^2$ PFU) caused a mortality rate of 60%, with the surviving mice losing over 10% of their body weight prior to their recovery (**Figure 13B & C**). Controls, unvaccinated mice infected with the same virus strain suffered 100% mortality (**Figure 13A & C**). This demonstrates a critical role for CD8+T cells in the cross-protective immunity induced by $\gamma$-irradiated influenza virus.

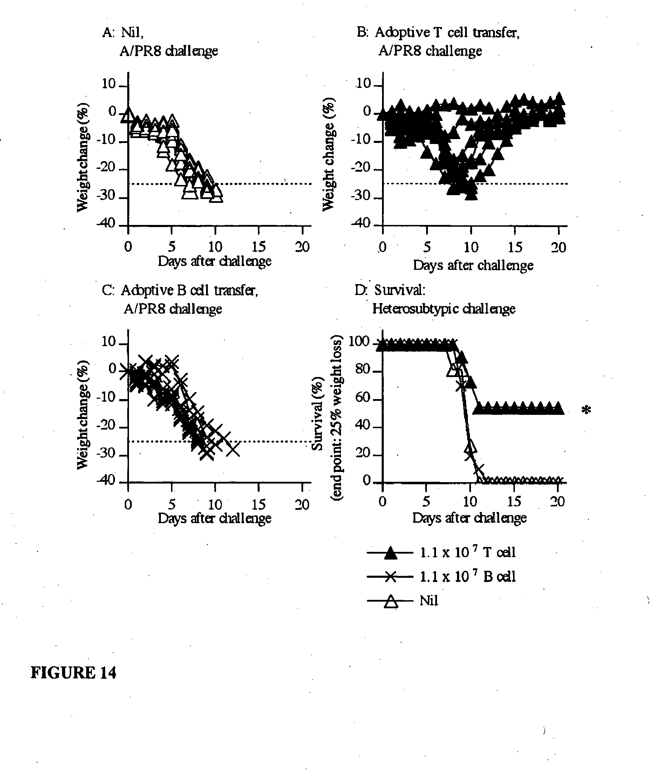

Although these results show a role for B, CD4+ T and CD8+ T cells in the cross-protective immunity against influenza, defective primary immune responses in the knock-out mice may obscure the cross-protective potential of both humoral and cellular memory responses. As an alternative approach to assess the nature of the effector cells, an adoptive transfer model was used, with splenocytes from 3 week earlier intravenously $\gamma$-irradiated A/PC (1 x 10$^8$ PFU equivalent) immunized mice as donor cells. Splenocytes were nylon wool-enriched T cells (82.8% T cells, 7.9% B cell) or B cells (84.2% B cells, 8.3% T cells) and intravenously transferred into naive mice. Three hour post-transfer mice were challenged with 0.1 x LD50 A/PR8 (7 x 10 PFU). Mice were monitored daily for weight loss and mortality for 20 days. T cell recipients were partially protected against A/PR8 challenge (**Figure 14A, B & D**). In contrast, no protection was observed in B cell recipient mice, which developed disease symptoms similar to that of controls (unvaccinated with no lymphocyte transfer) following A/PR8 challenge (**Figure 14A, C & D**). These adoptive transfer studies further support a critical role for T cells, but not B cells, in cross protective immunity against A/PR8 challenges.

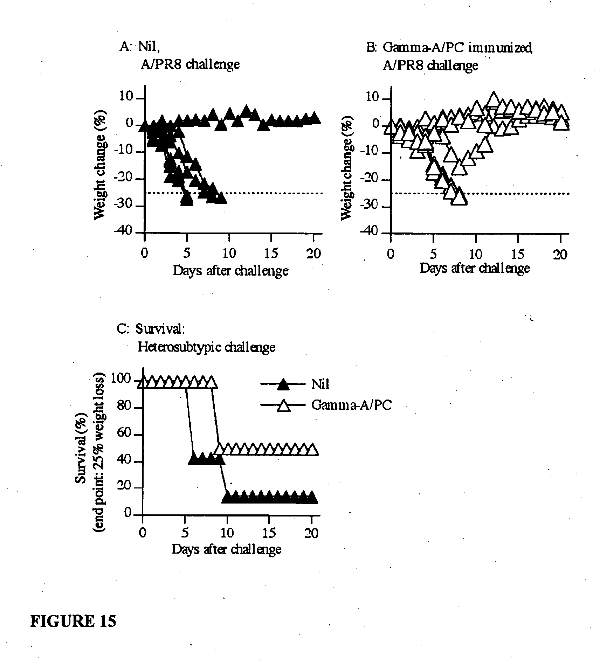

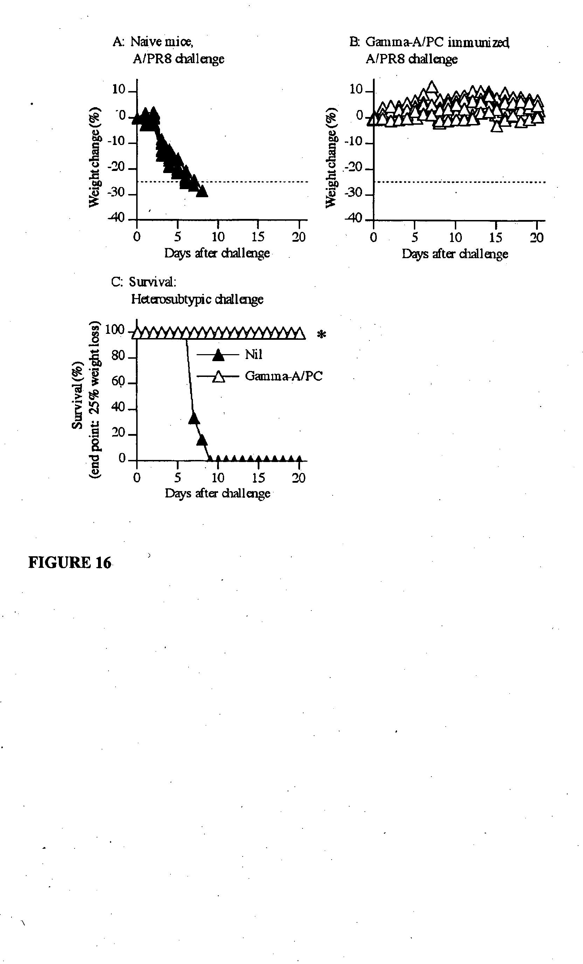

CD8+ T cells exert antiviral effects by either directly killing virus-infected cells or secreting cytokines such as IFN-$\gamma$ and TNF. To delineate which effector function of T cells provides heterosubtypic immunity, prf$^{-/-}$ mice (which lack perforin-mediated lytic function) and IFN-IIR$^{-/-}$ mice (whose immune cells are unresponsive to IFN-y) were utilised. Prf$^{-/-}$ mice were immunized intranasally with $\gamma$-irradiated A/PC (3.2 x 10$^6$ PFU equivalent). 3 weeks post-immunization, naive and immunized mice (9~10 mice per group) were challenged with the heterosubtypic strain A/PR8 (7 x 10$^2$ PFU). Mice were monitored daily for weight loss and mortality for 20 days. Vaccination with $\gamma$-irradiated A/PC failed to confer significant cross-protection to prf$^{-/-}$ mice **(Figure 15A, B & C)**. This strongly suggests that cross protection induced by $\gamma$-irradiated A/PC requires perforin-mediated lytic function, which is associated with CD8+ T and NK cells.
In contrast, IFN-IIR$^{-/-}$ mice immunised with $\gamma$-irradiated A/PC (same conditions) were fully protected against a lethal challenge with A/PR8 **(Figure 16A, B & C)**. Thus, IFN-$\gamma$ function is dispensable for the induction of the cross-protective immunity.

*Absence of cross-neutralizing activity in the serum of $\gamma$-irradiated A/PC immunized mice*

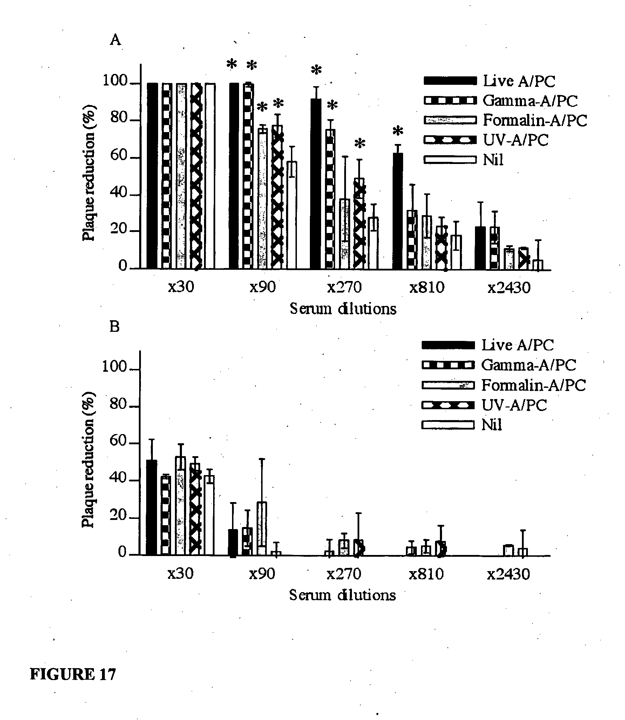

As noted in **Example 3** above, immunisation with $\gamma$-irradiated (but not formalin- or UV-inactivated) influenza viruses induce cross-protective immunity. Hence, the cross-neutralizing activity of the immune sera induced by variously inactivated influenza formulations against homologous and heterosubtypic strains of influenza A viruses was tested. Viral neutralizing activities against A/PC (H3N2) or A/PR8 (H1N1) were determined by plaque reduction assays for sera collected 3 weeks after immunization with live, $\gamma$-irradiated, formalin or UV-inactivated A/PC. After heat inactivation of serum samples at 56°C for 30 minutes, 190 $\mu$l of serially diluted (x10, x30, x90, x270) sera were mixed with 10$\mu$l virus suspension containing roughly 1 x10$^2$ PFU. After 60 minutes incubation the virus/serum mixtures were added to six well plates for plaque assay. Immune sera collected from all vaccinated animals contained high levels of neutralizing activity against the homologous strain A/PC (H3N2) **(Figure 17A)**. The same immune sera, when tested against a heterosubtypic strain A/PR8 (H1N1), showed levels of neutralizing activity similar to that of naive sera **(Figure 17B)**. These data demonstrate that immunization with any of the inactivated influenza virus preparations, including $\gamma$-irradiated influenza viruses, induces highly strain-specific neutralizing antibodies with limited, if any, cross neutralising activity.

*The induction and magnitude of cross-reactive Tc cell response by $\gamma$-irradiated A/PC is dose-dependent*

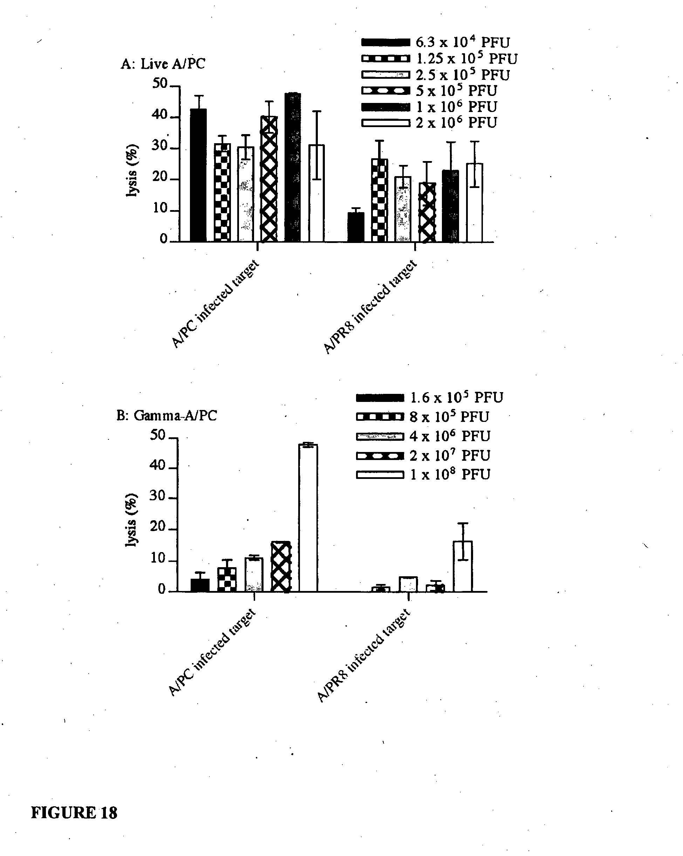

The absence of serum cross-neutralising activity between H3N2 and H1N1 influenza viruses, lack of cross-protective immunity in defined knock-out mice, and the results from adoptive transfer experiments indicate that cellular immunity plays a pivotal role in protecting mice against heterosubtypic challenges. To characterize the cytolytic function of T cells in vaccinated mice, mice were intravenously immunized with various doses of either live or $\gamma$-irradiated A/PC and their splenocytes tested for specific target cell killing 6 days post-immunization. Groups of two BALB/c mice were immunized intravenously with various doses of either live or $\gamma$-irradiated A/PC and splenocytes were harvested on day 6 post-immunization. Splenocytes were used as effector cells against mock treated or A/PC or A/PR8 infected P815 target cells. Live A/PC elicited strong Tc cell responses over a wide range of immunization doses **(Figure 18A)**. Immunization with a high dose (10$^8$ PFU equivalent) of $\gamma$-irradiated A/PC also elicited a strong Tc cell responses against both A/PC and A/PR8 infected targets **(Figure 18B)**. However, immunization with low doses (1.6 x 10$^5$ PFU equivalent or less) did not induce statistically significant cross-reactive Tc cell responses. Thus, the magnitude of the Tc cell response by $\gamma$-irradiated A/PC correlates with immunization dose.

*Induction of memory Tc cells by $\gamma$-irradiated A/PC*

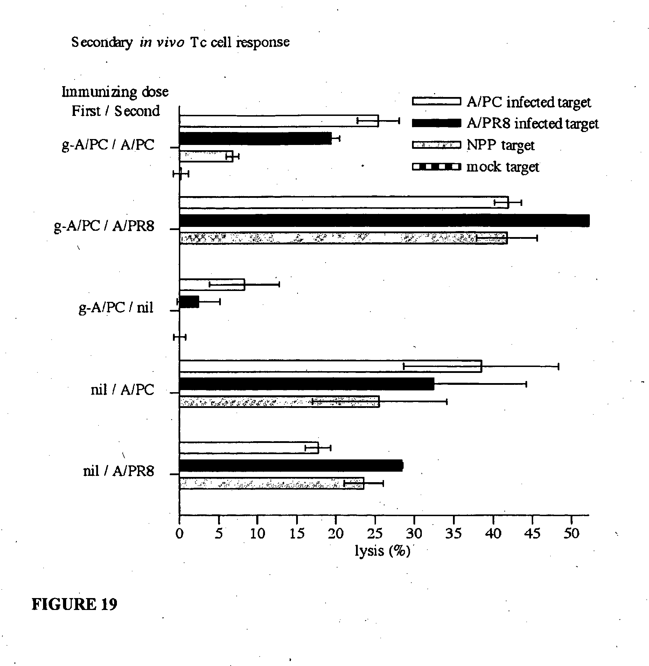

As noted in **Example 3** above, immunization with $\gamma$-irradiated A/PC provides cross-protection for at least 3 months. Hence, the longevity of memory T cells that may account for the long lasting protection was investigated. Groups of two BALB/c mice were intravenously immunized once or twice with either live A/PC (2 x 10$^6$ PFU), A/PR8 (1 x 10$^7$ PFU) or $\gamma$-irradiated A/PC (1 x 10$^8$ PFU equivalent). The secondary immunization was given 3 weeks after priming. Splenocytes were harvested 7 days after the second immunization and used as effector cells against mock, A/PC- or A/PR8 infected P815 target cells or labelled with $K_d$ restricted nucleoprotein derived peptide (NPP). The secondary immunization with live heterosubtypic strain A/PR8 induced a strong secondary Tc cell response **(Figure 19)**. In contrast, mice that received live homologous strain A/PC as a secondary immunization showed no increase in Tc cell potency **(Figure 19)**.

(iii) Discussion

As noted in the Examples above, $\gamma$-ray inactivated influenza A virus, especially when administered intranasally, confers robust protection against lethal homologous and heterosubtypic virus challenges, including the virulent avian H5N1 strain. These data demonstrate that cross-reactive cellular immunity mediated predominantly by Tc cells, and not humoral immunity, is the essential element responsible for the cross protective immunity induced by γ-irradiated influenza virus. This conclusion is based on several independent lines of evidence: 1) β2M$^{-/-}$ mice did not generate cross protective immune responses; 2) transfer of enriched T cells, but not of B cells or immune sera, from γ-inactivated influenza immune donor mice conferred cross reactive immunity to naive recipients; and 3) cross-protective immunity induced by the preparation was dependent on the essential cytolytic effector molecule perforin.

The latter observation, together with the induction of cross-protective immunity induced in IFN-IIR$^{-/-}$ mice strongly suggests that cytolytic Tc cells provide protection through killing of virus-infected cells via their granule-exocytosis pathway of cytotoxicity, and not by CD8 + T cell IFN-γ-mediated virus control. Consistent with the above, immunization with γ-irradiated influenza virus can elicit similar T cell responses to that induced by live virus. This cross reactive T cell potential persists for at least 3 months and led to a stronger secondary Tc response upon *in vivo* re-immunization. This correlates with the observation that mice immunised with γ-irradiated influenza virus are well protected against heterosubtypic challenge for at least 3 months. However, the immunization with a homologous strain did not enhance the secondary Tc response. This observation suggests that the primary immunization elicited highly strain specific antibody responses which neutralized the secondary challenge with the homologous virus, thus preventing memory Tc cell activation.

Induction of cross-reactive Tc cell responses was highly dose dependent on γ-irradiated virus in contrast to replicative live virus. With Tc cells identified as the dominant factor in providing heterosubtypic protection, there was still some evidence of a contribution by B cells to this protective response, since μMT$^{-/-}$ mice also showed increased susceptibility to heterosubtypic challenge. The absence of cross-neutralizing antibody responses in serum as well as a lack of protective effect of transferred serum suggests that the contribution of B cells is independent of their principle soluble product, antibody. In certain circumstances, Naive B cells are thought to be capable to restore immunity in μMT$^{-/-}$ mice against secondary infections in an antibody-independent manner. In addition, B cells may have a role in promoting effector Tc cell function. Hence, the possible role of cross protective antibodies cannot be neglected and mucosal cross-neutralizing antibodies may also be contributing to heterosubytpic immunity. The observed partial cross protection, although not statistically significant, in β2M$^{-/-}$ and prf$^{-/-}$ mice supports this notion. Moreover, passive immunity obtained by adoptive T cell transfer was only partially successful in protecting against a sublethal dose of virulent A/PR8. Thus, both antibodies as well as Tc cells may ultimately contribute to optimal cross-protection.

## Example 5: Superiority of γ-ray inactivated-influenza vaccine compared to UV-inactivated and formalin-inactivated influenza vaccines

(i) Materials and Methods

*Mice*

Nine- to ten-week-old female BALB/c mice were routinely used in these studies.

*Viruses and cells*

P815 mastocytoma, Madin-Darby canine kidney (MDCK) and baby hamster kidney (BHK) cells were grown and maintained in EMEM plus 5% FCS at 37°C in a humidified atmosphere with 5% CO$_2$. The influenza type A viruses, A/PR/8 [A/Puerto Rico/8/34 (H1N1)] and A/PC [A/Port Chalmers/1/73 (H3N2)] were grown in 10-day-old embryonated chicken eggs. Each egg was injected with 0.1 ml normal saline containing 1 hemagglutinin unit (HAU) of virus, incubated for 48 hours at 37°C, and held at 4°C overnight. The allantoic fluids were then harvested, pooled and stored at -80°C. Titres were 10$^7$ PFU/ml (A/PC) and 2 x 10$^8$ PFU/ml (A/PR8) using plaque assays on MDCK cells. Viruses were purified using chicken red blood cells for vaccine preparation as described in (Sheffield, et al. (1954), "Purification of influenza virus by red-cell adsorption and elution", British journal of experimental pathology, 35:214-222). Briefly, infectious allantoic fluid was incubated with red blood cells for 45 minutes at 4°C allowing the hemagglutinin to bind red blood cells, and then centrifuged to remove the allantoic fluid supernatant. The pellets were suspended in normal saline, incubated for 1 hour at 37°C to release the red blood cells from the virus and then centrifuged to remove the red blood cells and collect the virus in the supernatant. Purified A/PC stock titre was 5 x 10$^8$ PFU/ml.

*Virus inactivation*

For formalin inactivation, the viruses were incubated with 0.2 % formalin at 4°C for a week. The formalin was then removed by pressured dialysis using normal saline for 24 hours at 4°C. The dialysis method was adapted from Current Protocols in Immunology (see Andrew et al.,. (2001), "Dialysis and concentration of protein solutions", in Current protocols in immunology, Coligan et al. (eds), Appendix 3: Appendix 3H). For UV inactivation, the viruses were placed in 60-mm petri dishes with a fluid depth of 10 mm. The virus was exposed to 4000 ergs per $cm^2$ for 45 minutes at 4°C. For $\gamma$-ray inactivation, influenza viruses received a dose of 10kGy from a $^{60}$Co source (Australian Nuclear Science and Technology Organization - ANSTO). The virus stocks were kept frozen on dry ice during $\gamma$-irradiation. Loss of viral infectivity was confirmed by titration of inactivated virus preparations in eggs. The HAU titres of inactivated virus stock were determined to be 7.29 x $10^4$ HAU/ml for $\gamma$-A/PC, 2.43 x $10^4$ HAU/ml for formalin- and UV-A/PC.

*Freeze-drying of $\gamma$-ray inactivated-influenza*

For freeze-drying, one vial containing 0.5 ml of $\gamma$-ray inactivated A/PC was placed in a Manifold Freeze-Dryer (FTS SYSTEMS, Dura-DryTM MP).

*Hemagglutination assay*

Live and inactivated virus preparations were serially diluted in a 100 $\mu$l volume on 96-well U-bottom microtiter plate. 0.5% chicken red blood cell suspensions were added to all wells and plates were incubated for 30 minutes on ice. This method was adapted from Current Protocols in Microbiology (see Szretter et al. (2006), "Influenza: propagation, quantification, and storage", in Current Protocols in Microbiology, Coico et al. (eds), Chapter 15: Unit 15G 11).

*Protection experiment*

BALB/c mice were bred under specific pathogen-free conditions. 10-14-week-old females were used. Mice were immunized intranasally with inactivated virus preparations (3.2 x $10^6$ PFU equivalent) or trivalent inactivated subunit influenza vaccine (CSL fluvax vaccine; A/Solomon Islands/3/2006 H1N1,A/Brisbane/10/2007 H3N2, B/Florida/4/2006; 3 mg hemagglutinin). The formalin inactivated A/PC vaccinated mice were re-immunized once or twice 2 and 3 weeks later. For lethal challenge, at 1-3 weeks post-immunization, mice were infected intranasally with 50% mouse lethal dose (MLD50). MLD50 was determined to be 7x$10^2$ PFU and 3.2 x $10^5$ PFU for A/PR8 and A/PC, respectively, in preliminary experiments. For analysis of lung virus titers, 3 mice were euthanized on day 3 and 6 post-challenge. The remaining animals were monitored for body weight and mortality until day 20 post-challenge.

*Plaque assay*

The lung tissue samples were collected 3 and 6 days after intranasal challenge. After removal, whole lungs were homogenized in normal saline. Homogenates were centrifuged at 1500 rpm for 5 minutes. Supernatants were collected and were stored at - 20°C. Serial dilutions of the samples were inoculated on MDCK cells cultured on 6-well tissue culture plates. After 1 hour adsorption, the cells were overlaid with EMEM medium containing 1.8% Bacto-Agar. After incubation for 2-3 days, cell monolayers were stained with 2.5% crystal violet solution and the plaques were enumerated.

*Lung-histology*

Lung tissue samples were fixed for a minimum of 24 hours in 10% neutral buffered formaldehyde. 10$\mu$m sections were stained with Haemtoxilin-Eosin and evaluated by light microscopy.

*Cytotoxic T Lymphocyte (Tc cell) Assay*

Influenza-specific Tc cells were generated by intravenously injecting BALB/c mice with either live A/PC or inactivated, $10^8$ PFU equivalent, A/PC (□-irradiated, formalin-, or UV-inactivated). Spleens were harvested at 7 days post immunization and red blood cell-depleted cell suspensions were prepared for use as effector cells. Target cells were prepared by infecting P815 cells at a multiplicity of infection (m.o.i) of 1 for live A/PC and 10 for inactivated A/PC, followed by 1 hour incubation in medium containing 100~200 □Ci of $^{51}$Cr. After washing, target cells were mixed with effector cells at different ratios in an 8 hour chromium release assay. The level of radioactivity in the supernatant was measured in a gamma counter. Specific lysis is given as mean percent lysis of triplicate wells and values were calculated using the formula: (experimental cpm - spontaneous cpm)/(maximal release cpm - spontaneous cpm) x100.

(ii) Results

*The effect of virus inactivation on hemagglutination activity*

Hemagglutination activity after virus inactivation provides one indicator as to the denaturing effect of the sterilization treatment. Purified influenza stock was aliquoted into batches and treated with either formalin, UV or $\gamma$-irradiation. Following complete inactivation of infectivity verified by the absence of virus growth in embryonated eggs, the hemagglutination activity of live and inactivated viruses was compared **(Table 7)**.

**Table 7.** *Hemagglutination activity of inactivated influenza virus A/PC preparations.*

| Strain | Method of inactivation | HAU/ml |
|---|---|---|
| A/PC | Original live purified stock | $2.2 \times 10^5$ |
| | Gamma-ray inactivation | $7.3 \times 10^4$ |
| | Formalin-inactivation | $2.4 \times 10^4$ |
| | UV-inactivation | $2.4 \times 10^4$ |

Hemagglutination activity was reduced by 3-fold for $\gamma$-irradiated viruses, whereas formalin and UV inactivation resulted in 9-fold reduced hemagglutination titres. These results provide evidence that, of these three virus sterilization methods, y -irradiation denatures viral protein structure least.

*Gamma-irradiated, but not formalin or UV-inactivated, virus preparations induce heterosubtypic immunity*

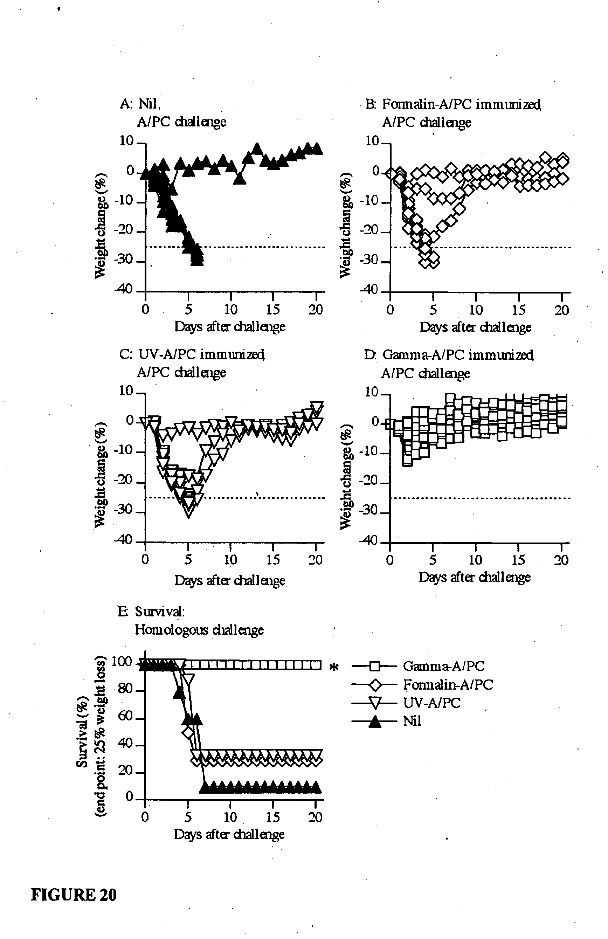

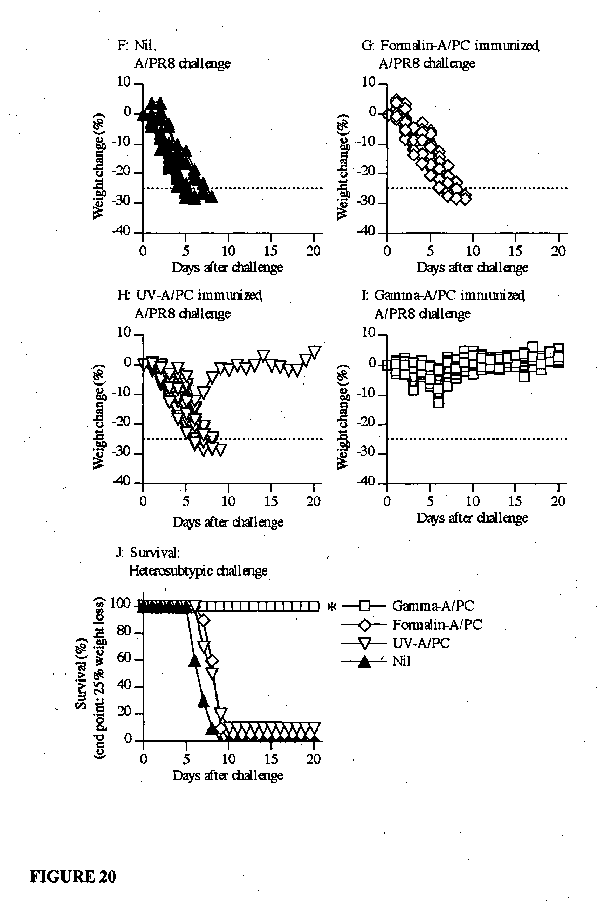

The protective efficacy of $\gamma$-irradiated, formalin-, or UV inactivated influenza virus preparations against homo- and heterosubtypic live virus challenges was compared. Groups of 9-10 BALB/c mice were mock treated or immunized intranasally either with formalin-, UV- or $\gamma$-ray inactivated A/PC ($3.2 \times 10^6$ PFU equivalent) and at week 3 after the immunization, naive and immunized mice (9~10 mice per group) were intranasally infected with A/PC (MLD50; $3.2 \times 10^5$ PFU) or A/PR8 (MLD50; $7.0 \times 10^2$ PFU). Survival of infected mice was monitored daily for 20 days. As shown in **Figure 20 A, E, F & J,** intranasal infection of naive mice with A/PC or A/PR8 caused a rapid weight loss with 90-100% mortality (based on 25% weight loss as an end point). Mice immunized with either formalin inactivated A/PC **(Figure 20B & E)** or UV-inactivated A/PC **(Figure 20C & E)** also developed significant weight loss and resulted in ~70% mortality when challenged with live homologous virus. When similarly vaccinated mice were challenged with the heterosubtypic strain A/PR8, the animals lost substantial body weight with 90~100% mortality **(Figure 20G, H, & J).** In both cases, homologous and heterosubtypic challenge, the induced protection was considered inadequate to be used as a vaccine (P-value > 0.05, Fisher's exact test). In contrast, mice immunized with a single dose of $\gamma$-inactivated A/PC were not only protected against homologous virus challenge, but also against heterosubtypic challenge, with mice losing only 5% of their body weight on average **(Figure 20D, E, I & J).** Hence, $\gamma$ -irradiated influenza virus proved by far to be the most effective vaccine preparation to induce protective immunity against homo- and heterosubtypic influenza virus challenges (P < 0.05).

*Can multiple doses of formalin-inactivated influenza virus preparation enhance the protective effect?*

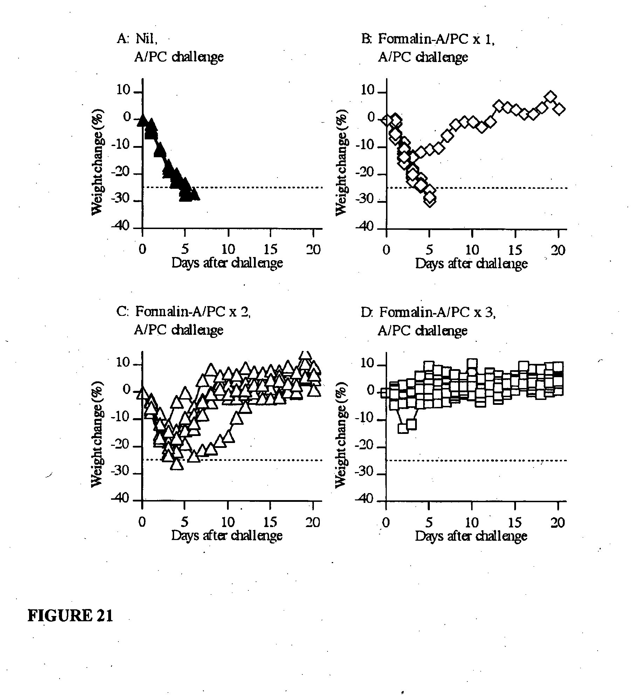

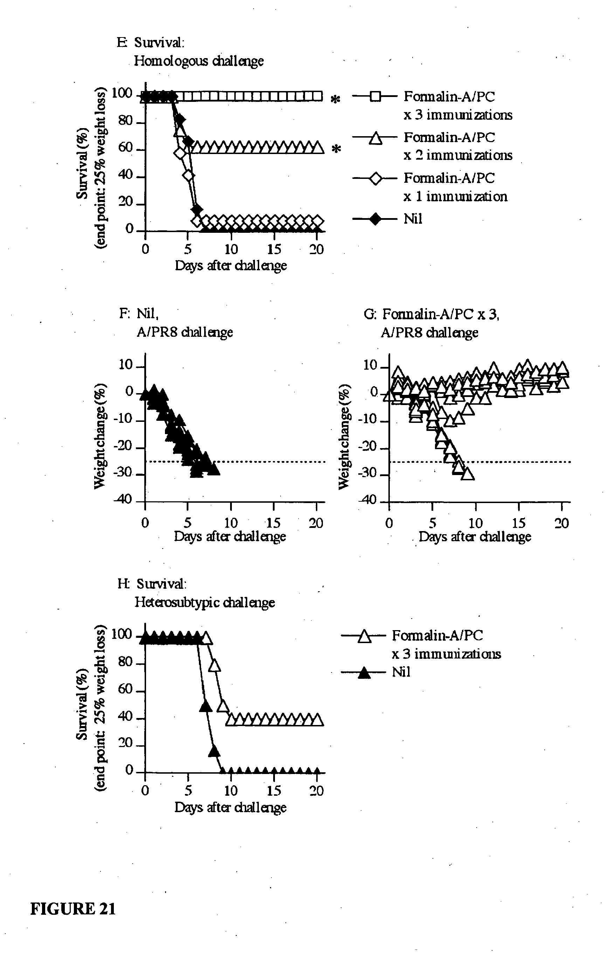

$\gamma$-ray inactivated A/PC was clearly more effective after only one intranasal dose than multiple intranasal administrations of formalin-inactivated preparations. It was then determined whether the weak protective efficacy of formalin-inactivated A/PC could be improved by testing different immunization schedules. Groups of 9-10 BALB/c mice were mock treated or immunized either once, twice or three times with formalin-inactivated A/PC ($9.6 \times 10^6$ PFU equivalent or HAU dose equivalent to that of $\gamma$-ray inactivated A/PC; 2300 HAU). Mock treated or single dose immunized mice were challenged with A/PC (MLD50; $3.2 \times 10^5$ PFU) at three weeks post immunization. Double or triple dose immunized mice were intranasally infected with A/PC (MLD50; $3.2 \times 10^5$ PFU) or A/PR8 (MLD50; $7 \times 10^2$ PFU) one week after the final immunization. Survival of infected mice were monitored daily for 20 days. The group of mice that received a single immunization had no improved survival rate compared to that of unvaccinated mice **(Figure 21A, B & E)**. In contrast, double immunization improved the survival rate to 60% (P < 0.05) although the majority of mice still showed a significant loss in bodyweight, indicating that they experienced severe illness **(Figure 21C & E)**. The mice receiving triple immunization with formalin-inactivated A/PC showed complete protection with no mortality and little weight loss **(Figure 21D & E)**. Triple immunization conferred partial protection from heterosubtypic challenge (P < 0.05) **(Figure 21F, G & H)**. Thus, formalin-inactivated A/PC requires more doses and fails to elicit the cross-protection suggesting that the induced immunity is not only quantitatively, but also qualitatively, substantially inferior to that induced by $\gamma$-ray inactivated A/PC.

*Trivalent flu vaccine is ineffective against drifted strains*

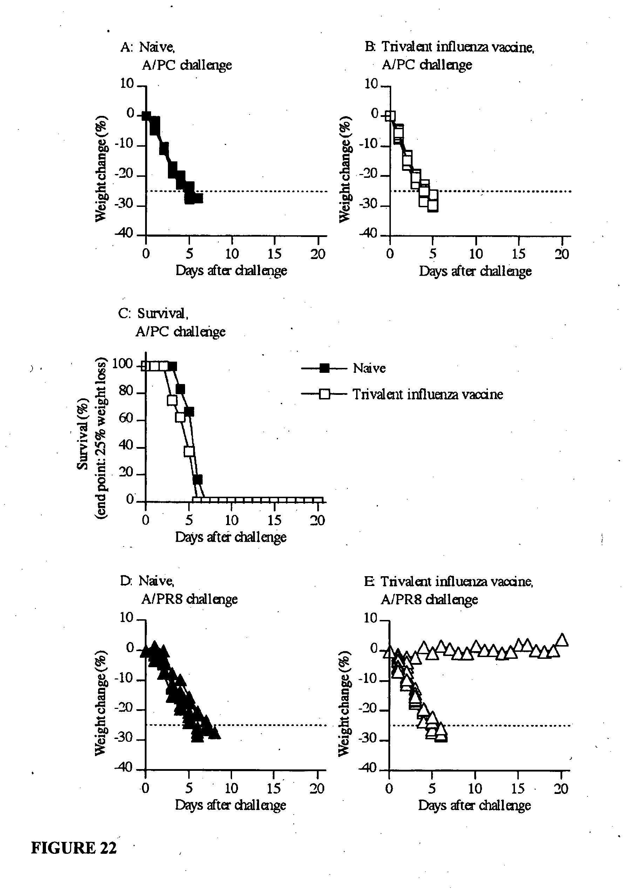

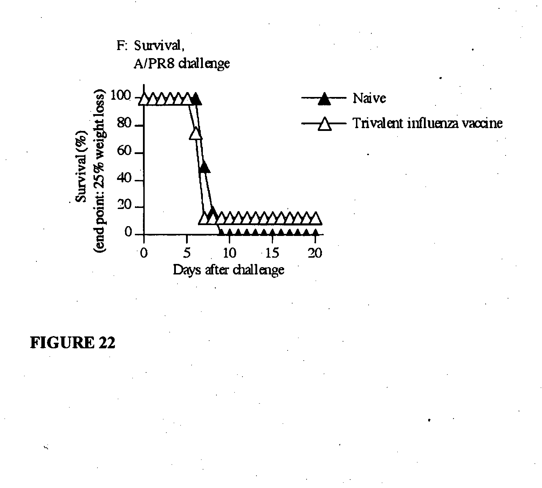

For a direct comparison, the protective efficacy of a commercially available trivalent influenza vaccine was tested in the experimental approach described herein. Mice were immunized once intranasally with trivalent influenza vaccine (CSL fluvax vaccine; A/Solomon Islands/3/2006 H1N1, A/Brisbane/10/2007 H3N2, B/Florida/4/2006; 3μg hemagglutinin) and at 3 week post immunization, naive and immunized mice were intranasally challenged with either A/PC (3.2 x $10^5$ PFU/mouse) or A/PR8 (7 x $10^2$ PFU). Survival of infected mice was monitored daily for 20 days. As shown in **Figure 22,** single intranasal immunization of mice conferred no statistically significant protection (P > 0.05) against both A/PC (3.2 x$10^5$PFU) **(Figure 22A, B & C)** and A/PR8 strain (7 x $10^2$ PFU) **(Figure 22D, E & F).** This clearly shows that the current influenza vaccine does not confer appreciable cross-protection after a single dose, even against strains within the same subtype.

*Minimal influenza infection-induced lung inflammation after vaccination with γ-ray inactivated A/PC*

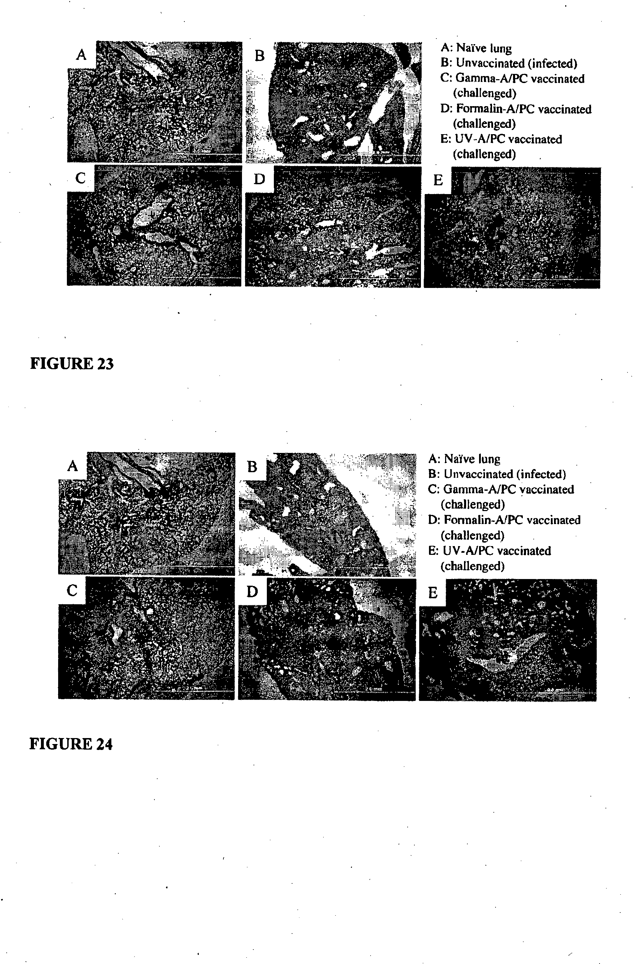

Three weeks following vaccination (3.2 x $10^6$ PFU equivalent) with γ-irradiated, formalin- and UV-A/PC, mice were challenged with either A/PC (homologous) or A/PR8 strain (heterosubtypic) of live virus. Lungs of surviving mice were harvested 21 days post-challenge and lungs processed for histology. The lung samples displayed remarkable histological differences, corresponding to the type of immunization given. As shown in **Figure 23,** limited inflammatory responses were seen when vaccinated mice were challenged with the homologous virus A/PC. Lung sections from all three vaccinated groups (γ-irradiated, formalin or UV-inactivated A/PC) were comparable in their appearance to that of naive tissue **(Figure 23 A, C, D, & E)**. In contrast, lung tissues from unvaccinated, A/PC-challenged, mice showed extensive inflammatory responses **(Figure 23B)**. The heterosubtypic challenge resulted in clear differences among the various vaccinated groups.

The inflammatory responses in formalin- and UV-A/PC vaccinated animals were strong and similar to that of the unvaccinated animals following A/PR8 challenge 21 days post-vaccination (**Figure 24B, D & E**). In contrast, lung inflammation in γ-irradiated A/PC vaccinated mice was limited following heterosubtypic challenge with A/PR8 (**Figure 24C**). Although these lungs exhibited localised inflammation with weak lymphocyte infiltration, the overall condition was similar to that of naive lungs (**Figure 24A**).

*Gamma-ray inactivated A/PC vaccine does not prevent infection but facilitates viral clearance*

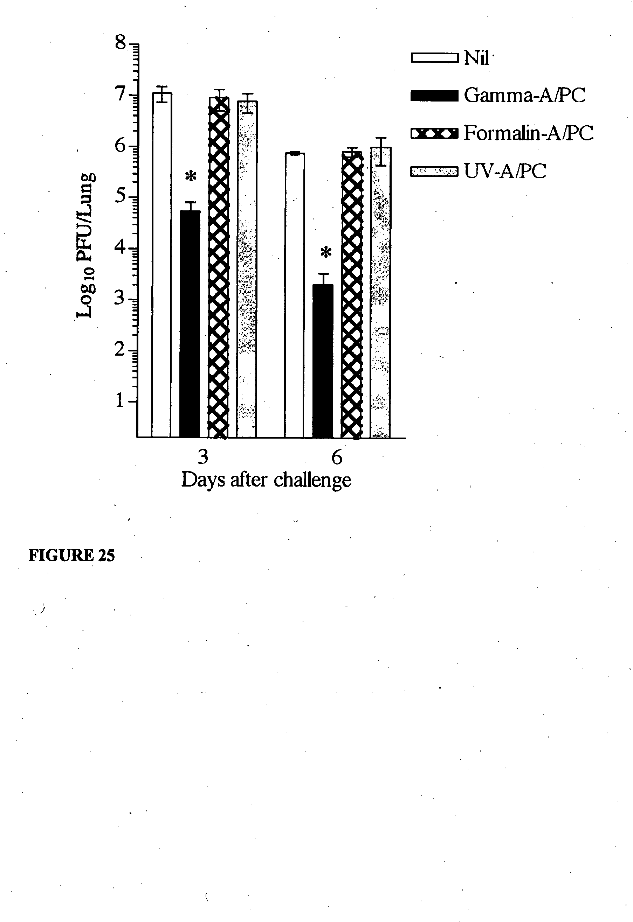

The effect of vaccination on pulmonary viral load at days 3 and 6 after heterosubtypic challenge with A/PR8 was evaluated. BALB/c mice were intranasally immunized either with γ-irradiated, formalin or UV inactivated A/PC (3.2 x $10^6$ PFU equivalent) and at week 3-post immunization, naive and immunized mice were intranasally challenged with A/PR8 virus (MLD50). On day 3 and 6 post infection, three mice per group were sacrificed and the viral titres in lungs determined by the plaque assay using MDCK cells as described above. High virus titres reaching $10^7$ and $10^6$ PFU/lung for days 3 and 6 post-infection, respectively, were detected in unvaccinated mice (**Figure 25**). Virus titres in the lungs of formalin- and UV-inactivated A/PC immunized mice were comparable to those detected in unvaccinated control mice. In contrast, the γ -irradiated A/PC vaccinated group showed a > 100-fold reduction of A/PR8 lung virus titres both at days 3 and 6 post-challenge (P < 0.05 using Student's T test) compared to that seen in unvaccinated control mice.

*Gamma-irradiated, but not formalin- or UV-inactivated virus retains Tc cell immunogenicity*

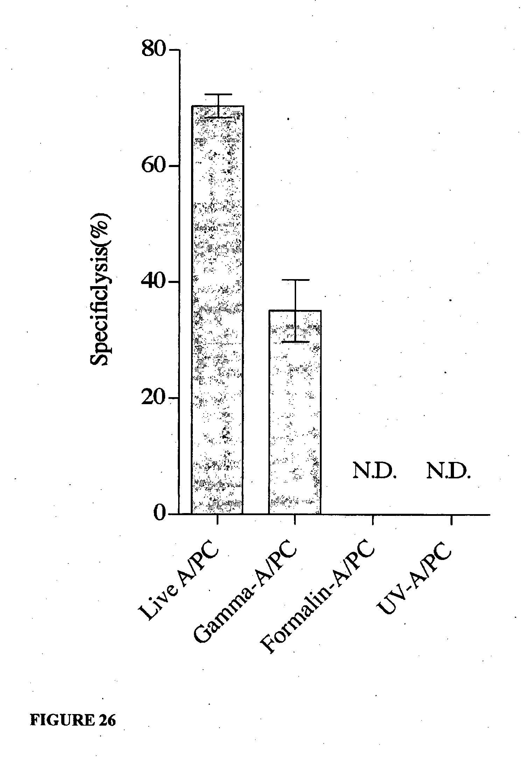

The ability to generate influenza-immune cytotoxic T (Tc) cell responses by live A/PC and inactivated A/PC (γ-ray, formalin-, and UV) was compared. BALB/c mice were intravenously immunized with live, γ-irradiated, formalin-, or UV-inactivated A/PC. Splenocytes were harvested 7 days post immunization and were used as effector cells against A/PC infected P815 target cells. The peak of the Tc cell response following live virus infection was detected at day 7 post immunization (data not shown). On day 7 after intravenous immunization two mice from each group were assessed. Effector splenocytes harvested from mice immunized with live ($10^7$ PFU) or γ-ray inactivated A/PC ($10^8$ PFU equivalent) lysed A/PC infected target cells, whereas effector cells from formalin- or UV-inactivated A/PC immunized mice did not (**Figure 26**).

*Intranasal immunization with γ-ray inactivated A/PC confers protection against high dose heterosubtypic challenges.*

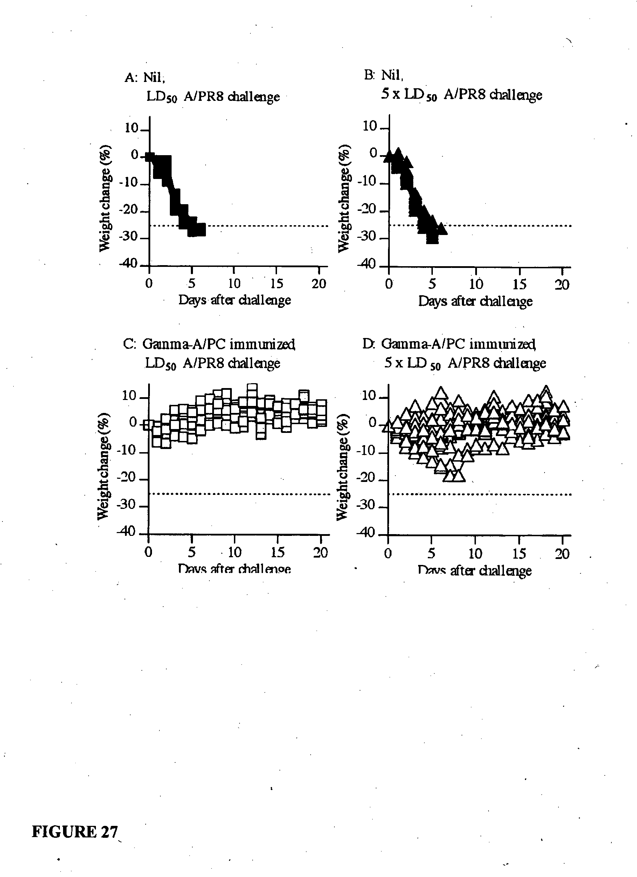

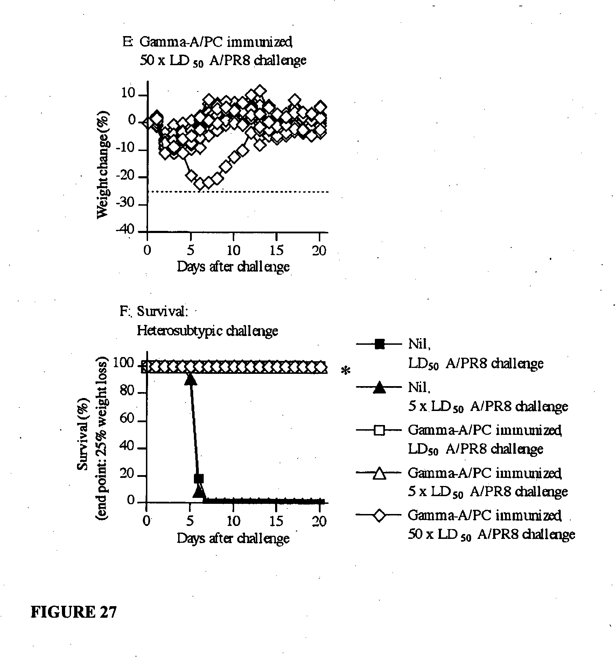

Given the excellent protective capacity of γ-irradiated A/PC to protect mice from heterosubtypic challenge, the limit of protection was investigated by challenging with increased influenza virus doses **(Figure 27)**. Groups of 9-10 BALB/c mice were mock treated or immunized intranasally with γ-ray inactivated A/PC (3.2 x $10^6$ PFU/ml equivalent) and at 3 weeks post immunization mice were intranasally challenged with either LD50 A/PR8, 5 x LD50 A/PR8, or 50 x LD50A/PR8. Survival and weight loss of infected mice was monitored for 20 days. Immunized mice receiving heterosubtypic challenge of 1 x LD50 all survived and there was little or no weight loss **(Figure 27C & F)**. Immunized mice given a challenge dose of 5 x LD50 initially lost weight during the first 7 days post-challenge, but not significantly, and all fully recovered **(Figure 27D & F)**. The mice receiving 50 x LD50 lost on average 8% of body weight but here, too, all mice fully recovered **(Figure 27E & F)**. Naive mice receiving 1 x LD50 or 5 x LD50 progressively lost weight and failed to survive the challenge **(Figure 27A, B & F)**.

*Long-lived heterosubtypic protection conferred by γ-ray inactivated preparations*

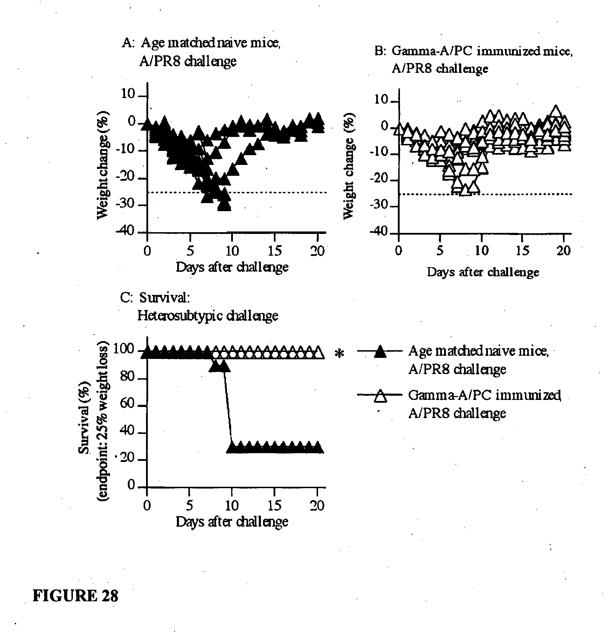

A critical requirement for an effective influenza vaccine is the induction of persistent heterosubtypic immunity. Groups of 9-10 BALB/c mice were either mock treated or immunized intranasally with γ-ray inactivated A/PC ($3.2 \times 10^6$ PFU equivalent) and at 3 months post immunization mice were intranasally challenged with MLD50 A/PR8 ($7 \times 10^2$ PFU). Survival and weight loss of infected mice was monitored for 20 days. The vaccinated mice challenged with 1 x LD50 A/PR8 lost on average only up to 10% body weight and fully recovered **(Figure 28B & C)**. In contrast, the majority of challenged naive mice lost substantial weight, reaching an end point of 25% total body weight loss at around 7 days post challenge **(Figure 28A & C)**.

*Freeze-drying does not destroy the immunogenicity of γ-ray inactivated-A/PC*

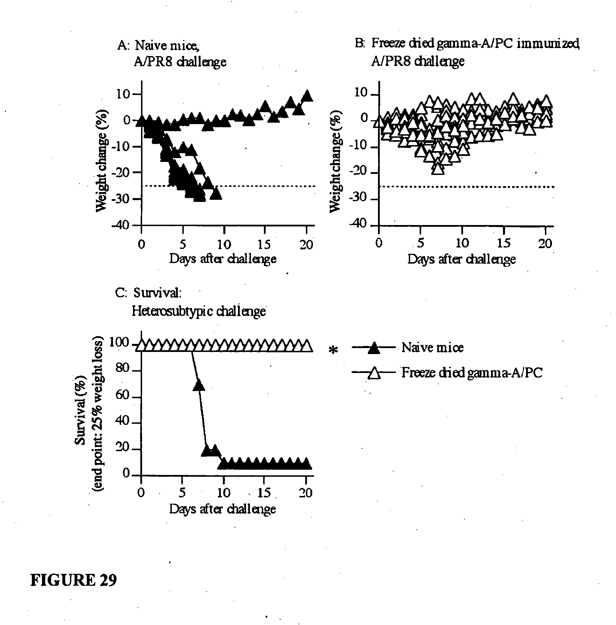

A known shortcoming of the current liquid based influenza vaccine is the requirement of refrigerated storage that imposes a problem for vaccine distribution, particularly in developing countries. In an attempt to overcome the stringent storage requirement of the current influenza vaccine, freeze-drying γ-ray inactivated influenza virus was assessed as a means to curtail refrigeration requirements. Gamma-ray inactivated A/PC stock was freeze-dried and resuspended in distilled water immediately prior to intranasal administration ($3.2 \times 10^6$ PFU equivalent). Groups of 9-10 BALB/c mice were either mock treated or immunized with freeze-dried γ-ray inactivated A/PC and challenged with heterosubtypic strain A/PR8 ($7 \times 10^2$ PFU) at week 3-postimmunization. Survival and weight loss of mice was monitored daily for 20 days. The majority of mice lost less than 10% total body weight and only 2/10 mice lost over 10% total body weight showing mild symptoms. All vaccinated mice survived the heterosubtypic challenge with A/PR8 ($7 \times 10^2$ PFU) as opposed to 10% survival in naive mice **(Figure 29A, B & C)**. These data suggest that the freeze-drying process does not markedly reduce the ability of γ-ray inactivated A/PC to induce heterosubtypic immunity.

*Superiority of γ-ray inactivated-influenza compared to split vaccine*

A comparison of the cross-protective efficacy of the commercially available flu vaccine, Flu-vax, with gamma-irradiated purified influenza virus after intranasal vaccination in mice revealed the following:

**Table 8:** *cross-protective immunity induced by γ-flu vs flu-vax*

| | | | Protection | |
|---|---|---|---|---|
| | μg HA | pfu equiv. | Homosubtypic | Hetersubtypic |
| Flu- Vax | 3 | $9 \times 10^7$ | +- | --- |
| γ- irradiated influenza vaccine | $4.5 \times 10^{-2}$ | $3 \times 10^6$ | +++ | ++ |

Accordingly, gamma-irradiated influenza virus is on an equivalent virus dose basis at least 30-100 times more efficient than the commercially available flu vaccine and is superior in quality to the present commercially available flu vaccine.

(iii) Discussion

The present study evaluated in a comparative setting the protective efficacy of three types of inactivation regimens; γ-irradiation, and formalin-, or UV- inactivation, to assess whether the currently used chemical inactivation method, used since 1945, is the most suitable choice for influenza vaccine preparation. It was shown that γ-ray inactivated A/PC ($3.2 \times 10^6$ PFU equivalent, 2300 HAU) had superior immunogenicity compared to the other sterilization methods, and confers a high level of protection against both homologous and heterosubtypic challenges. This superior protection was reflected in 100% survival and lower weight loss, which correlated with histological evaluations of lung tissues after infection as well as reduced lung viral load compared to naive and formalin- or UV- inactivated-virus vaccinated mice. Similarly, single doses of a currently used trivalent influenza vaccine provided no protection against A/PC or A/PR8 challenges.

Three-fold higher doses of formalin-inactivated A/PC (9.6 x $10^6$ PFU equivalent, 2300 HAU) and multiple immunizations were required to gain the level of protection afforded by γ-ray inactivated A/PC. Furthermore, formalin-inactivated A/PC conferred protection only against homologous (and not against heterosubtypic) virus challenge. Therefore, an increase in dose and frequency of immunization only improves the strain-specific immunity of formalin-inactivated virus. It is important to note that for per virus particle inactivated, γ-ray inactivated virus is more immunogenic than formalin-inactivated virus since a formalin-inactivated virus preparation required three times more PFU for a comparable HAU dose and triple immunizations, as opposed to single priming for γ-irradiated A/PC, to obtain strain specific immunity. These findings demonstrate that γ- ray inactivation maintains superior antigenicity and immunogenicity relative to the other procedures. Thus γ-ray inactivated virus could induce immunity that is not only quantitatively but also qualitatively superior to virus preparations inactivated by formalin treatment or UV-irradiation.

In the event of a pandemic, a single dose regimen, as promised by γ-irradiated virus, would be incomparably more desirable than a multiple, high dose, formalin-inactivated influenza vaccine immunization regimens which also require adjuvants. Moreover, the fact that no adjuvants are required for γ-ray inactivated influenza suggests that reactogenicity problems are less likely to be encountered. Alum is most commonly used adjuvant for human vaccines but it has been proven to be ineffective in enhancing the immunogenicity of influenza vaccine antigens. In addition, alum skews the immune response towards T helper (Th) type 2-supported humoral immune responses which may reduce the effectiveness of γ-ray inactivated virus, as the latter is known to induce Th1-type cellular immune responses, including Tc cell responses that correlate with heterosubtypic protection. Furthermore the efficacy of γ-irradiated influenza virus is highlighted by the fact that after a single dose of intranasal priming, the immunized mice were able to resist heterosubtypic challenge doses of up to 50 x LD50, for a period of up to 3 months, underscoring the robust immunity induced.

It is speculated that the cross protection induced by γ-irradiated virus is mediated by mucosal Tc cell responses. An alternative hypothesis is induction of cross-reactive secretory IgA antibodies to internal viral proteins. Some secretory IgA antibodies are capable of intracellular neutralization of influenza virus during transcytosis into the infected epithelial cells, and the present data suggests that cross-reactive Tc cells may be responsible for the cross protection observed here as other forms of inactivated influenza viruses, are unable to prime for influenza-immune Tc cell responses. Moreover, gamma-ray inactivation has less impact on hemagglutination activity than formalin or UV inactivation. γ-irradiated virus, retaining antigens similar to their native forms does appear to at least partially account for its superior immunogenicity. Intranasal administration targets the lung mucosa associated lymphoid organ for inducing immunity in the respiratory tract. However, a previously marketed intranasally administered influenza vaccine, was associated with an increase in the number of Bell's palsy cases - facial paralysis (see Mutsch, et al., (2004), "Use of the inactivated intranasal influenza vaccine and the risk of Bell's palsy in Switzerland", The New England journal of medicine, 350: 896-903) and consequently resulted in market withdrawal of this vaccine preparation. This adverse event has been attributed to the mucosal adjuvant used; *Escherichia coli* heat-labile toxin. Such safety concern would not be an issue with γ-ray inactivated influenza vaccine as it does not require the inclusion of potentially harmful adjuvants in its vaccine formulation.

Apart from the strong protective efficacy observed, several additional factors contribute to the attractiveness of γ-irradiation for influenza vaccines. Firstly, freeze dried γ-ray inactivated A/PC maintained its cross-protective property. Dry powder formulations will improve stability compared to liquid formulations under various storage conditions providing a significant advantage in distribution of the vaccine in an event of a pandemic. Secondly, the intranasal route of delivery, which requires little training or medical qualified personnel, would provide additional advantages for developing countries. Thirdly, γ-ray inactivated influenza vaccine would be comparatively easy and inexpensive to manufacture when compared to other vaccine production processes. Most importantly with regards to manufacturing considerations and availability, the robust heterosubtypic protection induced by γ-ray inactivated influenza may render annual reformulation of influenza vaccines obsolete.

### Example 6: Preparation of influenza virus for gamma-irradiation

Viruses used in current influenza vaccines are generally purified before attenuation using ultracentrifugation which has been associated with loss of viral-antigen and/or destruction of virion structure. The induction of cytotoxic T cell responses by γ-irradiated influenza vaccines will benefit from an alternative method of virus purification (differential/tangential filtration) prior to γ-irradiation which preserves the integrity of virion structure.

It is envisaged that virus stocks will be clarified using centrifugation at low speed (~3000rpm) and used in size exclusion based centrifugation. Clarified stocks will be spun through filtering device with pore size 50-80nm. In general, the size of influenza virus will be 80-120nm. Thus, variable pore size (e.g less than 80nm) will be used to purify influenza virus at low centrifugation speeds (4000 -10000 rpm) (variable speed can be used) at 4°C for as long as needed to get liquid through the filter. The initial virus stock liquid flow path on the upstream side of the filter will be tangential or across the filter surface. Upon centrifugation, the majority of the liquid will pass through the filter (permeate), while a small portion will be retained in the central reservoir as the retentate (containing all the virus).

The retentate will be rediluted with PBS (normal saline, or any other media) that may contain sugar (dextran, sucrose) to maintain the osmotic pressure and consequently virus integrity. These diluted preparations may be filtered again, if needed. Concentrated virus from the final centrifugation step will be treated by γ-irradiation as described in the Examples above. Free radical scavengers, such as Ascorbate, can be added to purified virus stocks prior to irradiation to reduce possible damage to viral proteins while inactivating viral genome during γ-irradiation.

For example, the following protocol may be utilised for the purification of intact influenza virus to be used for γ-irradiation:

1. influenza virus stock can be harvested from embryonated eggs, or *in vitro* tissue culture.
2. using filter devices with a cut off of 300 Kd, virus stock can be clarified by centrifuging at 300g for 30 minutes at 4°C causing both influenza viruses and proteins of the allentoic fluids (or tissue culture media) to pass through the filter.
3. using filter devices with a cut off of 100 Kd, clarified virus stock can be purified by centrifuging at 300g for 30 minutes at 4°C. In this step influenza viruses do not pass through the filter (thereby concentrating the virus) on one side of the filter.
4. concentrated virus can be washed with normal saline (or any buffered media) to remove any remaining egg proteins (washing may be performed as many times as required). Washing can be performed by diluting the concentrated viruses with saline and centrifuging as described in step 3 above.
5. the final virus concentrate will contain intact virion.

In general, pore size cut off levels for filtering devices used in the above technique can be designed to match virion size of 80-120 nm. All procedures may be conducted at 4°C and no ultracentrifugation is required. Viral infectivity can be tested for original stock and final products. Prior knowledge of virus titres and volume can facilitate estimation of level of concentration. The purity of the final product can be determined using standard biochemical analyses.

**Example 7: Gamma-irradiated influenza vaccine enhances immune responses induced by co-administered immunogen**

(i) Materials and Methods

*Cells*

Vero cells were maintained *in vitro* using DMEM media with HEPES supplemented with 1% L-Glutamine, 1% Steptomysin/Pentamysin and 5% Foetal calf serum (FCS). Cells were grown to approximately 90% confluence and were split accordingly twice a week using trypsin: 1/10 split on week days and a 1/20 split prior to weekends. All cell culture work required sterility and was done inside a laminar flow hood. Cell stocks were frozen in freezing mix (50% FCS + 40% media + 10% dimethyl sulfoxide) in cryovials and stored in liquid Nitrogen. Cells were recovered by addition and mixing of 37°C pre-warmed media followed by washing (centrifuging cells at 1500 rpm, 5 minutes at 25°C and then resuspended in media).

*Viral Stock*

aSFV: Avirulent SFV (A7 strain) was grown *in vitro* by infecting vero cells at a multiplicity of infection of 1 PFU: 1 cell, and infected flasks were incubated for 24h at 37°C 5% $CO_2$. Culture supernatants were then collected into a 50ml tube and clarified to remove cellular debris by centrifugation at 1500rpm for 5 mins. The supernatant was then collected and aliquoted into eppendorf tubes and placed in -80°C freezer.

*Influenza*

Influenza A virus stocks were prepared as described in the Examples above. The mouse adapted influenza A virus strain A/PR8 (A/Puerto Rico/8/34 [H1N1]) was propagated in the allantonic cavity of 10 day old embryonated hen eggs (inoculated with $1\times10^4$ PFU/egg) at 37°C for 48 hours. Infected eggs were chilled overnight at 4°C and the allantonic fluid was harvested and clarified by centrifugation at 1500rpm for 5 mins at 4°C. Clarified virus stocks were aliquoted and stored in -80°C freezer.

*Virus titration using Plaque Assay*

Vero cells at approximately 90% confluent growth were trypsinised as previously described to obtain single cell suspensions and the final cell concentration was determined using a hameocytometer. Cells were then resuspended at a concentration of $1.5 \times 10^5$ cells/ml and 6-well tissue culture plates were seeded with 3mls/ well to give a final concentration of $4.5 \times 10^5$ cells/well. Plates were then incubated overnight at 37°C 5% $CO_2$. The following day, duplicate of Vero cells monolayers were infected with 10-fold serial dilutions of virus stock in DMEM culture media containing 5 % FCS and antibiotics. After 1 hour of adsorption in 37°C, 5% $CO_2$ incubator, 3mls of an agar overlay containing 50% of 1.8% Bacto-Agar, 40% DMEM media, 10% FCS and 0.002 % Fungizone was added to each well and plates were incubated for 3 days at 37°C, 5% $CO_2$. Following 3 days incubation, cells were fixed with 5% Formalin (1ml/well) for 1 hour at room temperature. The overlay was then carefully removed and 400µl of 0.2% Crystal violet was added to each well. After 20 mins, the plates were washed 3 times with water and left to air dry overnight. Plaques were enumerated using a microscope and virus titre was calculated in plaque forming units per ml (PFU/ml). SFV titre was ~$3 \times 10^8$ PFU/ml as determined by plaque assay on vero cells. Similar approach was taken to determine the titre for A/PR8 virus using MDCK cells. A/PR8 titre was $5 \times 10^7$ PFU/ml.

*Gamma-irradiated vaccines*

*γ-FLU vaccine:* Virus stock was purified by cRBC agglutination of virus HA by incubation on ice for 45 minutes, and was centrifuged (4°C, 1500 rpm, 10 minutes) to obtain cRBC and virus pellet, which was resuspended in normal saline. The cRBC and virus was incubated for 1 hour in a 37°C water bath. The eluted virus was clarified by spinning and the supernatant containing virus was collected, titrated ($1 \times 10^8$ PFU/ml), and stored at -80°C in 10mL yellow capped tubes. Concentrated virus stocks were inactivated by exposure to 25kGy of gamma-irradiation from Colbalt - 60 Source at the Australian Nuclear Science and Technology Organisation (ANSTO) at Lucas Hights/NSW. Inactivated stocks were passaged 5 times in 10-days embryonated eggs.

*γ-SFV vaccine:* the γ-SFV vaccine was previously prepared as follows. Using Millipore filtering devices with 100kDa cut-off (Millipore), SFV stock was concentrated by centrifuging at 2000 rpm for 1 hour at 4°C using eppendorf bench top centrifuge. Flow through liquid was discarded, and virus particles containing liquid retained within the collection reservoir was collected and stored at -80 °C. The concentrated SFV stock with a titre of $5 \times 10^8$ PFU/ml was inactivated by exposure to 50 kGy gamma-irradiation from Colbalt - 60 Source and later tested for inactivation using plaque assay. Inactivated stocks were passaged 5 times using Vero cells.

*Viral antigen concentration for ELISA:* live A/PR8 virus ($5 \times 10^7$ pfu/ml) or SFV ($3 \times 10^8$ pfu/ml) was concentrated 10x using Millipore filtering devices with 100kDa cut-off (Milipore) and centrifuged at 2500 rpm using eppendorf bench top centrifuge. Concentrated virus was diluted 1:10 with PBS and reconstituted again by centrifugation. This step was repeated twice prior to resuspending the10x concentrated materials in borate buffer. Concentrated antigen was then aliquoted into eppendorf tubes and stored in the -80°C freezer.

*Mice and animal work:* 7-8 week old female wild-type C57B/6 mice (B6 mice) were purchased from the University of Adelaide and housed in the infectious suites at the medical south animal house. Mice were allowed 2 weeks to adapt to the new environment before experimental procedures commenced. All experiments were approved by the University of Adelaide's Animal Ethics Committee and in accordance with the institutional animal care and regulations.

For i.v injection, Mice were warmed using a heating lamp to increase the blood flow to the tail. Individual mice were kept in a holding container to minimise their movements. Each mouse was injected in the tail vein with 200µL of the relevant virus or vaccine preparation and mice were monitored briefly. In general, 10 fold serial dilutions were used to dilute vaccines (y-FLU and γ-SFV) direct from stock (direct) prior to vaccination. 10 fold serial dilutions referred to as $10^{-1}$, $10^{-2}$ and $10^{-3}$. **Table 9** shows vaccination doses as PFU-equivalent. Note, for simplicity, abbreviations will be used to denote particular doses of the relative vaccine preparations throughout the study.

*Table 9: Vaccination doses as PFU-equivalent/mouse.*

| Vaccination group | Dose of vaccination and abbreviations |
|---|---|
| γ-SFV ($10^8$) | $1 \times 10^8$ PFU-equivalent |
| γ-SFV ($10^7$) | $1 \times 10^7$ PFU-equivalent |
| γ-SFV ($10^6$) | $1 \times 10^6$ PFU-equivalent |
| γ-Flu ($10^5$) | $2 \times 10^5$ PFU-equivalent |
| γ-Flu ($10^4$) | $2 \times 10^4$ PFU-equivalent |
| SFV ($10^7$) | $6 \times 10^7$ PFU-equivalent |
| FLU ($10^7$) | $1 \times 10^7$ PFU-equivalent |

*Blood collection and serum samples*

For kinetic analysis of antibody responses, blood was collected via tail bleeds at 3,6 and 12 days post vaccination or infection. Blood samples for day 20 post vaccination or infection was collected directly from the heart cavity during autopsy. Blood samples were stored overnight at 4°C to allow clot formation and serum samples were obtained by centrifugation at 3000 rpm for 5 minutes. Serum samples were stored in the -80°C freezer.

*Testing lymphocyte activation Flow using FACS*

Mice were injected i.v. with the appropriate live or inactivated viral preparations and 24 hours post injection, spleens were harvested and maintained in 5ml of DMEM + 1%FCS media on ice. Spleens were then meshed in single cell strainers to obtain single cell suspensions in PBS containing 1% FCS and subsequently washed by centrifugation at 300g for 5 minutes. Samples were depleted of red blood cells by the addition of 5mls of RBC lysis buffer ($NH_4Cl$) and left to incubate for 5-7 minutes at 37°C, 5% CO2. 5mls of PBS+1%FCS was then added, cells were centrifuged at 300g for 5 minutes and supernatant was removed. Cells were washed again twice using 5mls of PBS+1%FCS and then resuspended to a final concentration of $1x10^7$ cells/ml in PBS 1%FCS. 100$\mu$l of cell suspensions at the required concentration of $1x10^6$ cells/well were added to the appropriate wells in a 96-well round bottom plate along with 100$\mu$l of PBS +1%FCS. Cells were washed by centrifugation at 400g for 5 minutes and the supernatant was discarded. 5$\mu$l of 1:10 dilution of FcR blocker (Biolegend) was added to each of the wells and left at room temperature for 15 minutes. Then, the appropriate antibody staining mixture (15$\mu$l/well) was added. Staining mixtures were prepared by mixing equivalent amount of 1:20 diluted anti CD3-APC conjugated, and anti B220-FITC conjugated, and either anti CD69-PE conjugated, or anti CD25-PE conjugated, or anti CD86-PE conjugated (Biolegend). Plates were then wrapped in aluminium foil and incubated for 30 mins in the dark. Single stains for each of the flurophores were used as the controls. 150ul of PBS +1%FCS was added to each of the wells and cells were washed twice by centrifugation at 400g for 5 min. The cells were then fixed by the addition of 200ul of 4% paraformaldehyde and the plate was then wrapped in aluminium foil and left at 4°C. Stained samples were analysed within 48h of staining using FACSCalibur (BD Bioscience) and the data was interpreted using FlowJo software (Tree Star). APC-CD3+ and FITC-B220+ antibody staining was used to gate on T and B lymphocytes respectively.

*Plaque Reduction Assay to test neutralization of SFV*

Vero cells were trypsinised as previously described and single cell suspension at a concentration of $1.5x10^5$ cells/ml was used to seed 24-well tissue culture plates using 1ml/well ($1.5x10^5$ cells/well) and plates were incubated overnight at 37°C, 5% $CO_2$. On the next day, aliquots of the serum samples from control and vaccinated animals were incubated at 56°C for 30 mins to inactivate complements. Inactivated serum samples were serially diluted using DMEM media without FCS. Then, 100ul of the diluted samples were mixed with equivalent amount of DMEM media containing 100 PFU of SFV. Mixtures (sera and virus) were incubated for 1.5 hours at 37°C, and then used to infect 4 wells (50ul/well) of the confluent vero cell monolayers prepared earlier. Initially, culturing media was removed from each well prior to addition of 50$\mu$l from virus/serum mixture. Then, plates were incubated for 2 hours at 37°C to allow infection of monolayers. Following incubation, the infecting mixture was removed and cells were wash twice with DMEM+5% FCS. Then 1ml of an agar overlay containing 50% of 1.8% Bacto-Agar, 40% DMEM media, 10% FCS and 0.002 % Fungizone was added to each well and plates were incubated for 3 days at 37°C, 5% CO2. Following 3 days incubation, cells were fixed with 5% Formalin (500$\mu$l well) for 1 hour at room temperature. The overlay was then carefully removed and 200$\mu$l of 0.2% Crystal violet was added to each well. After 20 mins, cells were washed 3 times with water and left to dry. Plaques were enumerated to determine the effect of the serum on virus infectivity.

*HA U inhibition assay to test neutralization of influenza virus*

Aliquots of sera were incubated at 56°C to inactivate complements for 30 mins, then diluted in PBS containing 1% red blood cells (RBCs) and left for another 30 min incubation at room temperature. RBC's within samples were then pelleted for approximately 5 secs at 1400rpm using a microcentrifuge and 2 fold serial dilutions were performed with aliquots of the supernatant using a 96 well plate. 50ul of diluted virus (FLU) at a concentration of 80 HAU/ml was then added to each dilution of sera and left to incubate for 30 mins at room temperature. PBS containing 1% RBC's was then added to each well and was left to incubate at 4°C. Results were analysed 24 hours later and neutralizing antibodies in each dilution was determined by the presence of a pellet of RBCs at the bottom of the wells.

*Direct ELISA to test serum antibody responses*

A direct ELISA was used to measure SFV-specific and FLU-specific antibody responses in serum samples. In brief, maxisorp plates were coated with SFV or FLU viral antigen diluted in bicarbonate coating buffer ($Na_2CO_3$, $NaHCO_3$, water at pH 9.6) and left to incubate overnight at room temperature. Then, coated plates were washed 4 times with washing buffer (PBS+0.05%Tween) and 200$\mu$l of the blocking buffer (PBS+2% skim milk powder) was added to each wells and incubated at room temperature for 2 hours. After incubation, plates were washed 4 times with washing buffer and 50$\mu$l of diluted (1: 200 dilution unless specified) serum samples were added to the appropriate wells and plates were incubated for 2 hours at room temperature. Then, plates were washed 4 times with washing buffer and 50$\mu$l of horse radish peroxidase conjugated secondary antibodies (i.e. IgG (Thermo Scientific), IgG1(Zymed), IgG2c (Southern Biotech), Total Ig (Millipore)) diluted in blocking buffer were added and plates were incubated at room temperature for 2 hours. After incubation, plates were washed 4 times and 75$\mu$l of TMB peroxidase substrate was added and left to develop in the dark for 30 mins. The reaction was then stopped with 50$\mu$l of 2 mol $H_2SO_4$ and absorbance was measured at 450nm using a Microplate ELISA reader (Bio-Tek Instruments).

*Sandwich ELISA to test Serum IFN-$\alpha$ levels*

A sandwich ELISA was used to measure levels of IFN-$\alpha$ present in serum samples. Maxisorp plates were coated with rat anti-mouse IFN-$\alpha$ diluted in coating buffer ($Na_2PO_4$) and left at room temperature overnight. Plates were then washed 4 times with washing buffer (PBS+ 0.05% Tween-20) and 50ul of diluted recombinant mouse IFN-$\alpha$ (to provide the standard curve) or diluted serum samples were added. The plates were incubated for 2 hours at room temperature. After that, plates were washed 4 times and 50$\mu$l of polyclonal rabbit anti-mouse IFN-$\alpha$ was added, and plates were incubated at room temperature for 2 hours. Plates were later washed 4 times and 50ul of HRP conjugated goat anti-rabbit IgG was added and plates were incubated for 2 hours at room temperature. After incubation, plates were washed 4 times and 75$\mu$l of TMB peroxidase substrate was added and left to develop in the dark for 30 mins. The reaction was then stopped with 50$\mu$l of 2 mol $H_2SO_4$ and absorbance was measured at 450nm using a Microplate ELISA reader (Bio-Tek Instruments).

*Statistical analysis*

Graph Prism software version 5.0 was used for statistical analyses. Results were expressed as mean $\pm$ SEM. Statistical significance among samples was calculated using an unpaired Student t test. P values <0.05 were considered statistically significant.

(ii) Results

*IFN-I mediated partial lymphocyte activation*

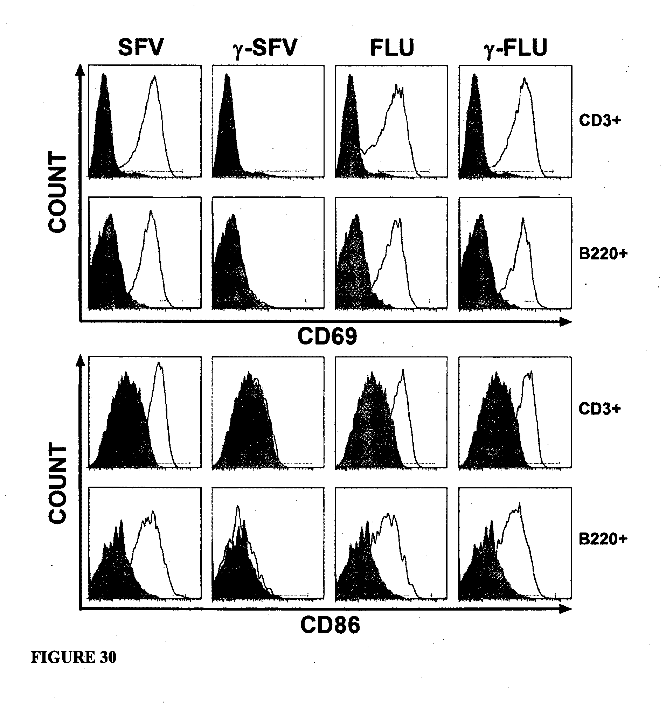

Intravenous administration of live FLU or SFV results in IFN-I mediated systemic, partial lymphocyte activation characterised by upregulated CD69 and CD86 expression within the first 24 hours post injection. However, i.v. administration of gamma irradiated viruses results in alternative outcomes, as $\gamma$-SFV is unable to induce IFN-I mediated partial lymphocyte activation in contrast to $\gamma$-FLU. To confirm these differences, WT B6 mice were injected i.v with either live viruses SFV($10^7$) or FLU($10^7$) or their gamma irradiated forms $\gamma$-SFV($10^8$) or $\gamma$-FLU($10^7$). 24 hours post injection, splenocytes were analysed for cell surface expression of CD69 and CD86 using FACS. Anti-CD3 and B220 cells were used to gate on T and B lymphocytes respectively. Splenocytes from naive mice were used as the negative control. Mice injected with SFV, FLU and $\gamma$-FLU showed upregulated expression of CD69 and CD86 on both B and T lymphocytes **(Figure 30)**. In contrast, CD69 and CD86 expression levels were not upregulated on splenocytes harvested from mice vaccinated with $\gamma$-SFV. This indicates that gamma-irradiated viruses differ in their ability to induce partial lymphocyte activation

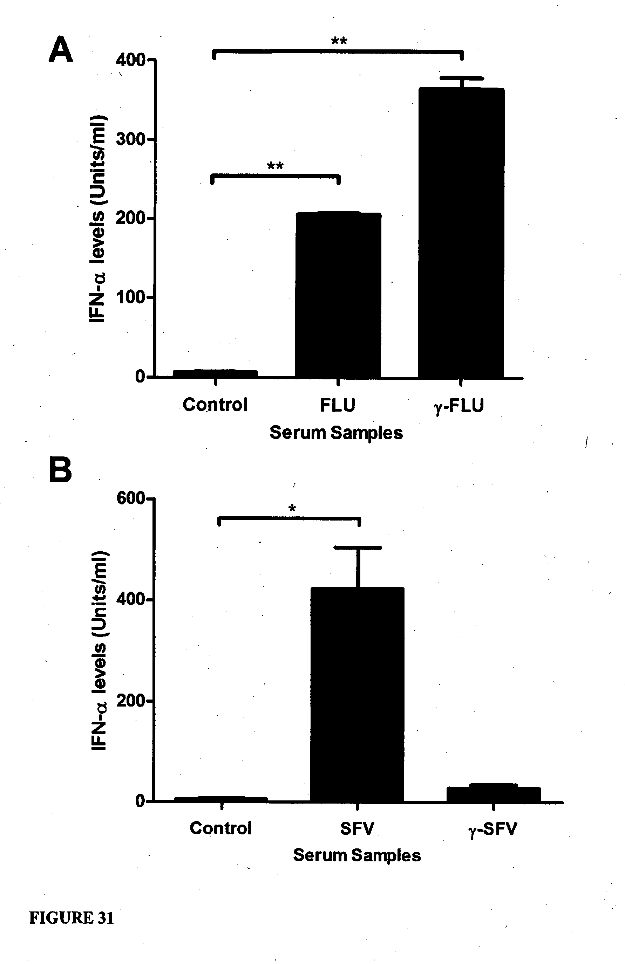

Partial lymphocyte activation is be mediated by IFN-I. Therefore in addition to the analysis of lymphocyte activation in **Figure 30,** serum IFN-$\alpha$ levels were measured by sandwich ELISA in mice were injected i.v. with SFV($10^7$), FLU($10^7$), $\gamma$-SFV($10^8$) or $\gamma$-FLU($10^7$) 24 hours post injection. The results illustrated that SFV, FLU and $\gamma$-FLU strongly induced high levels of IFN-$\alpha$ relative to the negative control (p<0.05), with $\gamma$-FLU inducing higher levels in comparison to that induced by its live form **(Figures 31A and 31B).** $\gamma$-SFV, in contrast to live SFV, did not induce detectable serum IFN-$\alpha$ levels relative to the negative control (sera from naive mice). This shows that live and gamma irradiated viruses differ in their ability to induce IFN-$\alpha$.

*Vaccine efficacy of γ-SFV*

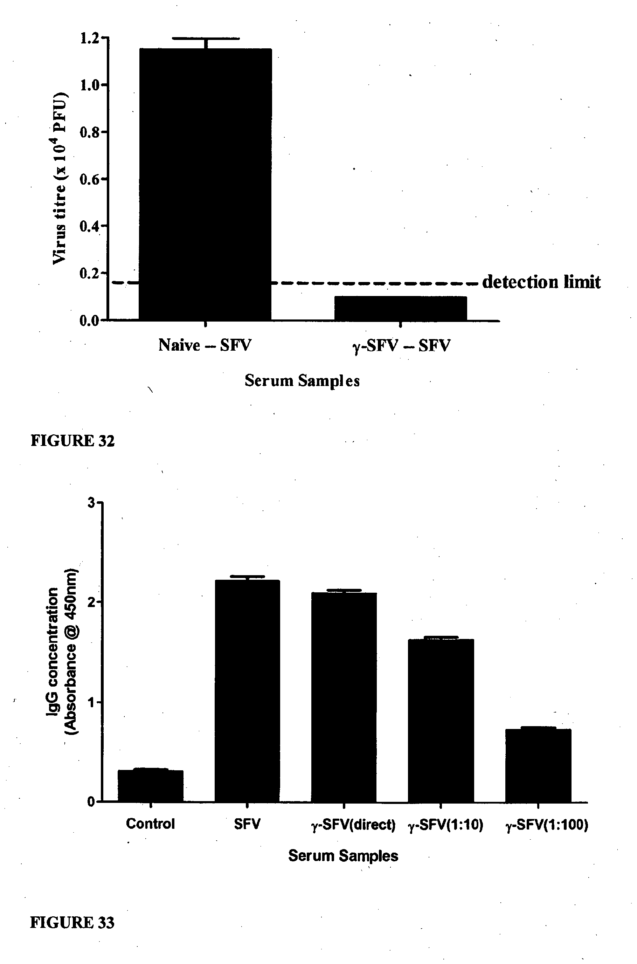

Considering the differences in IFN-$\alpha$ levels induced by γ-SFV, the efficacy of the γ-SFV vaccine was investigated by analysing the effect of prior vaccination on secondary challenge with SFV. Mice were vaccinated with γ-SFV($10^7$) and 3 weeks later, were challenged with SFV($10^8$). Naive animals infected with SFV were used as the positive control. 24 hours post challenge, serum samples were collected and analysed for virus titres by plaque assay. Naive mice infected with SFV served as the positive control. Serum samples from control mice showed approximately $1.2 \times 10^4$ PFU/ml at day 1 p.i **(Figure 32)**. In contrast, no viral infectivity was detected in all serum samples from previously vaccinated mice at day 1 post challenge with SFV. Hence, prior vaccination with γ-SFV was shown to prevent viremia upon secondary challenge with SFV.

*Antibody responses to SFV and γ-SFV*

To confirm the ability of γ-SFV to induce effective antibody responses, mice were infected with SFV or vaccinated with variable doses of γ-SFV ($10^6$, $10^7$ or $10^8$). 20 days post injection, total SFV-specific IgG levels in the serum were determined by ELISA. Antibody levels in sera from naive mice and SFV infected mice were used as the negative and positive control respectively. Mice vaccinated with γ-SFV($10^8$) showed high serum levels of anti-SFV IgG levels relative to the negative control **(Figure 33)**. The serum anti-SFV IgG level showed a dose-dependent correlation with the dose of γ-SFV used for vaccination, with higher doses of γ-SFV inducing a higher level of anti-SFV IgG in the sera ($10^7$, $10^6$) ($p<0.001$). SFV-specific IgG concentrations in the serum of γ-SFV vaccinated mice decreased in a dose dependent manner.

*Effect of co-administration of γ-FLU and γ-SFV on anti-SFV-specific antibody responses*

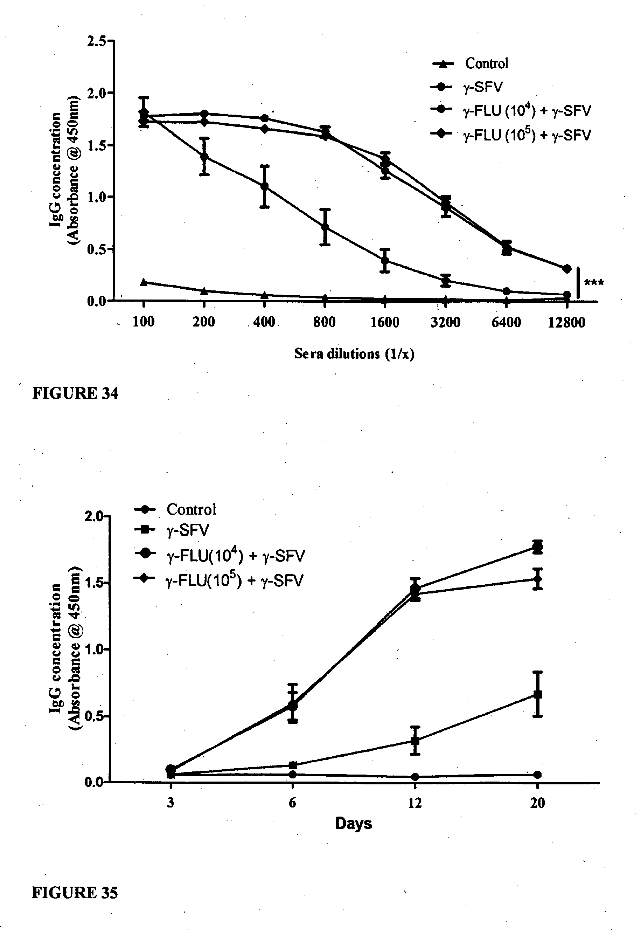

To investigate the potential adjuvant activity of γ-FLU and the associated IFN-I, on the antibody responses against γ-SFV, mice were co-injected with γ-SFV($10^7$) in combination with various doses of γ-FLU($10^4$, $10^5$) and serum SFV-specific IgG levels were measured at day 20 post vaccination at 2 fold serial dilutions of sera by a direct ELISA. Sera from naive mice served as the negative control. SFV-specific IgG levels were significantly enhanced in the serum from mice co-injected with γ-SFV and γ-FLU compared to the level induced by γ-SFV alone ($p<0.05$) **(Figure 34)**. The significant differences between groups were evident at sera dilutions beyond 1 in 200. Hence, it is concluded that co-administration of γ-FLU and γ-SFV enhances anti-SFV-specific IgG levels.

IgG levels in the serum from mice vaccinated with γ-SFV alone decreased to basal level at a sera dilution of 1 in 12,800, whereas SFV-specific IgG levels in serum samples from mice co-injected with γ-SFV and γ-FLU($10^4$ or $10^5$) remained significantly higher at this dilution ($p <0.05$).

To investigate the kinetics of SFV-specific antibody responses post co-administration with various doses of γ-FLU, mice were injected i.v. with γ-SFV($10^7$) alone or co-injected with various doses of γ-FLU ($10^4$ or $10^5$) and serum SFV-specific IgG levels were tested at days 3, 6, 12 and 20 post vaccination by direct ELISA, using sera dilutions of 1 in 800. SFV-specific IgG levels gradually increased throughout the course of the experiment for all groups **(Figure 35)**. Sera from naive mice served as the negative control. Importantly, serum from mice co-injected with γ-SFV and the various doses of γ-FLU showed significant increases in IgG levels relative to mice injected with γ-SFV alone at day 6 ($p <0.05$). Co-administration of γ-FLU and γ-SFV was this shown to enhance the kinetics and magnitude of SFV-specific IgG responses.

*The effect of γ-FLU on SFV neutralizing antibody responses induced by γ-SFV*

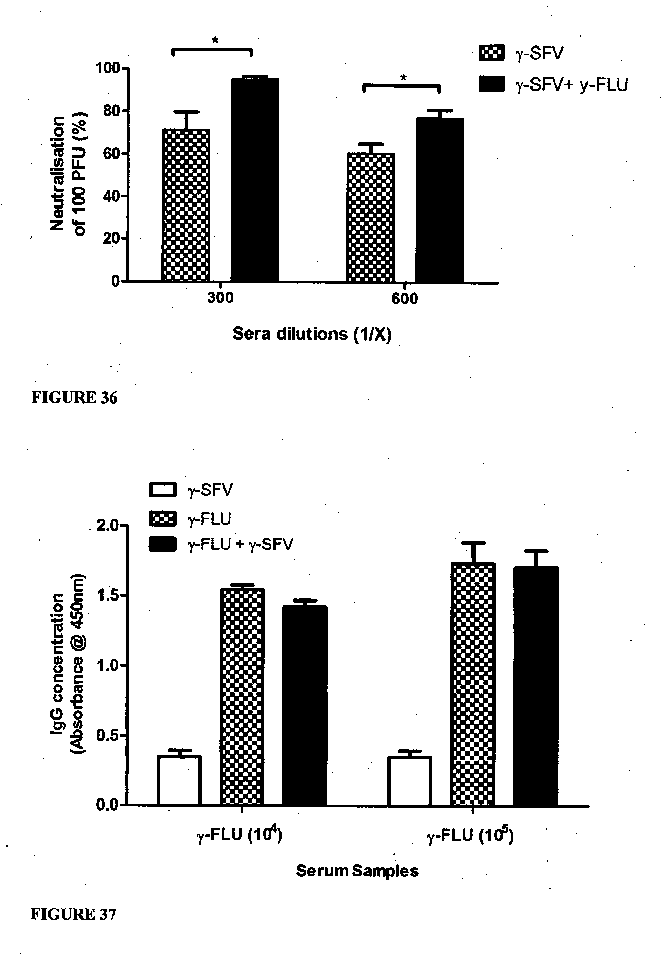

To determine whether enhancement of SFV-specific antibody responses mediated by co-administration of γ-FLU coincides with enhanced SFV neutralisation, serum samples were collected at day 20 post vaccination with γ-SFV ($10^7$) alone or co-injection with γ-FLU ($10^5$). Serum dilutions of 1 in 300 and 1 in 600 were used to test antibody efficiency in neutralizing 100 PFU of SFV using a plaque reduction assay where neutralisation of 100 PFU was measured as a (%). Approximately 70% of the virus concentration (100 PFU) was neutralised at a serum dilution of 1 in 300 from mice vaccinated with γ-SFV alone, and this level of neutralisation reduced to approximately 60% at a serum dilution of 1 in 600 **(Figure 36)**. In contrast, serum samples from mice co-administered with γ-SFV and γ-FLU show significantly higher neutralisation activity, with a 1 in 300 serum dilution showing approximately 95% neutralisation and 1 in 600 serum dilution showing approximately 77% neutralisation of 100 PFU of SFV ($p<0.05$). Thus, sera from mice vaccinated with both γ-FLU and γ-SFV show enhanced SFV neutralisation.

*Effect of co-administration of γ-FLU and γ-SFV on FLU-specific antibody responses*

γ-FLU has elicits neutralising antibodies in response to homotypic challenges. To determine whether the co-administration of γ-FLU and γ-SFV affected the hosts ability to generate effective FLU-specific humoral responses, FLU-specific IgG concentrations at day 20 post vaccination in serum samples from mice vaccinated i.v. with varying doses of γ-FLU ($10^4$ or $10^5$) or co-injected with a γ-SFV($10^7$) were measured by ELISA. Sera from γ-SFV injected mice served as the negative control. The data indicates that co-administration of γ-FLU with γ-SFV did not suppress the induction of FLU-specific IgG titres/responses **(Figure 37)**.

To determine whether the co-administration of γ-FLU and γ-SFV affected the ability of FLU-specific antibodies to neutralise FLU virions, serum samples were tested for the ability to neutralize 80 HAU/ml of FLU using an HA inhibition assay. Mice were injected i.v. with various doses of γ-FLU ($10^4$, $10^5$) or co-injected with γ-SFV($10^7$) and FLU-specific IgG concentrations in the serum were analysed at day 20 post injection by a direct ELISA. Sera from γ-SFV injected mice served as the negative control.

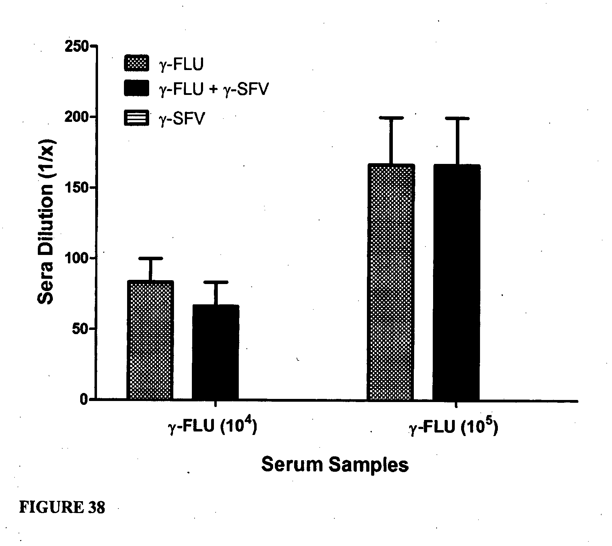

The results show that co-administration of γ-FLU and γ-SFV did not impact the neutralisation activity of FLU-specific antibody responses **(Figure 38)** as the virus concentration (80 HAU/ml) was neutralised at similar dilutions of sera compared to the to the corresponding γ-FLU doses alone. Put another way, co-administration of γ-FLU and γ-SFV did not impact FLU-specific IgG titres.

(iii) Discussion

*Differences in the induction of IFN-I among gamma irradiated viruses*

The major focus of the study was to exploit the IFN-I responses by γ-FLU and analyse how this could benefit immunity towards additional vaccines. The first aim of the study was to confirm the differences between γ-FLU and γ-SFV in terms of their ability to induce IFN-I responses and the associated partial lymphocyte activation. This was very important in defining the advantage of using γ-SFV as the experimental vaccine model. SFV and Influenza are both single-stranded RNA viruses which are capable of inducing strong IFN-I responses and partial lymphocyte activation within the first 24 hours of infection, as confirmed by upregulation of CD69 and CD86 on lymphocytes **(Figure 30)**. The potent induction of IFN-α induced by SFV as opposed to live infection with FLU has could be explained by the nature of their infection strategies. Importantly, this study has identified some interesting differences among the live and gamma-irradiated forms of these viruses. γ-SFV is unable to induce IFN-α and fails to induce partial lymphocyte activation as observed by the absence of CD69 and CD86 upregulation **(Figures 30 and 31)**. This contrasts to γ-FLU which elicits a strong IFN-α response and induces partial systemic lymphocyte activation analogous to its live form.

*Antibody responses induced by γ-SFV*

Given the lack of primary T cell responses induced during the course of live SFV infection in B6 mice, and therefore the dependence on antibody responses to prevent viral dissemination throughout the host, the quality of antibody responses generated by γ-SFV were investigated to establish the vaccine experimental model. Initially it was confirmed that the absence of systemic IFN-I responses shown in **Figures 30 and 31** does not hinder the ability of the vaccine to mount effective antibody responses. Plaque assay testing viremia at day 1 post SFV infection confirmed that prior vaccination with γ-SFV induced effective and protective antibody responses illustrated by the lack of detectable infectious particles in the serum upon challenge with live SFV **(Figure 32)**. The high IgG levels in the serum of γ-SFV vaccinated animals **(Figure 33)** further highlights the efficacy of pre-existing antibody induced by vaccination, in neutralising the virus from the blood circulation upon secondary challenge. In addition, the level of SFV-specific antibody responses induced by γ-SFV is dose dependent. Therefore, γ-SFV($10^7$) was selected as the appropriate dose for co-administration with γ-FLU as it was still able to induce significantly higher antibody responses in comparison to the minimal levels induced by a γ-SFV($10^6$), but significantly lower levels than those induced by γ-SFV($10^8$). This provided flexibility to test suppression or enhancement of antibody responses upon co-administration with γ-FLU.

*The effect of γ-FLU and γ-SFV co-administration on SFV-specific antibody responses*

γ-FLU vaccines generate cross protective CD8+ T cell responses upon homologous and heterosubtypic influenza virus challenges, as well as eliciting protective antibody responses. Therefore, γ-FLU is considered as a potential universal influenza vaccine. Based on the potent IFN-I mediated immunostimulatory effects of γ-FLU, it was hypothesized that the IFN-I-mediated activity induced by γ-FLU may serve as an efficient adjuvant to enhance antibody responses towards a less immunogenic antigen such as γ-SFV.

One of the major reasons to incorporate adjuvants into vaccine preparations is to amplify the immunological responses generated towards the antigen of interest. Thus, to determine whether γ-FLU can be used as a potential adjuvant within vaccine preparations, serial doses of γ-FLU were combined with γ-SFV($10^7$) and mean antibody titres were measured to quantify any differences among the experimental groups. The data revealed that co-administration of γ-SFV with γ-FLU($10^4$ or $10^5$) were effective in enhancing SFV-specific antibody responses in comparison to mice injected with γ-SFV alone **(Figure 34).** The observed trend was also consistent over a time course following co-administration of γ-FLU and γ-SFV **(Figure 35).** Adjuvants are often used to achieve qualitative alterations within immunity such as increasing the speed of an immunological response and this is important especially at crucial times where a pandemic outbreak of infection could occur. Interestingly, the enhanced titres observed at day 6 post vaccination with co-injection of γ-FLU and γ-SFV were equal to the titres following vaccination at day 20 with γ-SFV alone, therefore demonstrating that co- administration of γ-SFV and γ-FLU caused an earlier induction and an amplification of SFV humoral responses.

It is essential within any vaccine preparations that the immunity it elicits within the host is effective and adequate at providing protection. Based on the significant enhancement in SFV-specific IgG levels shown in **Figures 34 and 35,** antibody responses in the sera from mice co-injected with γ-FLU($10^5$) and γ-SFV were analysed for their neutralisation efficiency. The antibody responses in these mice were shown to be more proficient at neutralising SFV virions than injection of γ-SFV alone **(Figure 36).** This effect is most likely due to an increase in antibody yield versus antibody affinity. Therefore, this confirmed that γ-FLU enhanced the quality and quantity of SFV-specific antibody responses.

*The effect of γ-FLU and γ-SFV co-administration on FLU-specific antibody responses*

It is important that using γ-FLU to enhance the immune response towards another immunogen in a co-administered vaccine does not compromise the immune response to γ-FLU. Therefore, humoral responses generated against FLU antigens were analysed to certify that immunological responses were not hindered when a second vaccine is present in the environment. The results show that co-administration of γ-FLU and γ-SFV did not affect the titres of FLU-specific IgG **(Figure 37)** in comparison to their corresponding individual doses of γ-FLU. In addition, co-administration of γ-FLU and γ-SFV did not affect the neutralising activity of FLU-specific antibodies **(Figure 38),** verifying that co-administration of γ-FLU and γ-SFV did not compromise the vaccine efficacy of γ-FLU.

(iv) Conclusion

These findings show that γ-FLU can be employed to increase the quality and magnitude of immune responses towards an alternative less immunogenic vaccine. The results demonstrate that co-administration of γ-FLU and γ-SFV significantly enhanced protective, SFV-specific antibody responses towards a less immunogenic dose of γ-SFV, and resulted in an overall increase in the speed of the SFV-specific humoral response. Co-administration of γ-FLU and γ-SFV also did not affect the hosts' ability to generate appropriate humoral responses against the influenza virus. These findings provide evidence that γ-FLU, a safe and inexpensive vaccine, can be utilised to enhance immunity towards a co-administered vaccine that is otherwise less immunogenic in addition to providing protective immune responses against the influenza virus. Accordingly, co-administration of γ-FLU with vaccines targeting secondary infections that commonly arise during or after an episode of influenza may serve to induce protective immune responses against the influenza virus and enhance the immunogenicity of the co-administered vaccine targeting the secondary infection.

**Example 8: Preparation and administration of a combination vaccine containing gamma-irradiated influenza viruses and antigens against a secondary infection associated with flu**

A combination vaccine targeted at the preventing a secondary *Streptococcus pneumoniae* infection associated with flu infection in a mammal (e.g. a human subject) may be prepared according to the following instructions.

*(i) Preparation*

*Gamma-irradiated influenza virus*

Influenza virus stock (e.g. H1N1 APR/8/34) may be inactivated using an appropriate dose of gamma-irradiation (e.g. $5 \times 10^5$ rad (5 KGy) - $1 \times 10^6$ rad (10 KGy) of γ-rays) by exposure to a suitable gamma emitter (e.g. a commercially available device such as a Gammacell irradiator manufactured by Atomic Energy of Canada Ltd., Canada).

*Streptococcus pneumoniae antigens*

A solution of antigens from *Streptococcus pneumoniae* may be prepared by isolating and purifying capsular polysaccharides from various serotypes of *S. pneumoniae* (serotypes 1, 2, 3, 4, 5, 6B, 7F, 8, 9N, 9V, 10A, 11A, 12F, 14, 15B, 17F, 18C, 19F, 19A, 20, 22F, 23F, and 33F).

Alternatively, a PPV23 formulation may be purchased from a commercial source (e.g. Pneumovax® 23).

Alternatively, whole inactivated (killed) *Streptococcus pneumoniae* may be produced by fermentation followed by inactivation using for example, 0.5% to 2% formaldehyde or an appropriate dose of gamma irradiation (e.g. 5x10$^4$ rad (0.5 KGy) - 1x10$^6$ rad (10 KGy) of γ-rays) by exposure to a suitable gamma emitter (e.g. a commercially available device such as a Gammacell irradiator manufactured by Atomic Energy of Canada Ltd., Canada).

*(ii) Dosage and formulations*

Single doses of gamma-irradiated influenza virus preparations comprising 10 x e$^2$ haemagglutinating units/kg body weight (or, for example, 0.5 to 5 mcg of HA equivalent) may be formulated in saline suitable for administration as a droplet, spray, dry powder or via the use of a nebuliser for respiratory delivery.

Single doses of *S. pneumoniae* preparations including purified antigen comprising 25mcg of each antigen may be formulated in 0.9% sodium chloride suitable for administration by intranasal spray. Alternatively, *S. pneumoniae* preparations may be formulated in 0.9% sodium chloride and an acceptable adjuvant for injection or mixed with an appropriate excipient and formulated in an enteric capsule for oral administration.

*(iii) Administration*

Each preparation may be administered to the subject simultaneously or separately. Multiple doses (re-vaccination) of either or both components may be administered over time.

Persons of ordinary skill in the art will recognise that combination vaccines comprising gamma-irradiated influenza viruses and antigens derived from other agents causative of secondary infections that arise during or after flu infection can be prepared and administered using methodology similar to that described above, without requiring inventive effort.

**Claims**

1. Gamma-irradiated influenza virus and an immunogen against an agent causative of an infection for use in preventing or treating the infection in a subject, wherein the infection is a secondary infection following influenza virus infection and said immunogen is:

   (i) a bacterium selected from the group consisting of *Streptococcus pneumoniae, Pseudomonas aeruginosa, Haemophilus influenzae, Staphylococcus aureus, Moraxella catarrhalis,* and *Mycoplasma sp.,* or a component thereof; or
   (ii) a virus selected from the group consisting of a rhinovirus, adenovirus, coxsackievirus, picornavirus, togavirus and coronavirus, or a component thereof.

2. The gamma-irradiated influenza virus and immunogen for use according to claim 1, wherein the immunogen is a gamma-irradiated microorganism.

3. The gamma-irradiated influenza virus and immunogen for use according to claim 1 or claim 2, wherein the gamma-irradiated influenza virus and the immunogen are formulated for administration to the subject simultaneously.

4. The gamma-irradiated influenza virus and immunogen for use according to claim 1 or claim 2, wherein the gamma-irradiated influenza virus and the immunogen are formulated for administration to the subject separately.

5. The gamma-irradiated influenza virus and immunogen for use according to any one of claims 1 to 4, wherein the gamma-irradiated influenza virus and the immunogen are formulated for intranasal administration.

6. A vaccine composition comprising a gamma-irradiated influenza virus and an additional immunogen, wherein the additional immunogen is:

   (ii) a bacterium selected from the group consisting of *Streptococcus pneumoniae, Pseudomonas aeruginosa, Haemophilus influenzae, Staphylococcus aureus, Moraxella catarrhalis, Mycoplasma sp.,* and components thereof; or

(ii) a virus selected from the group consisting of a rhinovirus, adenovirus, coxsackievirus, picornavirus, togavirus, coronavirus, and components thereof.

7. The vaccine composition of claim 6, wherein the additional immunogen is a gamma-irradiated microorganism.

8. The vaccine composition according to claim 6 or claim 7, wherein the gamma-irradiated influenza virus is an influenza A H1N1 subtype virus, or an APR/8/34 virus.

## Patentansprüche

1. Gammabestrahltes Influenzavirus und ein Immunogen gegen ein Mittel, welches eine Infektion verursacht, zur Verwendung bei Prävention oder Behandlung der Infektion bei einem Individuum, wobei die Infektion eine einer Influenzavirusinfektion folgende sekundäre Infektion ist und das Immunogen folgendes ist:

   (i) ein Bakterium, ausgewählt aus der Gruppe bestehend aus *Streptococcus pneumoniae, Pseudomonas aeruginosa, Haemophilus influenzae, Staphylococcus aureus, Moraxella catarrhalis,* and *Mycoplasma sp.*, oder ein Bestandteil davon; oder
   (ii) ein Virus, ausgewählt aus der Gruppe bestehend aus einem Rhinovirus, einem Adenovirus, einem Coxsackievirus, einem Picornavirus, einem Togavirus und einem Coronavirus, oder ein Bestandteil davon.

2. Gammabestrahltes Influenzavirus und Immunogen zur Verwendung nach Anspruch 1, wobei das Immunogen ein gammabestrahlter Mikroorganismus ist.

3. Gammabestrahltes Influenzavirus und Immunogen zur Verwendung nach Anspruch 1 oder Anspruch 2, wobei das gammabestrahlte Influenzavirus und das Immunogen zur gleichzeitigen Verabreichung an das Individuum formuliert sind.

4. Gammabestrahltes Influenzavirus und Immunogen zur Verwendung nach Anspruch 1 oder Anspruch 2, wobei das gammabestrahlte Influenzavirus und das Immunogen zur separaten Verabreichung an das Individuum formuliert sind.

5. Gammabestrahltes Influenzavirus und Immunogen zur Verwendung nach einem der Ansprüche 1 bis 4, wobei das gammabestrahlte Influenzavirus und das Immunogen zur intranasalen Verabreichung formuliert sind.

6. Impfstoffzusammensetzung, umfassend ein gammabestrahltes Influenzavirus und ein zusätzliches Immunogen, wobei das zusätzliche Immunogen folgendes ist:

   (ii) ein Bakterium, ausgewählt aus der Gruppe bestehend aus *Streptococcus pneumoniae, Pseudomonas aeruginosa, Haemophilus influenzae, Staphylococcus aureus, Moraxella catarrhalis, Mycoplasma sp.* und Bestandteilen davon; oder
   (ii) ein Virus, ausgewählt aus der Gruppe bestehend aus einem Rhinovirus, einem Adenovirus, einem Coxsackievirus, einem Picornavirus, einem Togavirus, einem Coronavirus, und Bestandteilen davon.

7. Impfstoffzusammensetzung nach Anspruch 6, wobei das zusätzliche Immunogen ein gammabestrahlter Mikroorganismus ist.

8. Impfstoffzusammensetzung nach Anspruch 6 oder Anspruch 7, wobei das gammabestrahlte Influenzavirus ein Influenza A H1N1 Virussubtyp oder ein APR/8/43 Virus ist.

## Revendications

1. Virus de la grippe irradié aux rayons gamma et un immunogène contre un agent responsable d'une infection pour utilisation dans la prévention ou le traitement de l'infection chez un sujet, dans laquelle l'infection est une infection secondaire suivant une infection par le virus de la grippe et ledit immunogène est :

   (i) une bactérie sélectionnée dans le groupe constitué de *Streptococcus pneumoniae, Pseudomonas aerugi-*

*nosa, Haemophilus influenzae, Staphylococcus aureus, Moraxella catarrhalis* et *Mycoplasma sp.,* ou un composant de celle-ci ; ou

(ii) un virus sélectionné dans le groupe constitué d'un rhinovirus, d'un adénovirus, d'un virus Coxsackie, d'un picornavirus, d'un togavirus et d'un coronavirus, ou un composant de celui-ci.

2. Virus de la grippe irradié aux rayons gamma et immunogène pour utilisation selon la revendication 1, dans laquelle l'immunogène est un microorganisme irradié aux rayons gamma.

3. Virus de la grippe irradié aux rayons gamma et immunogène pour utilisation selon la revendication 1 ou la revendication 2, dans laquelle le virus de la grippe irradié aux rayons gamma et l'immunogène sont formulés pour une administration simultanée au sujet.

4. Virus de la grippe irradié aux rayons gamma et immunogène pour utilisation selon la revendication 1 ou la revendication 2, dans laquelle le virus de la grippe irradié aux rayons gamma et l'immunogène sont formulés pour une administration séparée au sujet.

5. Virus de la grippe irradié aux rayons gamma et immunogène pour utilisation selon l'une quelconque des revendications 1 à 4, dans laquelle le virus de la grippe irradié aux rayons gamma et l'immunogène sont formulés pour une administration intranasale.

6. Composition de vaccin comprenant un virus de la grippe irradié aux rayons gamma et un immunogène supplémentaire, dans laquelle l'immunogène supplémentaire est :

    (i) une bactérie sélectionnée dans le groupe constitué de *Streptococcus pneumoniae, Pseudomonas aeruginosa, Haemophilus influenzae, Staphylococcus aureus, Moraxella catarrhalis* et *Mycoplasma sp.,* ou un composant de celle-ci ; ou
    (ii) un virus sélectionné dans le groupe constitué d'un rhinovirus, d'un adénovirus, d'un virus Coxsackie, d'un picornavirus, d'un togavirus et d'un coronavirus, ou un composant de celui-ci.

7. Composition de vaccin selon la revendication 6, dans laquelle l'immunogène supplémentaire est un microorganisme irradié aux rayons gamma.

8. Composition de vaccin selon la revendication 6 ou la revendication 7, dans laquelle le virus de la grippe irradié aux rayons gamma est un virus de sous-type H1N1 de la grippe A, ou un virus APR/8/34.

FIGURE 1

FIGURE 2

**FIGURE 3**

**FIGURE 4**

**FIGURE 5**

FIGURE 6

**FIGURE 7**

**FIGURE 8**

FIGURE 9

FIGURE 10

A: Nil,
A/PR8 challenge

B: Gamma-A/PC immunized,
A/PR8 challenge

C: Survival:
Heterosubtypic challenge

**FIGURE 11**

A: Nil,
A/PR8 challenge

B: Gamma-A/PC immunized
A/PR8 challenge

C: Survival:
Heterosubtypic challenge

**FIGURE 12**

A: Nil,
A/PR8 challenge

B: Gamma-A/PC immunized
A/PR8 challenge

C: Survival:
Heterosubtypic challenge

Nil
Gamma-A/PC

**FIGURE 13**

A: Nil, A/PR8 challenge

B: Adoptive T cell transfer, A/PR8 challenge

C: Adoptive B cell transfer, A/PR8 challenge

D: Survival: Heterosubtypic challenge

—▲— 1.1 x 10⁷ T cell
—✕— 1.1 x 10⁷ B cell
—△— Nil

**FIGURE 14**

A: Nil,
A/PR8 challenge

B: Gamma-A/PC immunized,
A/PR8 challenge

C: Survival:
Heterosubtypic challenge

FIGURE 15

A: Naive mice,
A/PR8 challenge

B: Gamma-A/PC immunized
A/PR8 challenge

C: Survival:
Heterosubtypic challenge

**FIGURE 16**

**FIGURE 17**

FIGURE 18

Secondary *in vivo* Tc cell response

**FIGURE 19**

A: Nil,
A/PC challenge

B: Formalin-A/PC immunized
A/PC challenge

C: UV-A/PC immunized
A/PC challenge

D: Gamma-A/PC immunized
A/PC challenge

E: Survival:
Homologous challenge

Gamma-A/PC
Formalin-A/PC
UV-A/PC
Nil

**FIGURE 20**

EP 2 667 891 B1

FIGURE 20

73

A: Nil,
A/PC challenge

B: Formalin-A/PC x 1,
A/PC challenge

C: Formalin-A/PC x 2,
A/PC challenge

D: Formalin-A/PC x 3,
A/PC challenge

**FIGURE 21**

FIGURE 21

A: Naive,
A/PC challenge

B: Trivalent influenza vaccine,
A/PC challenge

C: Survival,
A/PC challenge

Naive

Trivalent influenza vaccine

D: Naive,
A/PR8 challenge

E: Trivalent influenza vaccine,
A/PR8 challenge

**FIGURE 22**

F: Survival,
A/PR8 challenge

**FIGURE 22**

A: Naïve lung
B: Unvaccinated (infected)
C: Gamma-A/PC vaccinated (challenged)
D: Formalin-A/PC vaccinated (challenged)
E: UV-A/PC vaccinated (challenged)

**FIGURE 23**

A: Naïve lung
B: Unvaccinated (infected)
C: Gamma-A/PC vaccinated (challenged)
D: Formalin-A/PC vaccinated (challenged)
E: UV-A/PC vaccinated (challenged)

**FIGURE 24**

**FIGURE 25**

**FIGURE 26**

A: Nil,
LD$_{50}$ A/PR8 challenge

B: Nil,
5 x LD$_{50}$ A/PR8 challenge

C: Gamma-A/PC immunized
LD$_{50}$ A/PR8 challenge

D: Gamma-A/PC immunized
5 x LD$_{50}$ A/PR8 challenge

**FIGURE 27**

E. Gamma-A/PC immunized
50 x LD 50 A/PR8 challenge

F. Survival:
Heterosubtypic challenge

FIGURE 27

A: Age matched naive mice,
A/PR8 challenge

B: Gamma-A/PC immunized mice,
A/PR8 challenge

C: Survival:
Heterosubtypic challenge

**FIGURE 28**

A: Naive mice,
   A/PR8 challenge

B: Freeze dried gamma-A/PC immunized,
   A/PR8 challenge

C: Survival:
   Heterosubtypic challenge

* —▲— Naive mice
  —△— Freeze dried gamma-A/PC

**FIGURE 29**

FIGURE 30

FIGURE 31

FIGURE 32

FIGURE 33

**FIGURE 34**

**FIGURE 35**

**FIGURE 36**

**FIGURE 37**

**FIGURE 38**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010012045 A1
- WO 2007092315 A2
- US 7270990 B
- WO 2002067983 A
- WO 2005113756 A
- US 5824536 A
- US 6344354 B
- US 7192759 B
- WO 2006108846 A
- US 6825036 B
- US 6455298 B
- US 5698433 A
- US 5753489 A
- US 6146873 A
- US 6951752 B
- US 6673591 B
- US 6686190 B
- WO 2005024039 A
- US 7132271 B
- US 3557370 A
- US 3567938 A
- US 4356270 A
- US 4458066 A
- EP 1216053 A
- US 6372223 B
- EP 0399843 A
- US 7029678 B
- WO 2007006939 A
- WO 199602555 A
- WO 199400153 A
- WO 199633739 A
- WO 199517210 A
- US 6569458 B

### Non-patent literature cited in the description

- **ALSHARIFI et al.** *PloS ONE,* 2009, vol. 4 (4), e5336
- **DIEBOLD et al.** Innate Antiviral Responses by Means of TLR7-Mediated Recognition of Single-Stranded RNA. *Science,* 2004, vol. 303 (5663), 1529-1531
- Current Protocols in Microbiology. John Wiley and Sons, Inc, 2007
- Vaccine production. **FURMINGER et al.** Textbook of Influenza. Blackwell Science, 1998, 324-332
- Production of influenza virus in cell cultures for vaccine preparation. **MERTEN et al.** Novel Strategies in Design and Production of Vaccines. 1996, 141-151
- **LAVER.** Purification of influenza virus. Academic Press, 1969, 82-86
- **SOKOLOV et al.** Purification and concentration of influenza virus. *Archiv fiir die gesarate Virusforschung,* 1971, vol. 35, 356-363
- **SATO et al.** Separation and purification of the hemagglutinins from Bordetella pertussis. *Infect. Immun.,* 1983, vol. 41, 313-320
- **KHORLIN et al.** Synthetic inhibitors of Vibrio cholerae neuraminidase and neuraminidases of some influenza virus strains. *FEBS Lett.,* 1970, vol. 8, 17-19
- **VAN DEUSEN et al.** Micro neuraminidase-inhibition assay for classification of influenza A virus neuraminidases. *Avian Dis.,* 1983, vol. 27, 745-50
- Current Protocols in Microbiology. John Wiley & Sons, Inc, 2000
- Current Protocols in Molecular Biology. John Wiley & Sons, Inc, 2000
- **COLIGAN et al.** Current Protocols in Protein Science. John Wiley and Sons, 2000
- **SAMBROOK et al.** Molecular Cloning: A Laboratory Manual. Cold Spring Harbor Laboratory Press, 1989
- Current Protocols in Molecular Biology. John Wiley and Sons, Inc, 2000
- Current Protocols in Protein Science. John Wiley and Sons, Inc, 2000
- The Recombinant Protein Handbook. Pharmacia Biotech, 1994
- **STEWARD et al.** Solid Phase Peptide Synthesis. H. Freeman Co, 1963
- **MEIENHOFER.** 2. *Hormonal Proteins and Peptides,* 1973, 46
- **SCHRODER et al.** The Peptides. Academic Press, 1965, vol. 1, 72-75
- **NARANG et al.** Improved phosphotriester method for the synthesis of gene fragments. *Meth. Enzymol.,* 1979, vol. 68, 90

- **BROWN et al.** Chemical Synthesis and Cloning of a Tyrosine tRNA Gene. *Meth. Enzymol.,* 1979, vol. 68, 109-151
- **BEAUCAGE ; CARUTHERS.** Deoxynucleotide phosphoramidite. *Tetrahedron Letters,* 1981, vol. 22, 1859-1862
- Remington's Pharmaceutical Sciences. Mack Publishing Co, 1990
- Handbook of Pharmaceutical Excipients. The Pharmaceutical Press, London, American Pharmaceutical Association, 2003
- Methods in Cell Biology. Academic Press, 1976, vol. XIV, 33
- **BROWN ; JACKSON.** Lipid based self adjuvanting vaccines. *Current Drug Delivery,* 2005, vol. 23, 83
- **THOELEN et al.** A prophylactic hepatitis B vaccine with a novel adjuvant system. *Vaccine,* 2001, vol. 19, 2400-2403
- **SATO et al.** Immunostimulatory DNA sequences necessary for effective intradermal gene immunization. *Science,* 1996, vol. 273, 352-354
- **VOLLER et al.** New Trends and Developments in Vaccines. University Park Press, 1978
- Vaccine Adjuvants: Preparation Methods and Research Protocols. Methods in Molecular Medicine. Humana Press Inc, 2000
- Virtis Model. Bench, 10-324
- **COSTANTINO et al.** Protein spray freeze drying. 2. Effect of formulation variables on particle size and stability. *J Pharm Sci.,* 2002, vol. 91, 388-395
- **COSTANTINO et al.** Protein spray-freeze drying. Effect of atomization conditions on particle size and stability. *Pharm Res.,* 2000, vol. 17, 1374-1383
- **MAA et al.** Protein inhalation powders: spray drying vs spray freeze drying. *Pharm Res,* 1999, vol. 16, 249-254
- **CARRASQUILLO et al.** Non-aqueous encapsulation of excipient-stabilized spray-freeze dried BSA into poly(lactide-co-glycolide) microspheres results in release of native protein. *J Control Release,* 2001, vol. 76, 199-208
- **CARRASQUILLO et al.** Reduction of structural perturbations in bovine serum albumin by non-aqueous microencapsulation. *J Pharm Pharmacol.,* 2001, vol. 53, 115-120
- **BOMMER.** Advances in Nasal drug delivery Technology. *Pharmaceutical Technology Europe,* 1999, 26-33
- Goodman And Gilman's: The Pharmacological Bases of Therapeutics. Pergamon Press, 1990
- **YAP et al.** Cytotoxic T cells specific for influenza virus-infected target cells. *Immunology,* 1977, vol. 32, 151
- **PARISH ; MÜLLBACHER.** Automated colorimetric assay for T cell cytotoxicity. *J. Immunol Meth.,* 1983, vol. 58, 225-237
- **MÜLLBACHER.** Hyperthermia and the generation and activity of murine influenza-immune cytotoxic T cells in vitro. *J. Virol.,* 1984, vol. 52, 928-931
- **MÜLLBACHER et al.** Spontaneous mutation at position 114 in H-2Kd affects cytotoxic T cell responses to influenza virus infection. *Eur. J. Immunol.,* 1993, vol. 29, 1228-1234
- **HEINE, H.G. et al.** Rapid detection of highly pathogenic avian influenza H5N1 virus by TaqMan reverse transcriptase-polymerase chain reaction. *Avian Dis.,* 2007, vol. 51, 370-372
- **MÜLLBACHER ; THA HLA.** In vivo administration of major histocompatibility complex class I-specific peptides from influenza virus induces specific cytotoxic T cell hyporesponsiveness. *Eur. J. Immunol.,* 1993, vol. 23, 2526-2531
- **BENNINK et al.** Influenzal pneumonia: early appearance of cross-reactive T cells in lungs of mice primed with heterologous type A viruses. *Immunology,* 1978, vol. 35, 503-509
- **SHEFFIELD et al.** Purification of influenza virus by red-cell adsorption and elution. *British journal of experimental pathology,* 1954, vol. 35, 214-222
- Dialysis and concentration of protein solutions. **ANDREW et al.** Current protocols in immunology. 2001
- Influenza: propagation, quantification, and storage. **SZRETTER et al.** Current Protocols in Microbiology. 2006
- **MUTSCH et al.** Use of the inactivated intranasal influenza vaccine and the risk of Bell's palsy in Switzerland. *The New England journal of medicine,* 2004, vol. 350, 896-903